(19) Europäisches Patentamt
European Patent Office
Office européen des brevets

(11) Publication number: 0 121 972 B1

(12) **EUROPEAN PATENT SPECIFICATION**

(45) Date of publication of patent specification: 24.05.89

(21) Application number: 84200372.5

(22) Date of filing: 14.03.84

(51) Int. Cl.$^4$: C 07 D 211/66, C 07 D 401/04, C 07 D 405/04, C 07 D 498/10, C 07 D 487/10, A 61 K 31/445, A 61 K 31/435 // C07D303/36, C07D303/22, C07D203/26, C07D211/74, C07D211/78, C07D211/44 ,(C07D498/10, 263:00, 221:00),(C07D487/10, 235:00, 221:00)

(54) **N-aryl-alpha-amino-carboxamides.**

(30) Priority: 11.04.83 US 484021
21.02.84 US 581948

(43) Date of publication of application:
17.10.84 Bulletin 84/42

(45) Publication of the grant of the patent:
24.05.89 Bulletin 89/21

(84) Designated Contracting States:
AT BE CH DE FR GB IT LI LU NL SE

(56) References cited:
EP-A-0 035 902
EP-A-0 068 331
CH-A- 462 835

ARZNEIMITTEL-FORSCHUNG (DRUG RESEARCH), vol. 26, no. 8, 1976, pages 1521-1531; P.G.H. VAN DAELE et al.: "Synthetic analgesics: N-(1-2-arylethylr-4-substituted 4-piperidinyl) N-arylalkanamides"

(73) Proprietor: JANSSEN PHARMACEUTICA N.V.
Turnhoutsebaan 30
B-2340 Beerse (BE)

(72) Inventor: Van Daele, Georges Henri Paul
Kongostraat 156
B-2300 Turnhout (BE)
Inventor: De Bruyn, Marcel Frans Leopold
Pater Schrijversstraat 4
B-2323 Hoogstraten (BE)
Inventor: Verdonck, Marc Gustaaf Celine
Dijkzijde 28
B-2360 Oud Turnhout (BE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

In Drug Research *26*, 8, 1521—1531 (1976) 4-phenylamino-4-piperidinylcarboxamides are described as intermediates for the preparation of analgesic agents.

The compounds of the present invention differ from the said prior art compounds by their phenyl-substitution on the amide nitrogen atom, by their substitution on the 1 position of the piperidine or pyrrolidine ring and by their useful properties in normalizing irregular cardiac rhythms in patients suffering from same.

The present invention is concerned with carboxamides having the formula

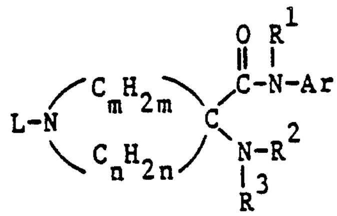

(I),

the pharmaceutically acceptable acid addition salts and the possible stereochemically isomeric forms thereof; wherein one of the hydrogen atoms within the radical —$C_mH_{2m}$— or —$C_nH_{2n}$ may be replaced by hydroxy, lower alkyloxy or lower alkyl; and wherein:

m and n are each the integer 1, 2 or 3, the sum of m and n being 3 or 4;

$R^1$, $R^2$ and $R^3$ are each independently selected from the group consisting of hydrogen, lower alkyl, (aryl)lower alkyl and lower alkylcarbonyl; or $R^1$ and $R^2$, when taken together, may form a methylene function; or $R^2$ and $R^3$, taken together with the nitrogen atom, may form a 1-piperidinyl, 1-pyrrolidinyl or 4-morpholinyl radical;

Ar is aryl; and

L is a member selected from the group consisting of hydrogen; pyrimidinyl; tetrahydropyrimidinyl; tetrahydropyranyl; dihydroindenyl; lower alkenyl; (aryl)lower alkenyl; cycloalkenyl optionally substituted with a hydroxy or an oxo radical; cycloalkyl optionally substituted with up to two radicals selected from the group consisting of lower alkyl, substituted lower alkyl, a radical of formula —$OR^4$, a radical of formula —O—CO—$R^5$, a radical of formula —$NR^6R^8$ and a radical of formula —NH—CO—$R^7$, wherein said substituted lower alkyl is lower alkyl being substituted with a member selected from the group consisting of (lower alkyl)carbonyl, (lower alkyloxy)carbonyl, amino, mono- and di(lower alkyl)amino, $R^4$ is hydrogen, lower alkyl or aryllower alkyl, $R^5$ is lower alkyl, aryl, or aryllower alkyl, wherein up to two hydrogen radicals in the lower alkyl part of the said aryllower alkyl radical may independently be replaced by a member selected from the group consisting of lower alkyloxy and trifluoromethyl, $R^6$ is hydrogen, lower alkyl, aryllower alkyl, lower alkylsulfonyl, or hydroxylower alkyl, $R^7$ is amino, lower alkyl, aryl, lower alkyloxy, or aryl-lower alkyl and $R^8$ is hydrogen, lower alkyl, hydroxylower alkyl, or aryllower alkyl;

a radical of formula

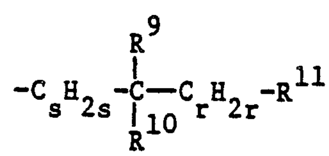

(a)

wherein r and s are independently 0 or an integer of from 1 to 6 inclusive, $R^9$ is hydrogen or hydroxy, $R^{10}$ is hydrogen, lower alkyl or hydroxylower alkyl, and $R^{11}$ is hydrogen, hydroxy, lower alkyloxy, aryloxy, aryllower alkyloxy, cyano, amino, mono- and di-(lower alkyl)amino, arylamino, mono- and di(aryllower alkyl)-amino, aminocarbonyl, mono- and di(lower alkyl)aminocarbonyl, 1-piperidinyl, 4-morpholinyl, 1-pyrrolidinyl, arylaminocarbonyl, 1-piperidinylcarbonyl, 4-morpholinylcarbonyl, 1-pyrrolidinylcarbonyl, lower alkyloxycarbonyl, arylcarbonyl, aryl, cycloalkyl, cycloalkylamino or cycloalkylaminocarbonyl, said cycloalkyl, as embraced in the definition of $R^{11}$, being optionally substituted with a hydroxy radical;

a radical of formula

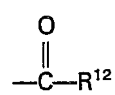

(b)

wherein $R^{12}$ is lower alkyl, lower alkyloxy, (aryl)lower alkyloxy, aryl or cycloalkyl optionally substituted with hydroxy; and

a radical of formula

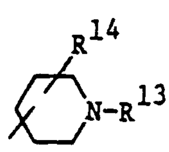

(c)

wherein $R^{13}$ is hydrogen, lower alkyl, lower alkyloxycarbonyl or [(aryl)lower alkyloxy]carbonyl, and $R^{14}$ is hydrogen, hydroxy or lower alkyloxy;
wherein aryl is phenyl optionally substituted with up to three substituents each independently selected from the group consisting of lower alkyl, halo, hydroxy, lower alkyloxy, trifluoromethyl, amino, mono- and di(lower alkyl)amino and nitro.

In the foregoing definitions the term "halo" is generic to fluoro, chloro, bromo and iodo; the term "lower alkyl" is meant to include straight and branch chained saturated hydrocarbon radicals having from 1 to 6 carbon atoms such as, for example, methyl, ethyl, 1-methylethyl, 1,1-dimethylethyl, propyl, 2-methylpropyl, butyl, pentyl, hexyl and the like; the term "lower alkenyl" refers to alkenyl radicals having from 3 to 6 carbon atoms, such as, for example, 2-propenyl, 2-butenyl, 3-butenyl, 2-pentenyl and the like; the term "cycloalkyl" is generic to cyclopropyl, cyclobutyl, cyclopentyl and cyclohexyl; and the term "cycloalkenyl" refers to cyclopentenyl and cyclohexenyl.

The compounds of formula (I) may contain in their structure a keto-enol system or a vinylog system thereof and consequently these compounds may be present in their keto form as well as their enol form.

Preferred compounds within the scope of the present invention are those wherein $R^1$ is hydrogen and Ar is 2,6-dimethylphenyl.

Particularly preferred compounds are those wherein $R^1$ is hydrogen, Ar is 2,6-dimethylphenyl and L is cycloalkyl optionally substituted with hydroxy.

The most preferred compound is 4-(dimethylamino)-*N*-(2,6-dimethylphenyl)-1-(2-hydroxycyclohexyl)-4-piperidinecarboxamide, a pharmaceutically acceptable acid addition salt or a stereochemically isomeric form thereof.

The compounds of formula (I) wherein $R^1$ and $R^2$ are other than a methylene function, said $R^1$ being represented by $R^{1-a}$, said $R^2$ being represented by $R^{2-a}$ and said compounds by the formula

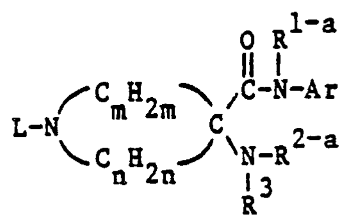

(I-a),

can generally be prepared by the amidation reaction of a carboxylic acid of formula

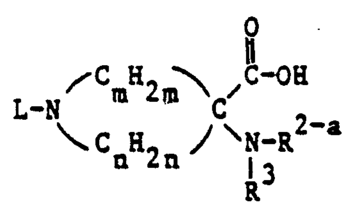

(II)

or a functional derivative thereof, such as an acid halide, an anhydride or an ester, with an amine of formula

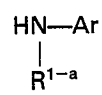

(III)

The said amidation reaction may conveniently be carried out by stirring and, if desired, heating the reactants together in the presence of a suitable reaction-inert solvent such as, for example, a halogenated hydrocarbon, e.g. dichloromethane and the like, an aromatic hydrocarbon, e.g. methylbenzene and the like, an ether, e.g., 1,1'-oxybisethane, tetrahydrofuran and the like. The addition of a suitable base such as, for example, an alkali metal or earth alkaline metal hydride, e.g. sodium hydride, calcium hydride and the like, an alkali metal alkyl, e.g., butyl lithium and the like, an alkali metal amide, e.g. lithium amide, sodium amide and the like, may enhance the rate of the reaction. The water, the alcohol or the acid which is liberated during the course of the reaction is preferably removed from the reaction mixture following art-known procedures such as, for example, by azeotropic distillation, by complexation, by salt-formation and the like methods.

Following a particular amidation reaction procedure the compounds of formula (I-a) wherein $R^{2-a}$ is hydrogen, said compounds being represented by the formula (I-a-1), may be derived from an intermediate of formula (II) wherein $R^{2-a}$ is hydrogen, (II-a), by converting the latter into a cyclic anhydride of formula (IV) and subsequently reacting the said anhydride (IV) with an amine of formula (III).

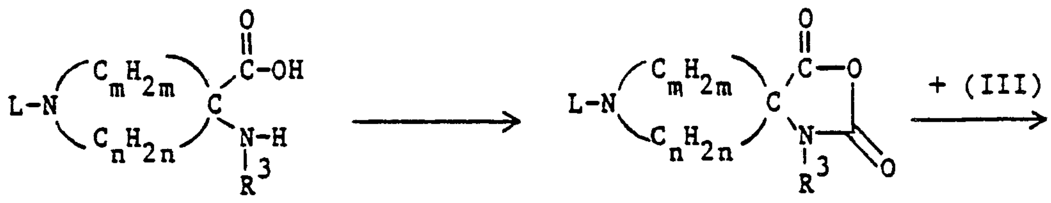


(II-a) (IV)

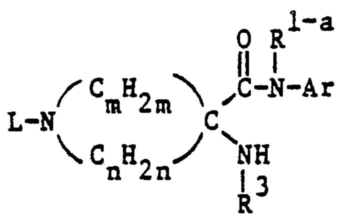

(I-a-1).

The conversion of (II-a) into the oxazolidinedione (IV) may generally be conducted in a suitable solvent in the presence of an appropriate carbonyl generating agent, such as, for example, phosgene or a carbonochloridate, e.g. the trichloromethyl-, the ethyl- or the phenylmethylcarbonochloridate.

The reaction of (IV) with (III) can conveniently be conducted by stirring and, if desired, heating the reactants together, optionally in the presence of a suitable solvent.

The compounds of formula (I-a) wherein $R^{1-a}$ is hydrogen, said compounds being represented by the formula (I-a-2), may also be prepared by reacting a ketone of formula (V) with an amine of formula (VI) and an isonitrile of formula (VII) in the presence of a suitable anion of formula $Y^-$.

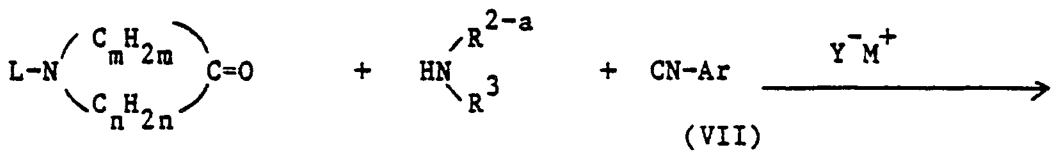


(VII)

(V) (VI)

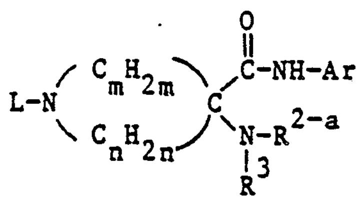

(I-a-2).

The said reaction is generally conducted by mixing the reactants in a suitable solvent at a temperature comprised between −10°C and 25°C and subsequently stirring the reaction mixture at an elevated temperature, preferably at the boiling point of the reaction mixture. Suitable anions $Y^-$ are, for example, anions corresponding to carboxylic acids, e.g., acetic acid and the like acids, and $M^+$ is an appropriate alkalimetal kation, e.g. a sodium- or potassium- kation.

The compounds of formula (I) wherein $R^1$ and $R^2$, taken together, represent a methylene function, said compounds being represented by the formula

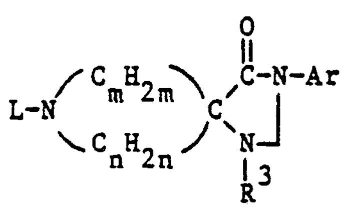

(I-b),

can generally be derived from an appropriate compound of formula (I-a-1) wherein $R^{1-a}$ is hydrogen, said compound being represented by the formula

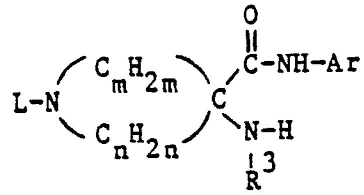

(I-a-1-a),

by reacting the latter with formaldehyde or a polymeric form thereof, e.g. poly(oxymethylene), in a suitable solvent such as *N,N*-dimethylformamide, *N,N*-dimethylacetamide, hexamethylphosphoric triamide and the like solvents, at a temperature comprised between 25°C and the boiling temperature of the reaction mixture.

The compounds of formula (I) may also be converted into each other following art-known functional grouptransformation procedures.

The compounds of formula (I) wherein L is hydrogen, said compounds being represented by the formula (I-c), can be converted into the corresponding compounds of formula (I) wherein L is other than hydrogen, said L being represented by the formula $L^1$ and said compounds by the formula (I-d) following art-known N-alkylating or N-acylating procedures by reacting the former with a reagent of formula (VIII).

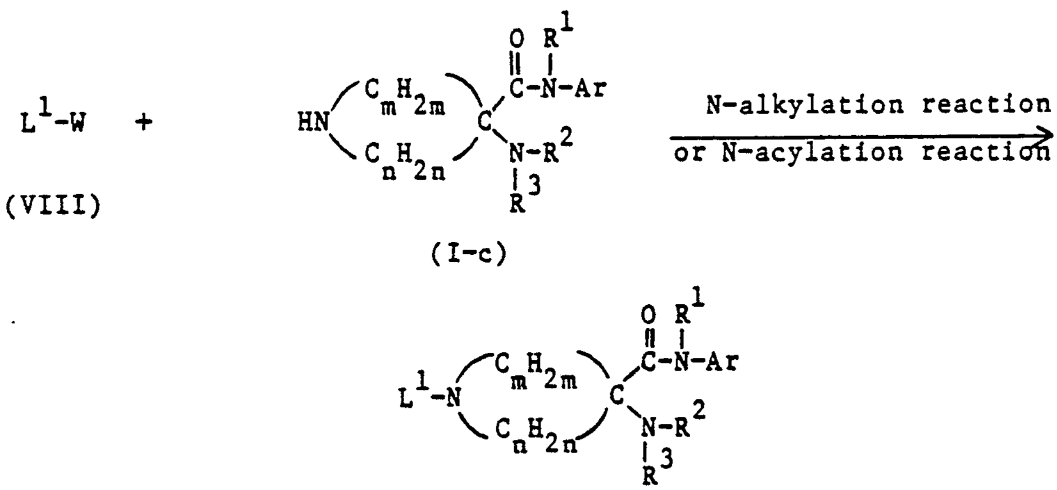


(VIII) (I-c) (I-d)

The N-alkylation or N-acylation reaction is conveniently conducted in an inert solvent such as, for example, an aromatic hydrocarbon, e.g., benzene, methylbenzene, dimethylbenzene and the like; a lower alkanol, e.g., methanol, ethanol, 1-butanol and the like; a ketone, e.g., 4-methyl-2-pentanone and the like; an ether, e.g., 1,4-dioxane, 1,1'-oxybisethane and the like; *N,N*-dimethylformamide; nitrobenzene and the like. The addition of an appropriate base such as, for example, an alkali metal carbonate or hydrogen carbonate, or an organic base such as, for example, *N,N*-diethylethanamine and the like may be utilized to pick up the acid which is liberated during the course of the reaction. In certain cases the addition of an iodide salt, preferably an alkali metal iodide, is appropriate. Somewhat elevated temperature may be used to enhance the reaction rate.

The compounds of formula (I) wherein L is other than hydrogen and other than a radical of formula (b), said L being represented by $L^2$ and said compounds by the formula (I-e), may also be prepared by the reductive amination reaction of an appropriate carbonyl-compound of formula $L^{2'}$=C=O (IX), said $L^{2'}$=C=O being a compound of formula $L^2$—H wherein a —$CH_2$— radical is oxidated to a carbonyl radical.

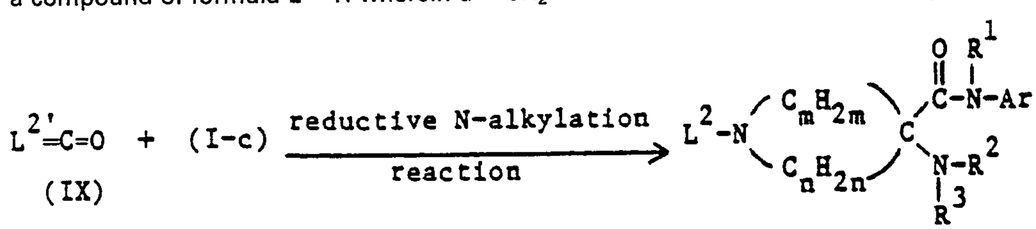


(IX) (I-c) (I-e)

Said reductive N-alkylation reaction may conveniently be carried out by catalytically hydrogenating a stirred and heated mixture of the reactants in a suitable reaction-inert organic solvent according to art-known catalytic hydrogenating procedures. Suitable solvents are, for example, water; lower alkanols, e.g. methanol, 2-propanol and the like; cyclic ethers, e.g. 1,4-dioxane and the like; halogenated hydrocarbons, e.g. trichloromethane and the like; *N,N*-dimethylformamide; dimethyl sulfoxide and the like; or a mixture of 2 or more of such solvents. The term "art-known catalytic hydrogenating procedures" means that the reaction is carried out under hydrogen atmosphere and in the presence of an appropriate catalyst such as, for example, palladium-on-charcoal, platinum-on-charcoal and the like. In order to prevent the undesired further hydrogenation of certain functional groups in the reactants and the reaction products it may be advantageous to add an appropriate catalyst-poison to the reaction mixture, e.g., thiophene and the like.

The compounds of formula (I), wherein L is an optionally substituted 1-cycloalkenyl radical, said compounds being represented by the formula (I-f), may be prepared by reacting an appropriate cycloalkanone of formula (X) with a compound of formula (I-c).

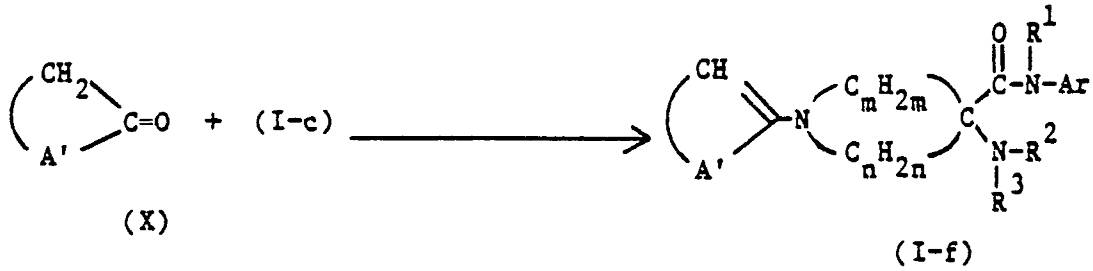

(X)

(I-f)

In (X) and (I-f) A', taken together with the ethanediyl respectively ethenediyl radical represents an optionally substituted cycloalkyl respectively cycloalkenyl radical. The said reaction of (X) with (I-c) may be conducted in a suitable solvent such as, for example, a hydrocarbon, e.g. benzene and the like. Additionally, the compounds of formula (I-f) can be converted into compounds of formula (I), wherein L is an optionally substituted five- or six-membered cycloalkyl radical following art-known reduction procedures, such as, for example, a catalytic hydrogenation reaction.

The compounds of formula (I) wherein L is a cycloalkyl radical which is substituted in its 2-position by a radical of formula XH, said compounds being represented by the formula (I-g) may also be derived from a compound of formula (I-c) by reacting the latter with a reagent of formula (XI).

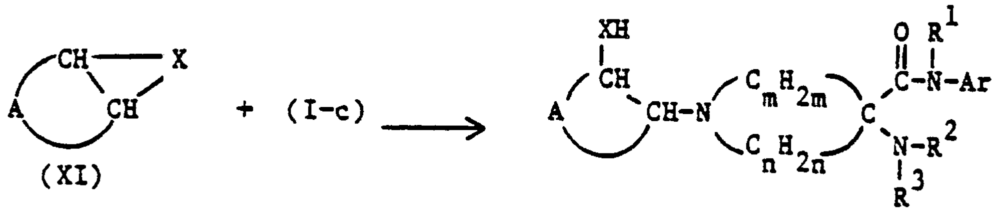

(XI)

(I-g)

In (XI) and (I-g) A, taken together with the ethanediyl radical, represents an optionally substituted cycloalkyl radical; X is 0 or $NR^{15}$ and said $R^{15}$ in combination with N forms an appropriate substituent of the said cycloalkyl radical. The reaction of (XI) with (I-c) may conveniently be conducted by stirring and, if desired, heating the reactants together in a suitable reaction-inert solvent, such as, for example, an alcohol, e.g., ethanol and the like.

The compounds of formula (I), wherein L is an (aryl)lower alkyl radical can be converted into compounds of formula (I), wherein L is a lower alkyloxycarbonyl radical by reacting the starting compounds with an appropriate lower alkyl carbonohalidate, e.g. ethyl carbonochloridate and the like, in the presence of a suitable solvent, such as, for example, a hydrocarbon, e.g. benzene, methylbenzene and the like solvents.

The compounds of formula (I-c) may generally be derived from an appropriate compound of formula (I-d) following art-known procedures of converting tertiary amines or amides into secondary amines.

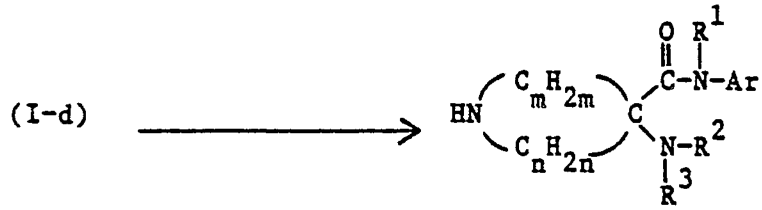

(I-c)

For example, the compounds of formula (I-d) wherein $L^1$ is a radical of formula (b), said compounds

being represented by the formula (I-d-1), may be converted into a compound of formula (I-c) following procedures depending upon the nature of $R^{12}$.

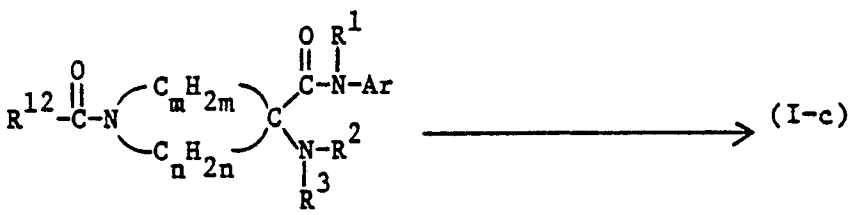

(I-d-1)

Generally, the conversion of (I-d-1) into (I-c) may be conducted in acidic or alkaline medium at a temperature comprised between 20°C and the boiling point of the reaction mixture.

Additionally, the compounds of formula (I-d), wherein $L^1$ is an (aryl)lower alkyl radical or an optionally substituted phenylmethoxycarbonyl radical may be converted into a compound of formula (I-c).

Said reaction may be conducted in a suitable solvent, e.g. an alcohol, under hydrogen atmosphere in the presence of an appropriate catalyst, e.g. palladium-on-charcoal, platinum-on-charcoal and the like.

The compounds of formula (I-a) wherein $R^{1-a}$ and/or $R^{2-a}$ and/or $R^3$ is/are hydrogen may be converted in the corresponding compounds of formula (I-a) wherein $R^{1-a}$ and/or $R^{2-a}$ and/or $R^3$ is/are other than hydrogen following art-known N-alkylating or N-acylating procedures as described hereinabove for the preparation of (I-d) starting from (VIII) and (I-c).

Additionally, the compounds of formula (I-a) wherein $R^{2-a}$ and/or $R^3$ is/are hydrogen may be converted into the corresponding compounds of formula (I-a) wherein $R^{2-a}$ and/or $R^3$ is/are lower alkyl by treating the former compounds with a borohydride salt, e.g. sodium borohydride, in the presence of a suitable carboxylic acid, ketone or aldehyde. For example, compounds of formula (I-a) wherein $R^{2-a}$ is hydrogen may be converted in compounds of formula (I-a) wherein $R^{2-a}$ is ethyl by reacting the former with sodium borohydride in the presence of acetic acid.

Conversely, the compounds of formula (I-a) wherein $R^{1-a}$ and/or $R^{2-a}$ and/or $R^3$ is/are lower alkylcarbonyl or (aryl)lower alkyl may be converted into the corresponding compounds of formula (I-a) wherein $R^{1-a}$ and/or $R^{2-a}$ and/or $R^3$ is/are hydrogen by stirring and, if desired, heating the former compounds in an appropriate acidic- or alkaline medium, respectively a suitable catalytic hydrogenating medium.

The compounds of formula (I) may also be converted into each other following functional grouptransformation procedures in the L-substituents.

For example, the compounds of formula (I) having a phenylmethyl group in the L-radical may be converted into the corresponding debenzylated compounds following art-known hydrogenolyzing procedures; compounds of formula (I) having an alcohol or a primary or secondary amine function may be alkylated following art-known O- or N-alkylating procedures as described hereinabove; compounds of formula (I) having a primary amine function may be converted into the corresponding lower alkylsulfonylamino, aminocarbonylamino or hydroxylower alkylamino derivatives following art-known procedures, such as for example, by reacting the said amine-containing compounds with a lower alkylsulfonylhalide, respectively isocyanate or an epoxide; compounds of formula (I) having a carbonyl-function in the L-substituent may be reduced to the corresponding alcohols following art-known carbonyl-to-alcohol reducing procedures; compounds of formula (I) having a lower alkyloxycarbonyl-amine group may be converted to the corresponding amine-compounds by hydrolyzing the former in acidic or alkaline medium; and compounds of formula (I) wherein L is tetrahydropyrimidinyl may be derived from the corresponding compounds of formula (I) wherein L is pyrimidinyl following art-known catalytic hydrogenating procedures.

The compounds of formula (I) have basic properties and, consequently, they may be converted to their therapeutically active non-toxic acid addition salt forms by treatment with appropriate acids, such as, for example, inorganic acids, such as hydrohalic acid, e.g. hydrochloric, hydrobromic and the like, and sulfuric acid, nitric acid, phosphoric acid and the like; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids. Conversely the salt form can be converted by treatment with alkali into the free base form.

From formula (I) it is evident that the compounds of this invention may have several asymmetric carbon atoms in their structure. Each of these chiral centers may be present in a R- and a S-configuration, this R- and S-notation being in correspondence with the rules described in J. Org. Chem. *35,* (9), 2849—2867 (1970). Pure stereochemically isomeric forms of the compounds of formula (I) may be obtained by the application of art-known procedures.

Diastereoisomers may be separated by physical separation methods such as selective crystallization and chromatographic techniques, e.g., counter current distribution, and enantiomers may be separated from each other by the selective crystallization of their diastereomeric salts with optically active acids.

Pure stereochemically isomeric forms may also be derived from the corresponding pure stereochemically isomeric forms of the appropriate starting materials, provided that the reaction occurs stereospecifically.

In several compounds and starting materials the stereochemical configuration is not experimentally determined. In those cases it is conventionally agreed to designate the stereochemically isomeric form which is first isolated as "A" and the second as "B", without further reference to the actual stereochemical configuration. Stereochemically isomeric forms of the compounds of formula (I) are naturally intended to be embraced within the scope of the invention.

A number of the intermediates and starting materials used in the foregoing preparations are known compounds, others may be prepared according to art-known methodologies of preparing similar compounds and some of them are novel and consequently their preparation will be described hereafter.

The intermediates of formula (II) may be prepared starting from a carbonyl-derivative of formula (V) by reacting the latter with an alkalimetal cyanide (XII) and an amine (VI) and subsequently converting the thus obtained nitrile (XIV) into the corresponding amide.

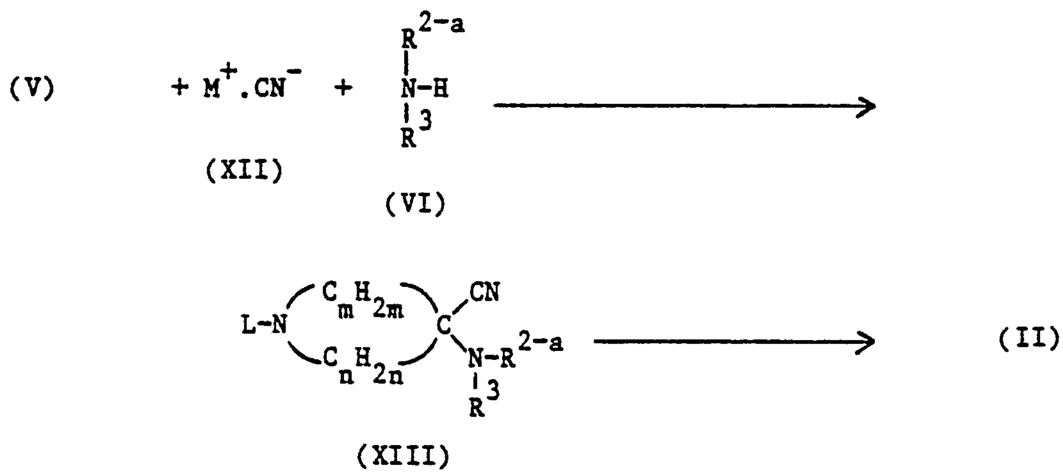

(V) (XII) (VI)

(XIII) (II)

The reaction of (V), (XII) and (VI) is generally conducted in an appropriate solvent, e.g. an alcohol, water and the like, and the conversion of the nitrile (XIII) is generally conducted in strong acidic medium, e.g. concentrated sulfuric acid.

The intermediates of formula (V), (XIV) and (II) may also be converted into each other following art-known functional group-transformation procedures. Some of these transformations are described hereinabove for the compounds of formula (I).

The compounds of formula (I) and their pharmaceutically acceptable acid addition salts and possible stereochemically isomeric forms thereof show excellent antiarrhythmic properties and as such they are useful in the normalization of irregular cardial rhythms. The antiarrhythmic effect of the compounds of this invention is clearly illustrated in the following experiment in dogs. The test was carried out under neuroleptanalgesia (1 ml per 10 kg body weight of fentanyl (0.4 mg/ml) and droperidol (20 mg/ml)). About 16 hours after the ligation of the anterior descendent branch of the left coronary artery, dogs exhibited multifocal ventricular arrhythmia.

The test compounds were given intravenously after a control period of 30 minutes. The lowest effective dose capable of normalizing the cardial rhythm, decreasing significantly the number of premature beats and increasing the number of normal beats, and the duration of action at said lowest effective dose in mg/kg body weight (LED in mg/kg body weight) are given in the following table.

The compounds listed therein are not given for the purpose of limiting the invention thereto but only to exemplify the useful antiarrhythmic properties of all the compounds within the scope of formula (I).

Table I

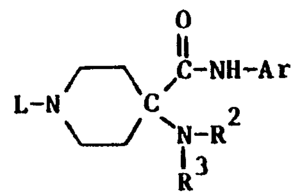

| No. | L | $R^2$ | $R^3$ | Ar | base/ salt | Isomeric form | Antiarrhythmic effects in dogs: LED in mg/kg body weight | Antiarrhythmic effects in dogs: duration in min. |
|---|---|---|---|---|---|---|---|---|
| 71 | H | $CH_3$ | $CH_3$ | $2,6-(CH_3)_2-C_6H_3$ | base | - | 1.25 | 240 |
| 132 | $(CH_3)_2CH$ | $CH_3$ | $CH_3$ | $2,6-(CH_3)_2-C_6H_3$ | base | - | 0.16 | 140 |
| 130 | cyclopentyl | $CH_3$ | $CH_3$ | $2,6-(CH_3)_2-C_6H_3$ | base | - | 0.16 | 65 |
| 91 | 2-OH-cyclopentyl | $CH_3$ | $CH_3$ | $2,6-(CH_3)_2-C_6H_3$ | base | trans | 0.08 | 220 |
| 162 | $CH_2=CHCH_2$ | $CH_3$ | $CH_3$ | $2,6-(CH_3)_2-C_6H_3$ | base | - | 0.31 | 25 |
| 134 | $C_2H_5$ | $CH_3$ | $CH_3$ | $2,6-(CH_3)_2-C_6H_3$ | base | - | 0.63 | 100 |
| 135 | cyclopropyl-$CH_2$ | $CH_3$ | $CH_3$ | $2,6-(CH_3)_2-C_6H_3$ | base | - | 0.16 | 190 |
| 136 | $n.C_3H_7$ | $CH_3$ | $CH_3$ | $2,6-(CH_3)_2-C_6H_3$ | base | - | 0.16 | 15 |
| 137 | $n.C_4H_9$ | $CH_3$ | $CH_3$ | $2,6-(CH_3)_2-C_6H_3$ | base | - | 0.16 | 145 |
| 163 | $(C_2H_5)_2N(CH_2)_2$ | $CH_3$ | $CH_3$ | $2,6-(CH_3)_2-C_6H_3$ | base | - | 0.31 | 220 |
| 73 | H | $C_2H_5$ | $CH_3$ | $2,6-(CH_3)_2-C_6H_3$ | base | - | 0.63 | 30 |
| 138 | $(CH_3)_2CH$ | $C_2H_5$ | $CH_3$ | $2,6-(CH_3)_2-C_6H_3$ | base | - | 1.25 | 100 |
| 174 | $H_2NCOCH(CH_3)$ | $C_2H_5$ | $CH_3$ | $2,6-(CH_3)_2-C_6H_3$ | base | - | 1.25 | 120 |

Table I (cont'd)

| No. | L | $R^2$ | $R^3$ | Ar | base or salt form | Iso-meric form | Antiarrhythmic effects in dogs: LED in mg/kg body weight | Antiarrhythmic effects in dogs: duration in min |
|---|---|---|---|---|---|---|---|---|
| 189 | $(CH_3)_2CH$ | $C_2H_5$ | H | $2,6-(CH_3)_2-C_6H_3$ | base | – | 0.63 | 175 |
| 53 | $(CH_3)_2CH$ | $C_2H_5$ | $C_2H_5$ | $2,6-(CH_3)_2-C_6H_3$ | base | – | 0.63 | 140 |
| 190 | cyclopentyl | H | H | $2,6-(CH_3)_2-C_6H_3$ | base | – | 1.25 | 110 |
| 54 | cyclopentyl | $C_2H_5$ | $C_2H_5$ | $2,6-(CH_3)_2-C_6H_3$ | base | – | 0.16 | 240 |
| 140 | $CH_3OCH_2CH(CH_3)$ | $C_2H_5$ | $C_2H_5$ | $2,6-(CH_3)_2-C_6H_3$ | base | – | 0.31 | 220 |
| 141 | cyclohexyl | $C_2H_5$ | $C_2H_5$ | $2,6-(CH_3)_2-C_6H_3$ | base | – | 0.16 | 40 |
| 92 | 2-OH-cyclopentyl | $C_2H_5$ | $C_2H_5$ | $2,6-(CH_3)_2-C_6H_3$ | base | trans | 0.31 | 205 |
| 93 | 2-OH-cyclohexyl | $C_2H_5$ | $C_2H_5$ | $2,6-(CH_3)_2-C_6H_3$ | base | trans | 0.63 | 30 |
| 143 | $(CH_3)_2CH$ | $C_2H_5$ | $C_2H_5$ | $5-Cl-2-CH_3-C_6H_3$ | base | – | 0.31 | 95 |
| 166 | cyclopentyl | $C_2H_5$ | $C_2H_5$ | $5-Cl-2-CH_3-C_6H_3$ | 2HCl. $1/2H_2O$ | – | 0.63 | 200 |
| 144 | $C_2H_5OOC-$ 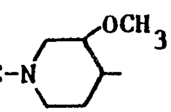 | $CH_3$ | $CH_3$ | $2,6-(CH_3)_2-C_6H_3$ | base | cis | 0.16 | 40 |
| 85 | 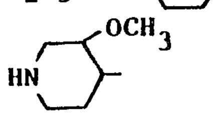 | $CH_3$ | $CH_3$ | $2,6-(CH_3)_2-C_6H_3$ | base | cis | 0.63 | 65 |
| 145 | $C_2H_5OOC-$ 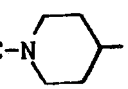 | $CH_3$ | $CH_3$ | $2,6-(CH_3)_2-C_6H_3$ | base | cis | 0.63 | 15 |

Table I (cont'd)

| No. | L | $R^2$ | $R^3$ | Ar | base or salt form | Isomeric form | Antiarrhythmic effects in dogs: LED in mg/kg body weight | Antiarrhythmic effects in dogs: duration in min |
|---|---|---|---|---|---|---|---|---|
| 86 | 4-piperidinyl | $CH_3$ | $CH_3$ | $2,6-(CH_3)_2-C_6H_3$ | base | - | 0.16 | 185 |
| 210 | 3-OH-4-piperidinyl | $CH_3$ | $CH_3$ | $2,6-(CH_3)_2-C_6H_3$ | base | trans | 0.16 | 25 |
| 211 | 4-OH-3-piperidinyl | $CH_3$ | $CH_3$ | $2,6-(CH_3)_2-C_6H_3$ | base | trans | 0.63 | 160 |
| 212 | $1-C_2H_5$-4-OH-3-piperidinyl | $CH_3$ | $CH_3$ | $2,6-(CH_3)_2-C_6H_3$ | base | trans | 0.31 | 60 |
| 167 | $HOCH_2CH_2$ | $CH_3$ | $CH_3$ | $2,6-(CH_3)_2-C_6H_3$ | base | - | 0.31 | 220 |
| 89 | 2-OH-cyclohexyl | $CH_3$ | $CH_3$ | $2,6-(CH_3)_2-C_6H_3$ | base | trans | 0.04 | 240 |
| 146 | $(C_2H_5)_2N-(CH_2)_3CH(CH_3)$ | $CH_3$ | $CH_3$ | $2,6-(CH_3)_2-C_6H_3$ | base | - | 1.25 | 45 |
| 96 | $(PhCH_2)_2NCH_2-CH(OH)CH_2$ | $CH_3$ | $CH_3$ | $2,6-(CH_3)_2-C_6H_3$ | base | - | 0.31 | 40 |
| 191 | $H_2NCH_2CH(OH)CH_2$ | $CH_3$ | $CH_3$ | $2,6-(CH_3)_2-C_6H_3$ | base | - | 1.25 | 20 |
| 97 | $HOCH_2CH(OH)CH_2$ | $CH_3$ | $CH_3$ | $2,6-(CH_3)_2-C_6H_3$ | base | - | 0.31 | 60 |

Table I (cont'd)

| No. | L | $R^2$ | $R^3$ | Ar | base or salt form | Iso-meric form | Antiarrhythmic effects in dogs: LED in mg/kg body weight | Antiarrhythmic effects in dogs: duration in min |
|---|---|---|---|---|---|---|---|---|
| 147 | $C_2H_5OOC(CH_2)_2$ $-CH(CH_3)$ | $CH_3$ | $CH_3$ | $2,6-(CH_3)_2-C_6H_3$ | base | - | 0.63 | 25 |
| 98 | $4-Cl-C_6H_4-O-CH_2$ $-CH(OH)CH_2$ | $CH_3$ | $CH_3$ | $2,6-(CH_3)_2-C_6H_3$ | base | - | 1.25 | 200 |
| 178 | $Ph-NHCH_2CH_2$ | $CH_3$ | $CH_3$ | $2,6-(CH_3)_2-C_6H_3$ | base | - | 0.31 | 110 |
| 148 | $2-[C_2H_5OC(O)CH_2]-$ cyclopentyl | $CH_3$ | $CH_3$ | $2,6-(CH_3)_2-C_6H_3$ | base | - | 0.63 | 40 |
| 193 | $(C_2H_5)_2NCH_2-$ $CH(OH)CH_2$ | $CH_3$ | $CH_3$ | $2,6-(CH_3)_2-C_6H_3$ | base | - | 0.31 | 95 |
| 99 | $2,3-(OH)_2$-cyclohexyl | $CH_3$ | $CH_3$ | $2,6-(CH_3)_2-C_6H_3$ | base | $1\alpha,2\alpha,3\beta$ | 0.16 | 130 |
| 194 | 1,4,5,6-tetrahydro-2-pyrimidinyl | $CH_3$ | $CH_3$ | $2,6-(CH_3)_2-C_6H_3$ | base | - | 0.16 | 25 |
| 100 | $2-OH,2-CH_3$cyclohexyl | $CH_3$ | $CH_3$ | $2,6-(CH_3)_2-C_6H_3$ | base | trans | 0.16 | 25 |
| 83 | H | $CH_3$ | $CH_3$ | $2-Cl,6-CH_3-C_6H_3$ | base | - | 0.04 | 65 |

Table I (cont'd)

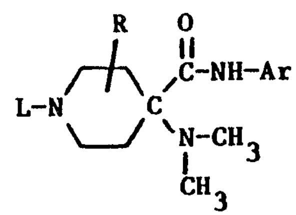

| No. | L | Ar | R | base or salt | Isomeric form | Antiarrhythmic effects in dogs: LED in mg/kg body weight | Antiarrhythmic effects in dogs: duration in min. |
|---|---|---|---|---|---|---|---|
| 101 | Ph-CH(OH)$CH_2$ | 2,6-$(CH_3)_2$-$C_6H_3$ | H | base | - | 0.31 | 65 |
| 102 | Ph-CH($CH_2$OH) | 2,6-$(CH_3)_2$-$C_6H_3$ | H | base | - | 0.04 | 50 |
| 103 | 2-OH-cyclohexyl | 2-Cl,6-$CH_3$-$C_6H_3$ | H | base | trans | 0.16 | 120 |
| 68 | H | 2,6-$(CH_3)_2$-$C_6H_3$ | 3-$OCH_3$ | base | - | 0.31 | 60 |
| 104 | 2-OH-cyclohexyl | 2,6-$(CH_3)_2$-$C_6H_3$ | 3-$OCH_3$ | base | - | 0.16 | 290 |
| 105 | 2-OH-cyclohexyl | 2,6-$(CH_3)_2$-$C_6H_3$ | 3-$CH_3$ | base | A | 0.16 | 120 |
| 106 | 2-OH-cyclohexyl | 2,6-$(CH_3)_2$-$C_6H_3$ | 3-$CH_3$ | base | B | 0.04 | 210 |
| 77 | H | 2,6-$(CH_3)_2$-$C_6H_3$ | 2-$CH_3$ | base | - | 0.16 | 20 |
| 195 | Ph-CH(OH)CH($CH_3$) | 2,6-$(CH_3)_2$-$C_6H_3$ | H | base | - | $\geqslant$ 0.63 | 65 |
| 109 | 2-OH-cyclohexyl | 2,6-$(CH_3)_2$-$C_6H_3$ | 2-$CH_3$ | 1/2$H_2O$ | trans | $>$ 0.16 | - |
| 149 | cyclohexyl | 2,6-$(CH_3)_2$-$C_6H_3$ | H | base | - | 0.16 | 270 |
| 182 | $H_2NC(O)CH_2$ | 2,6-$(CH_3)_2$-$C_6H_3$ | H | base | - | 0.31 | 30 |
| 150 | 2-OH-cyclohexyl | 2,6-$(CH_3)_2$-$C_6H_3$ | H | base | cis | 0.31 | 260 |

Table I (cont'd)

| No. | L | Ar | R | base or salt | Isomeric form | Antiarrhythmic effects in dogs: LED in mg/kg body weight | Antiarrhythmic effects in dogs: duration in min. |
|---|---|---|---|---|---|---|---|
| 110 | $CH_3CH(OH)CH_2$ | $2,6-(CH_3)_2-C_6H_3$ | H | base | – | 0.16 | 185 |
| 184 | $Ph-(CH_2)_3$ | $2,6-(CH_3)_2-C_6H_3$ | H | base | – | 0.08 | 40 |
| 196 | 4-OH-cyclohexyl | $2,6-(CH_3)_2-C_6H_3$ | H | base | trans | 0.31 | 70 |
| 234 | 2-OH-cyclohexyl | $2,6-(CH_3)_2-C_6H_3$ | H | base | (+)trans | 0.02 | 110 |
| 236 | 2-OH-cyclohexyl | $2,6-(CH_3)_2-C_6H_3$ | H | base | (-)trans | 0.04 | 60 |

Table I (cont'd)

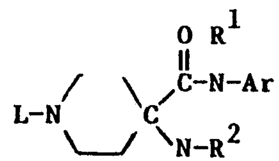

| No. | L | $R^1$ | $R^2$ | $R^3$ | Ar | base or salt form | Isomeric form | Antiarrhythmic effects in dogs LED in mg/kg body weight | duration in min. |
|---|---|---|---|---|---|---|---|---|---|
| 2 | $(CH_3)_2CH$ | H | $CH_3CO$ | $C_2H_5$ | $2,6-(CH_3)_2-C_6H_3$ | base | – | 0.63 | >150 |
| 157 | $(CH_3)_2CH$ | H | $CH_3CO$ | $CH_3$ | $2,6-(CH_3)_2-C_6H_3$ | base | – | 0.63 | 25 |
| 107 | 2-OH-cyclohexyl | H | H | $CH_3$ | $2,6-(CH_3)_2-C_6H_3$ | base | trans | 0.16 | 200 |
| 111 | $Ph-CH_2O(CH_2)_2-CH(OH)CH_2$ | H | $CH_3$ | $CH_3$ | $2,6-(CH_3)_2-C_6H_3$ | base | – | 0.31 | 170 |
| 198 | $HO(CH_2)_2CH(OH)CH_2$ | H | $CH_3$ | $CH_3$ | $2,6-(CH_3)_2-C_6H_3$ | base | – | 0.31 | 190 |
| 32 | H | H | H | $CH_3$ | $2-CH_3,4-CH_3O-C_6H_3$ | base | – | 0.63 | 25 |
| 112 | $2,3-(OH)_2$cyclohexyl | H | $CH_3$ | $CH_3$ | $2,6-(CH_3)_2-C_6H_3$ | base | $1\alpha,2\beta,3\alpha$ | 0.16 | 50 |
| 171 | $(Et)_2NCH_2CH(CH_3)$ | H | $CH_3$ | $CH_3$ | $2,6-(CH_3)_2-C_6H_3$ | base | – | 0.16 | 150 |
| 113 | $Ph-CH_2O(CH_2)_2-C(OH)(CH_3)CH_2$ | H | $CH_3$ | $CH_3$ | $2,6-(CH_3)_2-C_6H_3$ | base | – | $\geqslant$0.16 | 5 |
| 199 | $HO(CH_2)_2C(OH)-(CH_3)CH_2$ | H | $CH_3$ | $CH_3$ | $2,6-(CH_3)_2-C_6H_3$ | base | – | 0.31 | 100 |
| 114 | 2-HO-cyclohexyl | H | $CH_3$ | $CH_3$ | $2-CH_3,4-CH_3O-C_6H_3$ | base | trans | 0.63 | 70 |
| 115 | 2-HO-cyclohexyl | H | $CH_3$ | $CH_3$ | $2-CH_3,5-Cl-C_6H_3$ | base | trans | 0.08 | 95 |

Table I (cont'd)

| No. | L | $R^1$ | $R^2$ | $R^3$ | Ar | base or salt form | Isomeric form | Antiarrhythmic effects in dogs: LED in mg/kg body weight | duration in min. |
|---|---|---|---|---|---|---|---|---|---|
| 116 | 2-HO-cyclohexyl | H | $CH_3$ | $CH_3$ | $2-CH_3O-C_6H_4$ | base | trans | 0.63 | 190 |
| 80 | H | H | $CH_3$ | $CH_3$ | $2,4,6-(CH_3)_3C_6H_2$ | base | - | 0.31 | 30 |
| 117 | 2-HO-cyclohexyl | H | $CH_3$ | $CH_3$ | $2,4,6-(CH_3)_3C_6H_2$ | base | trans | 0.63 | 35 |
| 204 | 2-AcO-cyclohexyl | H | $CH_3$ | $CH_3$ | $2,6-(CH_3)_2C_6H_3$ | base | trans | 0.31 | 140 |
| 203 | 2-EtCOO-cyclohexyl | H | $CH_3$ | $CH_3$ | $2,6-(CH_3)_2C_6H_3$ | base | trans | 0.08 | 60 |
| 205 | 2-PhCOO-cyclohexyl | H | $CH_3$ | $CH_3$ | $2,6-(CH_3)_2C_6H_3$ | base | trans | 1.25 | 30 |
| 128 | $2-PhCH_2NH$-cyclohexyl | H | $CH_3$ | $CH_3$ | $2,6-(CH_3)_2C_6H_3$ | base | trans | 0.08 | 100 |
| 216 | $2-NH_2$-cyclohexyl | H | $CH_3$ | $CH_3$ | $2,6-(CH_3)_2C_6H_3$ | base | trans | 0.04 | 330 |
| 160 | 3-HO-cyclohexyl | H | $CH_3$ | $CH_3$ | $2,6-(CH_3)_2C_6H_3$ | base | cis | 0.08 | 320 |
| 222 | 2-EtCONH-cyclohexyl | H | $CH_3$ | $CH_3$ | $2,6-(CH_3)_2C_6H_3$ | base | trans | 0.16 | 280 |
| 218 | 2-AcNH-cyclohexyl | H | $CH_3$ | $CH_3$ | $2,6-(CH_3)_2C_6H_3$ | base | trans | 0.08 | 240 |
| 226 | $2-(CH_3)_2N$-cyclohexyl | H | $CH_3$ | $CH_3$ | $2,6-(CH_3)_2C_6H_3$ | base | trans | 0.16 | 220 |
| 126 | 2-HO-cyclohexyl | $CH_3$ | $CH_3$ | $CH_3$ | $2-CH_3,4-CH_3O-C_6H_3$ | base | trans | 0.63 | 190 |
| 223 | 2-PhCONH-cyclohexyl | H | $CH_3$ | $CH_3$ | $2,6-(CH_3)_2C_6H_3$ | base | trans | 0.31 | 170 |
| 200 | $2,5-(OH)_2$-cyclohexyl | H | $CH_3$ | $CH_3$ | $2,6-(CH_3)_2C_6H_3$ | base | ($\pm$)-1-($1\alpha,2\beta,5\beta$) | 0.08 | 65 |
| 119 | 2-HO-cyclohexyl | H | $CH_3$ | $CH_3$ | $2,6-(CH_3)_2,4-CH_3O-C_6H_2$ | base | ($\pm$)trans | 1.25 | 165 |

Table I (cont'd)

| No. | L | $R^1$ | $R^2$ | $R^3$ | Ar | base or salt form | Isomeric form | Antiarrhythmic effects in dogs: LED in mg/kg body weight | Antiarrhythmic effects in dogs: duration in min |
|---|---|---|---|---|---|---|---|---|---|
| 120 | 2-HO-cyclohexyl | H | $CH_3$ | $CH_3$ | 2,6-$(CH_3)_2$,4-n.BuO-$C_6H_2$ | base | trans | 1.25 | 30 |
| 122 | 2-HO,5-$PhCH_2O$-cyclohexyl | H | $CH_3$ | $CH_3$ | 2,6-$(CH_3)_2C_6H_3$ | base | (±)-[1-(1α,2β,5α)] | 0.63 | 85 |
| 123 | 2-HO,4-$PhCH_2O$-cyclohexyl | H | $CH_3$ | $CH_3$ | 2,6-$(CH_3)_2C_6H_3$ | base | (±)-[1-(1α,2β,4α)] | 1.25 | 175 |
| 230 | 2-EtNH-cyclohexyl | H | $CH_3$ | $CH_3$ | 2,6-$(CH_3)_2C_6H_3$ | base | trans | 0.04 | 150 |
| 229 | 2-$CH_3$NH-cyclohexyl | H | $CH_3$ | $CH_3$ | 2,6-$(CH_3)_2C_6H_3$ | base | trans | 0.16 | 65 |
| 221 | 2-EtOCONH-cyclohexyl | H | $CH_3$ | $CH_3$ | 2,6-$(CH_3)_2C_6H_3$ | base | trans | 0.16 | 25 |
| 220 | 2-$CH_3SO_2$NH-cyclohexyl | H | $CH_3$ | $CH_3$ | 2,6-$(CH_3)_2C_6H_3$ | base | trans | 0.08 | 300 |
| 24 | H | H | $CH_3$ | $CH_3$ | 2,6-$(CH_3)_2$-4-$(CH_3)_2N$-$C_6H_2$ | 1/2$H_2O$ | - | 0.63 | 20 |
| 124 | 2-HO-cyclohexyl | H | $CH_3$ | $CH_3$ | 2,6-$(CH_3)_2$-3-Br-$C_6H_2$ | base | trans | 0.31 | 225 |
| 201 | 2,5-$(OH)_2$-cyclohexyl | H | $CH_3$ | $CH_3$ | 2,6-$(CH_3)_2C_6H_3$ | base | (±)-[1-(1α,2β,5α)] | 0.16 | 30 |

Table I (cont'd)

| No. | L | $R^1$ | $R^2$ | $R^3$ | Ar | base or salt form | Isomeric form | Antiarrhythmic effects in dogs: LED in mg/kg body weight | Antiarrhythmic effects in dogs: duration in min. |
|---|---|---|---|---|---|---|---|---|---|
| 202 | 2,4-$(OH)_2$-cyclohexyl | H | $CH_3$ | $CH_3$ | 2,6-$(CH_3)_2C_6H_3$ | base | (±)-[1-(1α,2β,4α)] | 0.31 | 5 |
| 125 | 2-HO-cyclohexyl | H | $CH_3$ | $CH_3$ | 2,6-$(CH_3)_2$-4-$(CH_3)_2N$-$C_6H_2$ | base | trans | 0.08 | 165 |
| 224 | 2-$NH_2$CONH-cyclohexyl | H | $CH_3$ | $CH_3$ | 2,6-$(CH_3)_2C_6H_3$ | base | trans | 0.04 | 130 |
| 127 | 2-HO-cyclohexyl | H | 4-morpholinyl | | 2,6-$(CH_3)_2C_6H_3$ | base | trans | 0.31 | 55 |
| 18 | 2-$CH_3$O-cyclohexyl | H | $CH_3$ | $CH_3$ | 2,6-$(CH_3)_2C_6H_3$ | base | trans | 0.08 | 150 |
| 129 | 2-Ph$CH_2$NH-cyclopentyl | H | $CH_3$ | $CH_3$ | 2,6-$(CH_3)_2C_6H_3$ | base | trans | 0.08 | |
| 225 | 2-[(HO$CH_2CH_2$)-(Ph$CH_2$)N]cyclopentyl | H | $CH_3$ | $CH_3$ | 2,6-$(CH_3)_2C_6H_3$ | base | trans | 0.08 | 140 |
| 217 | 2-$NH_2$-cyclopentyl | H | $CH_3$ | $CH_3$ | 2,6-$(CH_3)_2C_6H_3$ | base | trans | 0.31 | 50 |
| 219 | 2-AcNH-cyclopentyl | H | $CH_3$ | $CH_3$ | 2,6-$(CH_3)_2C_6H_3$ | base | trans | 0.08 | 285 |
| 231 | 2-(HO$CH_2CH_2$)NH-cyclopentyl | H | $CH_3$ | $CH_3$ | 2,6-$(CH_3)_2C_6H_3$ | base | trans | 0.16 | >230 |

In view of their activity to normalize irregular cardial rhythms the subject compounds are useful as antiarrhythmic agents.

In view of their useful antiarrhythmic properties the subject compounds may be formulated into various pharmaceutical forms for administration purposes. To prepare the pharmaceutical compositions of this invention an effective amount of the particular compound or compounds, in base or acid-addition salt form, as the active ingredients, is combined in intimate admixture with a pharmaceutically acceptable carrier, which carrier may take a wide variety of forms depending on the form of preparation desired for administration, said amount being an amount which is effective to normalize irregular cardial rhythms.

These pharmaceutical compositions are desirable in unitary dosage form suitable, preferably, for administration orally, rectally or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets.

Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, may be prepared in which the carrier comprises saline solution, glucose solution or a mixture of saline and glucose solution. Injectable suspensions may also be prepared in which case appropriate liquid carriers, suspending agents and the like may be employed. Acid addition salts of (I), due to their increased water solubility over the corresponding base form, are obviously more suitable in the preparation of aqueous compositions.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier.

Examples of such dosage unit forms are tablets (including scored or coated tablets), capsules, pills, powder packets, wafers, injectable solutions or suspensions, teaspoonfuls, tablespoonfuls and the like, and segregated multiples thereof.

The amount of active ingredient per dosage unit is from about 0.25 mg to about 100 mg and, preferably from about 0.5 to about 20 mg.

The following formulations exemplify compositions typical for the normalization of irregular cardial rhythms in dosage unit form suitable for systemic administration to animal and human subjects in accordance with the instant invention.

*Oral drops:* The following formulation provides 50 litres of an oral-drop solution comprising 10 milligrams of 4-(dimethylamino)-*N*-(2,6-dimethylphenyl)-1-(2-hydroxycyclohexyl)-4-piperidinecarboxamide as the active ingredient (A.I.) per milliliter.

| | |
|---|---|
| A.I. | 500 grams |
| 2-hydroxypropanoic acid | 0.5 liter |
| Sodium saccharin | 1750 grams |
| Cocoa flavour | 2.5 liters |
| Purified water | 2.5 liters |
| Polyethylene glycol q.s. ad | 50 liters |

The A.I. was dissolved in the 2-hydroxypropanoic acid and 1.5 liters of the polyethylene glycol at 60—80°C. After cooling to 30—40°C there were added 35 liters of polyethylene glycol and the mixture was stirred well. Then there was added a solution of the sodium saccharin in 2.5 liters of purified water and while stirring there were added the cocoa flavor and polyethylene glycol q.s. ad volume. The resulting solution was filled into suitable containers.

*Injectable solution:* The following formulation provides 20 liters of a parenteral solution comprising 2 milligrams of 4-(dimethylamino)-*N*-(2,6-dimethylphenyl)-1-(2-hydroxycyclohexyl)-4-piperidinecarboxamide as the active ingredient per milliliter.

| | |
|---|---|
| A.I. | 40 grams |
| 2,3-dihydroxybutanedioic acid | 20 grams |
| methyl 4-hydroxybenzoate | 36 grams |
| propyl 4-hydroxybenzoate | 4 grams |
| water for injection q.s. ad | 20 liters |

The methyl and propyl 4-hydroxybenzoates were dissolved in about 10 liters of boiling water for injection. After cooling to about 50°C there were added while stirring the 2,3-dihydroxybutanedioic acid and thereafter the A.I. The solution was cooled to room temperature and supplemented with water for injection q.s. ad volume. The solution was sterilized by filtration (U.S.P. XVII p. 811) and filled in sterile containers.

*Oral solution:* The following formulation provides 20 liters of an oral solution comprising 5 milligrams of 4-(dimethylamino)-*N*-(2,6-dimethylphenyl)-1-(2-hydroxycyclohexyl)-4-piperidinecarboxamide as the active ingredient per teaspoonful (5 milliliters).

| | |
|---|---|
| A.I. | 20 grams |
| 2,3-dihydroxybutanedioic acid | 10 grams |
| Sodium saccharin | 40 grams |
| 1,2,3-propanetriol | 12 liters |
| Sorbitol 70% solution | 3 liters |
| Methyl 4-hydroxybenzoate | 9 grams |
| Propyl 4-hydroxybenzoate | 1 gram |
| Raspberry essence | 2 milliliters |
| Gooseberry essence | 2 milliliters |
| Purified water q.s. ad | 20 liters |

The methyl and propyl 4-hydroxybenzoates were dissolved in 4 liters of boiling purified water. In 3 liters of this solution were dissolved first the 2,3-dihydroxybutanedioic acid and thereafter the A.I. The latter solution was combined with the remaining part of the former solution and the 1,2,3-propanetriol and the sorbitol solution were added thereto. The sodium saccharin was dissolved in 0.5 liters of water and the raspberry and gooseberry essences were added. The latter solution was combined with the former, water was added q.s. ad volume and the resulting solution was filled in suitable containers.

*Film-coated tablets:* 10,000 Compressed tablets, each containing as the active ingredient 10 milligrams of 4-(dimethylamino)-*N*-(2,6-dimethylphenyl)-1-(2-hydroxycyclohexyl)-4-piperidinecarboxamide, were prepared from the following formulation:

| | | |
|---|---|---|
| Tablet core: | | |
| | A.I. | 100 grams |
| | Lactose | 570 grams |
| | Starch | 200 grams |
| | Polyvinylpyrrolidone (Kollidon K90) | 10 grams |
| | Microcrystalline cellulose (Avicel) | 100 grams |
| | Sodium dodecyl sulfate | 5 grams |
| | Hydrogenated vegetable oil (Sterotex) | 15 grams |
| Coating: | | |
| | Methyl cellulose (Methocel 60 HG) | 10 grams |
| | Ethyl cellulose (Ethocel 22 cps) | 5 grams |
| | 1,2,3-propanetriol | 2.5 milliliters |
| | Polyethylene glycol 6000 | 10 grams |
| | Concentrated colour suspension (Opaspray K-1-2109) | 30 milliliters |
| | Polyvinylpyrrolidone (Povidone) | 5 grams |
| | Magnesium octadecanoate | 2.5 grams |

*Preparation of tablet core:* A mixture of the A.I., the lactose and the starch was mixed well and thereafter humidified with a solution of the sodium dodecyl sulfate and the polyvinylpyrrolidone in about 200 milliliters of water. The wet powder was sieved, dried and sieved again. Then there was added the microcrystalline cellulose and the hydrogenated vegetable oil. The whole was mixed well and compressed into tablets.

*Coating:* To a solution of the methyl cellulose in 75 milliliters of denaturated ethanol there was added a solution of the ethyl cellulose in 150 milliliters of dichloromethane. Then there were added 75 milliliters of dichloromethane and 1,2,3-propanetriol. The polyethylene glycol was molten and dissolved in 75 milliliters of dichloromethane. The latter solution was added to the former and then there were added the magnesium octadecanoate, the polyvinylpyrrolidone and the concentrated colour suspension and the whole was homogenized.

The tablet cores were coated with the thus obtained mixture in a coating apparatus.

*Suppositories:* Hundred suppositories each containing 3 milligrams 4-(dimethylamino)-*N*-(2,6-dimethylphenyl)-1-(2-hydroxycyclohexyl)-4-piperidinecarboxamide as the active ingredient were prepared from the following formulations:

| | |
|---|---|
| A.I. | 0.3 grams |
| 2,3-dihydroxybutanedioic acid | 3 grams |
| Polyethylene glycol 400 | 25 milliliters |
| Surfactant (Span) | 12 grams |
| Triglycerides (Witepsol 555) q.s. ad | 300 grams |

The A.I. was dissolved in a solution of the 2,3-dihydroxybutanedioic acid in polyethylene glycol 400. The surfactant and the triglycerides were molten together. The latter mixture was mixed well with the former solution. The thus obtained mixture was poured onto moulds at a temperature of 37—38°C to form the suppositories.

Although the amount of the active ingredient to be administered may vary within rather wide limites depending upon the particular circumstances daily doses of from 0.005 mg to about 1 mg per kg of body weight, administered once or repeatedly, are in general satisfactory.

The following examples are intended to illustrate and not to limit the scope of the present invention. Unless otherwise stated all parts therein are by weight.

(A) Preparation of Intermediates:

Example 1

A mixture of 73.5 parts of 2,6-dimethyl-4-nitrobenzenamine and 180 parts of formic acid was stirred and refluxed for 5 hours. After cooling, the whole was stirred till room temperature. The mixture was poured into crushed ice. The precipitated product was filtered off and crystallized from 2-propanol. It was filtered off again and dried in vacuo at 80°C, yielding 62.3 parts (73%) of *N*-(2,6-dimethyl-4-nitrophenyl)-formamide (intermediate 1).

A mixture of 30 parts of *N*-(2,6-dimethyl-4-nitrophenyl)formamide and 400 parts of methanol was hydrogenated at normal pressure and at room temperature with 2 parts of palladium-on-charcoal catalyst 10%. After the calculated amount of hydrogen was taken up, the catalyst was filtered off and the product was allowed to crystallize. The precipitated product was stirred in warm 2-methoxyethanol. The whole was filtered and the filtrate was evaporated. The residue was crystallized from 400 parts of methanol. The product was filtered off (the filtrate was set aside) and dried, yielding a first fraction of 16.4 parts (66%) of *N*-(4-amino-2,6-dimethylphenyl)formamide. The filtrate, which was set aside (see above), was concentrated. The concentrate was allowed to crystallize. The product was filtered off and dried, yielding a second fraction of 7.2 parts (29%) of *N*-(4-amino-2,6-dimethylphenyl)formamide; mp. 233.4°C (intermediate 2).

A mixture of 23 parts of poly(oxymethylene), 23 parts of *N*-(4-amino-2,6-dimethylphenyl)formamide, 2 parts of a solution of thiophene in methanol 4% and 540 parts of 2-methoxyethanol was hydrogenated at normal pressure and at 70°C with 3 parts of palladium-on-charcoal catalyst 10%. After the calculated amount of hydrogen was taken up, 10 parts of acetic acid were added and the catalyst was filtered off. The filtrate was evaporated, the residue was taken up in water and treated with a 50% sodium hydroxide solution. The precipitated product was filtered off and crystallized from methanol. The product was filtered off and dried, yielding a first fraction of 16.61 parts of *N*-[4-(dimethylamino)-2,6-dimethylphenyl]form-amide.

The mother-liquor (methanol-filtrate) was evaporated and the residue was stirred in 2,2′-oxybispropane. The product was filtered off and dried, yielding a second fraction of 4.06 parts of *N*-[4-(di-methylamino)-2,6-dimethylphenyl]formamide; m.p. 216.3°C (intermediate 3).

A mixture of 3 parts of *N*-[4-(dimethylamino)-2,6-dimethylphenyl]formamide and 50 parts of a hydrochloric acid solution 1N was stirred and refluxed for 1 hour. After cooling, the whole was treated with a sodium hydroxide solution 50%. The product was extracted twice with dichloromethane. The organic layer was washed once with water, dried, filtered and evaporated. The residue was taken up in methylbenzene and the whole was evaporated again, yielding 2.4 parts (93%) of $N^1$,$N^1$,3,5-tetramethyl-1,4-benzenediamine as a residue (intermediate 4).

Example 2

To a stirred suspension of 100 parts of *N*-(4-hydroxy-2,6-dimethylphenyl)formamide in 1200 parts of 2-propanone were added portionwise 124.3 parts of potassium carbonate. 75.7 Parts of dimethylsulfate were added. Upon completion, stirring was continued overnight at reflux. The whole was filtered while hot and the filtrate was evaporated. The residue was crystallized from acetonitrile. After cooling, the product was filtered off and dried, yielding 62.4 parts (58%) of *N*-(4-methoxy-2,6-dimethylphenyl)formamide (intermediate 5).

A mixture of 18.5 parts of *N*-(4-methoxy-2,6-dimethylphenyl)formamide, 55.16 parts of *N,N*-diethyl-ethanamine and 377 parts of dichloromethane was stirred and refluxed. A solution of 21.61 parts of trichloromethyl carbonochloridate in 104 parts of dichloromethane was added dropwise, during a period of 2 hours. The whole was stirred and refluxed for 30 minutes. After cooling, the mixture was washed three times with a sodium carbonate solution 10%, dried, filtered and evaporated. The residue was purified by column chromatography over silica gel using methylbenzene as eluent. The first fraction was collected and the eluent was evaporated. The residue was crystallised from petroleumether. The product was filtered off and dried, yielding 8.2 parts (49%) of 2-isocyano-5-methoxy-1,3-dimethylbenzene (intermediate 6).

Example 3

To a stirred and cooled (0°C) mixture of 5 parts of 2-aminocyclohexanol, 5.3 parts of sodium carbonate and 180 parts of sodium carbonate were added dropwise 9.7 parts of 2-chloroacetyl chloride at a temperature between 0 and 5°C. The reaction mixture was allowed to reach slowly room temperature and 50 parts of water were added. The layers were separated. The organic phase was dried, filtered and

evaporated. The residue was purified by filtration over silica gel using a mixture of trichloromethane and methanol (90:10 by volume) as eluent. The pure fractions were collected and the eluent was evaporated and the residue was crystallized from 2,2′-oxybispropane. The product was filtered off and dried, yielding 5 parts (61%) of *trans*-2-chloro-*N*-(2-hydroxycyclohexyl)acetamide; mp. 121.3°C (intermediate 7).

Example 4

To a stirred solution of 9.2 parts of *trans*-2-[(diethylamino)methyl]cyclohexanol in 45 parts of tetrahydrofuran were added portionwise 2.4 parts of a sodium hydride dispersion 50%. Upon completion, stirring was continued for 1.30 hours at reflux. After cooling to 0°C, 5.85 parts of methanesulfonyl chloride were added dropwise at a temperature below 10°C (exothermic reaction). Upon completion, stirring was continued for 2 hours. After cooling to 0°C, the reaction mixture was decomposed by the dropwise addition of water. The product was extracted with 1,1′-oxybisethane. The organic layer was washed with water, dried, filtered and evaporated. The residual oil was purified by column chromatography over silica gel using a mixture of trichloromethane and methanol (90:10 by volume) as eluent. The pure fractions were collected. The eluent was evaporated, yielding 6.9 parts (90.4%) of *trans*-2-[(diethylamino)methyl]cyclohexanol methanesulfonate (ester) as an oily residue (intermediate 8).

Example 5

To a stirred mixture of 197.3 parts of *N*-(phenylmethyl)benzenemethanamine and 3 parts of water were added dropwise 92.5 parts of (chloromethyl)oxirane at −5°C (exothermic reaction: temp. rises to 3°C). After stirring for 4 hours at 0°C, the mixture was allowed to reach room temperature and stirring was continued over week-end at room temperature. Then there were added 1440 parts of tetrahydrofuran and 110 parts of a sodium methoxide solution 30% and the whole was stirred overnight at room temperature. 400 Parts of water were added and the layers were separated. The organic phase was washed with water, dried, filtered and evaporated, yielding 153.8 parts (100%) of *N,N*-bis(phenylmethyl)oxiranemethanamine as an oily residue. (intermediate 9).

Example 6

To a stirred mixture of 25 parts of [(3-butenyl)oxymethyl]benzene and 390 parts of dichloromethane were added portionwise 18.49 parts of sodium hydrogen carbonate. The whole was cooled to 5°C and 48.9 parts of 3-chlorobenzenecarboperoxoic acid were added portionwise quickly. Upon completion, stirring was continued first for 30 minutes in an ice-bath and then overnight at room temperature. The precipitate was filtered off and washed thoroughly with dichloromethane. The filtrate was washed successively five times with 100 parts of a saturated sodium sulfite solution, five times with 100 parts of a saturated sodium carbonate solution, twice with 200 parts of a sodium hydroxide solution 2.5% and twice with 200 parts of water. The organic layer was dried, filtered and evaporated. The residue was purified by column chromatography over silica gel using trichloromethane as eluent. The pure fractions were collected and the eluent was evaporated. The residue was evaporated with methylbenzene, yielding 24 parts (80%) of [2-(phenylmethoxy)ethyl]oxirane as a residue. (intermediate 10).

In a similar manner there was also prepared: methyl [2-(phenylmethoxy)ethyl]oxirane as a residue (intermediate 11).

Example 7

39 Parts of *trans* 2-[(phenylmethyl)amino]cyclopentanol were suspended in 100 parts of cyanomethane while cooling in an ice bath. Then there were added successively 60.3 parts of triphenylphosphine, 60 parts of cyanomethane, 31.2 parts of tetrachloromethane and 20.6 parts of *N,N*-diethylethanamine (endothermic reaction). The whole was stirred for 8 hours at ±8°C. After standing overnight in an ice box, the reaction mixture was filtered and the filter cake was washed with cold cyanomethane. The filtrate was evaporated. 175 Parts of petroleumether were added and the whole was evaporated again. The residue was stirred for 5 minutes, filtered and the filtrate was evaporated. The residue was distilled, yielding 29.8 parts (84%) of 6-(phenylmethyl)-6-azabicyclo[3.1.0]hexane; bp. 71—86°C at 0.5 mm pressure. (intermediate 12).

Example 8

A mixture of 100 parts of 4-(methylamino)-1-(phenylmethyl)-4-piperidinecarboxamide and 1000 parts of a concentrate hydrochloric acid solution was stirred and refluxed for 24 hours. The reaction mixture was evaporated. The residual oil was crystallized from 250 parts of hydrochloric acid solution 6N. The solid product was recrystallized from 200 parts of hydrochloric acid solution 6N, yielding 58 parts of 4-(methylamino)-1-(phenylmethyl)-4-piperidinecarboxylate; mp 146—255°C (dec.). (intermediate 13).

A solution of 101.7 parts of 4-(methylamino)-1-(phenylmethyl)-4-piperidinecarboxylic acid dihydrochloride in 300 parts of water was alkalized with ammonium hydroxide till the formed precipitate dissolved again. The solution was neutralized with acetic acid. After cooling, the precipitated product was filtered off and dried in vacuo at 120°C, yielding 49.8 parts of 4-(methylamino)-1-(phenylmethyl)-4-piperidinecarboxylic acid; mp 272°C (intermediate 14).

A solution of 4-(methylamino)-1-(phenylmethyl)-4-piperidinecarboxylic acid and 22 parts of sodium hydroxide in 700 parts of water was hydrogenated at normal pressure and at 30°C with 5 parts of palladium-on-charcoal catalyst 10%. After the calculated amount of hydrogen was taken up, the catalyst was filtered off and washed with water. From the filtrate, the layers were separated. The aqueous phase was

evaporated, yielding 89.7 parts of sodium 4-(methylamino)-4-piperidinecarboxylate as a residue. (intermediate 15).

90 Parts of sodium 4-(methylamino)-4-piperidinecarboxylate were dissolved in a mixture of 1200 parts of water and 2 parts of sodium hydroxide. Then there were still added 20 parts of sodium hydroxide. The resulting solution was diluted with 108 parts of tetrahydrofuran and cooled to 5°C. While stirring, there was added dropwise, during a 1.50 hours-period, a solution of 89.7 parts of (phenylmethyl) carbonochloridate in 162 parts of tetrahydrofuran at a temperature below 10°C. Upon completion, stirring was continued for 3.50 hours in an ice-bath at a temperature below 10°C. The reaction mixture was washed three times with 1,1'-oxybisethane. The aqueous phase was neutralized with acetic acid. The precipitated product was filtered off, washed with water and dried, yielding 108 parts (74%) of 4-(methylamino)-1-(phenylmethoxycarbonyl)-4-piperidinecarboxylic acid; mp >250°C (intermediate 16).

Through a stirred suspension of 107 parts of 4-(methylamino)-1-(phenylmethoxycarbonyl)-4-piperidinecarboxylic acid in 1500 parts of 1,4-dioxane, gazeous carbonic dichloride was bubbled first for 30 minutes at room temperature and further, after heating to reflux, for 1.75 hours at reflux temperature. Then dry nitrogen gas was introduced while meantime, the mixture was allowed to cool to room temperature. The solvent was evaporated and the residue was dissolved in dimethylbenzene. The latter was evaporated again in vacuo in a boiling water-bath, yielding 116.5 parts of (phenylmethyl) 1-methyl-2,4-dioxo-3-oxa-1,8-diazaspiro[4,5]decane-8-carboxylate as a residue (intermediate 17).

## Example 9

A solution of 184 parts of 4-(4-morpholinyl)-1-(phenylmethyl)-4-piperidinecarboxylate in 2880 parts of hydrochloric acid solution 12N was stirred and refluxed for 5 days. The reaction mixture was evaporated. The residue was dissolved in 1000 parts of water. The solution was first alkalized with sodium hydroxide to pH 7—8, washed successively three times with trichloromethane and once with 1,1'-oxybisethane, and further alkalized with sodium hydroxide. The precipitated product was filtered off and crystallized from a mixture of water and a sodium hydroxide solution 50%. The product was filtered off and recrystallized from water, yielding 101.7 parts (54.6%) of sodium 4-(4-morpholinyl)-1-(phenylmethyl)-4-piperidinecarboxylate (intermediate 18).

A mixture of 4.9 parts of sodium 4-(4-morpholinyl)-1-(phenylmethyl)-4-piperidinecarboxylate and 100 parts of hexamethylphosphoric triamide was stirred and heated to 160°C. After cooling to room temperature, 1.84 parts of bromoethane were added slowly. Stirring was continued for 24 hours at room temperature. The reaction mixture was poured into 400 parts of water. The precipitated product was filtered off, washed thoroughly with water and dissolved in trichloromethane. The solution was washed three times with water, dried, filtered and evaporated. The residue solidified on stirring in 2,2'-oxybispropane. The product was filtered off and dried, yielding 2.08 parts (41.7%) of ethyl 4-(4-morpholinyl)-1-(phenylmethyl)-4-piperidinecarboxylate; mp 76.4°C (intermediate 19).

## Example 10

To a stirred mixture of 580 parts of 1-(phenylmethyl)-3-piperidinone in 1200 parts of dichloromethane were added dropwise 360 parts of ethyl carbonochloridate. Upon completion, the whole was stirred and refluxed for 3 hours. Then there were added dropwise 353.5 parts of *N,N*-diethylethanamine and stirring at reflux temperature was continued overnight. The reaction mixture was cooled and washed with water. The organic layer was separated, dried, filtered and evaporated. The residue was distilled, yielding a first fraction of 271 parts of ethyl 3-oxo-1-piperidinecarboxylate; bp 127—131°C at 1 mm pressure and a second fraction of 57 parts of ethyl 3-oxo-1-piperidinecarboxylate; bp 131—140°C at 1—1.5 mm pressure. (intermediate 20).

## Example 11

To a stirred mixture of 17.1 parts of ethyl 4-oxo-1-piperidinecarboxylate and 225 parts of trichloromethane was added dropwise a solution of 16 parts of bromine in 75 parts of trichloromethane at −5—0°C. The trichloromethane phase was washed with ice water, dried, filtered and evaporated, yielding 25 parts of ethyl 3-bromo-4-oxo-1-piperidinecarboxylate as an oily residue. (intermediate 21).

To a stirred mixture of 200 parts of sodium methanolate solution 30% and 640 parts of methanol were added 250 parts of ethyl 3-bromo-4-oxo-1-piperidinecarboxylate at about 20°C. The whole was stirred for 3 hours at room temperature. The solvent was evaporated and the oily residue was dissolved in 2,2'-oxybispropane. The solution was washed with water, dried, filtered and evaporated, yielding 190 parts of ethyl 3-hydroxy-4,4-dimethoxy-1-piperidinecarboxylate as an oily residue. (intermediate 22).

To a stirred mixture of 35 parts of ethyl 3-hydroxy-4,4-dimethoxy-1-piperidinecarboxylate and 144 parts of *N,N*-dimethylformamide were added portionwise 8.2 parts of sodium hydride dispersion 50%; exothermic reaction (temp. rises to 30°C; cooling in a water bath was necessary to keep the temperature below 30°C). The whole was stirred for 1.50 hours at about 30°C and then it was cooled to room temperature. 24.1 Parts of iodomethane were added dropwise (strong exothermic reaction) while the temperature was kept below 30°C. Upon completion, stirring was continued over week-end at room temperature. The reaction mixture was poured onto water and the product was extracted with 4-methyl-2-pentanone. The extract was washed with water, dried, filtered and evaporated, yielding 35.9 parts (95.7%) of ethyl 3,4,4-trimethoxy-1-piperidinecarboxylate as an oily residue. (intermediate 23).

A mixture of 56.2 parts of ethyl 3,4,4-trimethoxy-1-piperidinecarboxylate and 1850 parts of a sulfuric acid solution 1% in water was stirred and refluxed for 2.50 hours. The reaction mixture was cooled to room temperature and alkalized with sodium carbonate. The product was extracted with dichloromethane. The extract was washed with a saturated sodium chloride solution, dried, filtered and evaporated, yielding 41.2 parts (90.4%) of ethyl 3-methoxy-4-oxo-1-piperidinecarboxylate as an oily residue. (intermediate 24).

Example 12

A mixture of 321.5 parts of 4-(ethylamino)-1-(phenylmethyl)-4-piperidinecarboxylate and 3600 parts of a hydrochloric acid solution 12N was stirred and refluxed for 48 hours. The reaction mixture was concentrated to a volume of about 1000 parts. The precipitated product was filtered off (the filtrate was set aside), washed with 100 parts of water and 240 parts of ethanol, and dried, yielding a first fraction of 283 parts (65.2%) of 4-(ethylamino)-1-(phenylmethyl)-4-piperidinecarboxylate acid dihydrochloride monohydrate; mp 260—262.5°C.

The filtrate which was set aside, was concentrated to a volume of 300 parts. The precipitated product was filtered off and crystallized from 300 parts of water. After cooling to 0°C and inocculating, the product was filtered off, washed with ethanol and dried, yielding a second fraction of 51 parts (11.75%) of 4-(ethylamino)-1-(phenylmethyl)-4-piperidinecarboxylic acid dihydrochloride monohydrate. (intermediate 25).

A mixture of 330 parts of 4-(ethylamino)-1-(phenylmethyl)-4-piperidinecarboxylic acid dihydrochloride monohydrate and 1800 parts of water was hydrogenated at normal pressure and at room temperature with 5 parts of palladium-on-charcoal catalyst 10%. After the calculated amount of hydrogen was taken up, the catalyst was filtered off and the filtrate was evaporated, yielding 245.8 parts of 4-(ethylamino)-4-piperidine-carboxylic acid dihydrochloride monohydrate as a residue. (intermediate 26).

To a stirred mixture of 245.8 parts of 4-(ethylamino)-4-piperidinecarboxylic acid dihydrochloride monohydrate in 1800 parts of water were added 149.5 parts of sodium hydroxide followed by the addition of 1080 parts of tetrahydrofuran. After cooling to 5°C, 187.5 parts of (phenylmethyl) carbonochloridate were added dropwise during a 2 hours period. Upon completion, stirring was continued for 4 hours at 5°C. The reaction mixture was washed twice with 350 parts of 1,1'-oxybispropane. The aqueous phase was neutralized with acetic acid. The precipitated product was filtered off and boiled in 2000 parts of water. After cooling to about 50°C, the product was filtered off, washed with ethanol and dried, yielding 223 parts (78%) of 4-(ethylamino)-1-[(phenylmethoxy)carbonyl]-4-piperidinecarboxylic acid. (intermediate 27).

A stirred solution of 153.2 parts of 4-(ethylamino)-1-[(phenylmethoxy)carbonyl]-4-piperidinecarboxylic acid in 1900 of 1,4-dioxane was heated to reflux while carbonic dichloride gas was gently introduced. During 2 hours, gaseous carbonic dichloride was gently bubbled through the refluxing suspension. The reaction mixture was allowed to cool while stirring and while dry nitrogen was introduced for 2 hours. The solvent was evaporated and the residue was evaporated again under benzene. The solid residue was crystallized from 4-methyl-2-pentanone. The product was filtered off and dried, yielding 112 parts (67.5%) of (phenylmethyl) 1-ethyl-2,4-dioxo-3-oxa-1,8-diazaspiro[4,5]decane-8-carboxylate; mp 127.5°C. (intermediate 28).

Example 13

To a stirred and refluxed mixture of 403 parts of ethyl 3-[(phenylmethyl)amino]butanoate and 160 parts of ethanol were added dropwise 100 parts of ethyl 2-propenoate. After stirring overnight at reflux temperature, a second portion of 100 parts of ethyl propenoate were added dropwise. Upon completion, stirring was continued at reflux temperature for 48 hours. The reaction mixture was evaporated, yielding a mixture of ethyl *N*-[2-(ethoxycarbonyl)-1-methylethyl]-*N*-(phenylmethyl)-β-alanine and ethyl *N*-[2-ethoxycarbonyl)ethyl]-*N*-(phenylmethyl)-β-alanine as an oily residue. The latter was stirred at room temperature together with 17 parts of sodium carbonate in 900 parts of trichloromethane. Then there were added dropwise 217 parts of ethyl carbonochloridate. Upon completion, stirring was continued overnight. The mixture was washed with water, dried, filtered and evaporated. The residue was extacted with a diluted hydrochloric acid solution. The aqueous acid phase was washed with 2,2'-oxybispropane. The free base was liberated with ammonium hydroxide and extracted with 2,2'-oxybispropane. The extract was washed with water, dried, filtered and evaporated, yielding 264.5 parts of ethyl *N*-[2-(ethoxycarbonyl)-1-methylethyl]-*N*-(phenylmethyl)-β-alanine as a residue. (intermediate 29).

A mixture of 192.8 parts of ethyl *N*-[2-(ethoxycarbonyl)-1-methylethyl]-*N*-(phenylmethyl)-β-aniline and 280 parts of absolute ethanol was stirred in an hydrogenation vessel. Then there were added 45 parts of hydrochloric acid solution. After cooling, 10 parts of palladium-on-charcoal catalyst 5% were added and the whole was shaken at room temperature while one equivalent of hydrogen was taken up. The catalyst was filtered off and the filtrate was evaporated, yielding 150 parts of ethyl *N*-[2-(ethoxycarbonyl)-1-methylethyl]-β-alanine hydrochloride as an oily residue. (intermediate 30).

To a stirred mixture of 105 parts of ethyl *N*-[2-(ethoxycarbonyl)-1-methylethyl]-β-alanine, 63.8 parts of *N,N*-diethylethanamine and 1200 parts of trichloromethane were added dropwise 61.4 parts of ethyl carbonochloridate at room temperature. Stirring at room temperature was continued overnight. The reaction mixture was evaporated. Water was added to the residue and the product was extracted with 2,2'-oxybispropane. The extract was dried, filtered and evaporated, yielding 114 parts of ethyl 3-[(ethoxycarbonyl)[2-(ethoxycarbonyl)ethyl]amino]butanoate as a residue. (intermediate 31).

To a stirred sodium ethoxide solution, previously prepared starting from 9.5 parts of sodium and 80 parts of ethanol, were added 225 parts of dimethylbenzene. The ethanol was distilled off. To the residue was added dropwise a mixture of 97 parts of ethyl 3-[(ethoxycarbonyl)[2-(ethoxycarbonyl)ethyl]amino]-butanoate and 45 parts of dimethylbenzene while heating at 110—120°C and while ethanol was still distilled off. Upon completion, stirring while heating at 110—120°C was continued for one hour. The reaction mixture was cooled and there were added 50 parts of acetic acid and 50 parts of water. After stirring for a while the layers were separated. The organic phase was dried, filtered and evaporated, yielding 54 parts of diethyl 2-methyl-4-oxo-1,3-piperidinecarboxylate as a residue. (intermediate 32).

54 Parts of diethyl 2-methyl-4-oxo-1,3-piperidinedicarboxylate and a solution of 42 parts of ethanedioic acid in 300 parts of water were stirred and refluxed for 40 hours. The reaction mixture was cooled. The product was extracted with trichloromethane. The extract was dried, filtered and evaporated, yielding 28 parts of ethyl 2-methyl-4-oxo-1-piperidinecarboxylate as a residue. (intermediate 33).

Example 14

A mixture of 111.5 parts of ethyl 4-amino-1-(phenylmethyl)-4-piperidinecarboxylate, 50 parts of poly(oxymethylene), 3 parts of a solution of thiophene in methanol 4% and 480 parts of ethanol was hydrogenated at normal pressure and at 50°C with 5 parts of palladium-on-charcoal catalyst 10%. After the calculated amount of hydrogen was taken up, the catalyst was filtered off and the filtrate was evaporated. The residue was dissolved in dichloromethane. The solution was washed with a dilute sodium hydroxide solution and once with water, dried, filtered and evaporated. The residue was taken up in methylbenzene and the whole was evaporated again, yielding 117.9 parts (96%) of ethyl 4-(dimethylamino)-1-(phenylmethyl)-4-piperidinecarboxylate as a residue. (intermediate 34).

A mixture of 40 parts of ethyl 4-(dimethylamino)-1-(phenylmethyl)-4-piperidinecarboxylate and 400 parts of ethanol was hydrogenated at normal pressure and at 50°C with 4 parts of palladium-on-charcoal catalyst 10%. After the calculated amount of hydrogen was taken up, the catalyst was filtered off and the filtrate was evaporated. The residue was taken up in methylbenzene and evaporation was continued, yielding 21.2 parts (76.8%) of ethyl 4-(dimethylamino)-4-piperidinecarboxylate as a residue. (intermediate 35).

Example 15

A mixture of 19 parts of 7-oxabicyclo[4.1.0]heptane, 21.2 parts of ethyl 4-(dimethylamino)-4-piperidinecarboxylate, 500 parts of water and 400 parts of ethanol was stirred and refluxed for 10 hours. The reaction mixture was concentrated to a volume of about 150 parts and the product was extracted with dichloromethane. The extract was washed with water, dried, filtered and evaporated. The residue was converted into the hydrochloride salt in 525 parts of 1,1'-oxybisethane and 2-propanol. The solvent was decanted and the residue was stirred in 1,1'-oxybisethane. The latter was decanted again and the residue was crystallized from 240 parts of acetonitrile. The product was filtered off and dried, yielding 40.0 parts of ethyl *trans*-4-(dimethylamino)-1-(2-hydroxycyclohexyl)-4-piperidinecarboxylate dihydrochloride; mp 173.4°C. (intermediate 36).

To a stirred mixture of 25.06 parts of ethyl *trans*-4-(dimethylamino)-1-(2-hydroxycyclohexyl)-4-piperidinecarboxylate and 270 parts of tetrahydrofuran were added portionwise 4.32 parts of a sodium hydride dispersion 50% (slightly exothermic reaction). The mixture was heated to 50°C and cooled again to room temperature. Then there were added dropwise 11.92 parts of iodomethane. Upon completion, the whole was heated to reflux and stirring was continued for 2 hours at reflux temperature. Another 0.8 parts of a sodium hydride dispersion 50% was added portionwise and the whole was heated to 50°C. After cooling to room temperature, 2.28 parts of iodomethane were added and stirring was continued for 2 hours at reflux temperature. The reaction mixture was cooled and 200 parts of a sodium chloride solution were added. The layers were separated and the aqueous phase was extracted with methylbenzene. The combined organic layers were dried, filtered and evaporated. The residue was purified by column chromatography over silica gel using a mixture of trichloromethane and methanol saturated with ammonia (95:5 by volume) as eluent.

The first fraction was collected and the eluent was evaporated. The residue was purified again by column chromatography over silica gel using a mixture of trichloromethane and methanol saturated with ammonia (95:5 by volume) as eluent. The pure fractions were collected and the eluent was evaporated, yielding a first fraction of 6.26 parts of ethyl *trans*-4-(dimethylamino)-1-(2-methoxycyclohexyl)-4-piperidinecarboxylate.

The second fraction was collected and the eluent was evaporated, yielding a second fraction of 8.7 parts of ethyl *trans*-4-(dimethylamino)-1-(2-methoxycyclohexyl)-4-piperidinecarboxylate. Total yield: 14.9 parts (56.9%) of ethyl *trans*-4-(dimethylamino)-1-(2-methoxycyclohexyl)-4-piperidinecarboxylate. (intermediate 37).

B. Preparation of final compounds:

Example 16

To a stirred and cooled (ice bath) mixture of 18 parts of acetic acid and 80 parts of 2-propanol were added 32.15 parts of benzenemethanamine. The whole was stirred and cooled to 10°C and 51.3 parts of ethyl 4-oxo-1-piperidinecarboxylate were added. After stirring for 10 minutes, 35.2 parts of 2-isocyano-1,3-dimethylbenzene were added and the whole was heated to reflux. Stirring was continued for 18 hours at

reflux temperature. The reaction mixture was evaporated, yielding 120 parts (75%) of ethyl 4-[acetyl(phenylmethyl)amino]-4-[[(2,6-dimethylphenyl)amino]carbonyl]-1-piperidinecarboxylate as an oily residue (compound 1).

In a similar manner there were also prepared:

4-(acetylethylamino)-*N*-(2,6-dimethylphenyl)-1-(1-methylethyl)-4-piperidinecarboxamide; mp 197.2°C (compound 2);

ethyl 4-(acetylethylamino)-4-[[(2,6-dimethylphenyl)amino]carbonyl]-1-piperidinecarboxylate (compound 3);

ethyl 4-(acetylmethylamino)-4-[(2-chloro-6-methylphenyl)amino]carbonyl]-1-piperidinecarboxylate (compound 4);

ethyl 4-(acetylmethylamino)-4-[(2,6-dimethylphenyl)aminocarbonyl]-3-methoxy-1-piperidinecarboxylate (compound 5);

(A+B)-methyl 4-(acetylmethylamino)-4-[(2,6-dimethylphenyl)-aminocarbonyl]-3-methyl-1-piperidinecarboxylate (compound 6);

ethyl 4-(acetylmethylamino)-4-[(2,6-dimethylphenyl)aminocarbonyl]-2-methyl-1-piperidinecarboxylate as a residue (compound 7);

ethyl 4-(acetylmethylamino)-4-[(4-methoxy-2-methylphenyl)aminocarbonyl]-1-piperidinecarboxylate (compound 8);

ethyl 4-(acetylmethylamino)-4-[(2-methoxyphenyl)aminocarbonyl]-1-piperidinecarboxylate (compound 9);

ethyl 4-(acetylmethylamino)-4-[(5-chloro-2-methylphenyl)aminocarbonyl]-1-piperidinecarboxylate (compound 10);

ethyl 4-(acetylmethylamino)-4-[(2,4,6-trimethylphenyl)aminocarbonyl]-1-piperidinecarboxylate (compound 11); and

ethyl 4-(acetylmethylamino)-4-[(4-methoxy-2,6-dimethylphenyl)aminocarbonyl]-1-piperidinecarboxylate (compound 12).

Example 17

45.5 Parts of 1,1′-oxybisethane were cooled in a 2-propanone/$CO_2$ bath at −50°C under nitrogen atmosphere. Then there were added 25 parts of 1-lithiumbutane at −40—50°C. A solution of 6.57 parts of 4-butoxy-2,6-dimethylbenzenamine in 14 parts of 1,1′-oxybisethane was added dropwise, during a period of 30 minutes, at about −45°C. Upon completion, stirring was continued for 15 minutes at −40°C. A solution of 8.95 parts of ethyl 4-(dimethylamino)-1-(phenylmethyl)-4-piperidinecarboxylate in 14 parts of 1,1′-oxybisethane was added dropwise, during a period of 45 minutes. The whole was allowed to reach slowly room temperature and stirred overnight at this temperature. 1,1′-Oxybisethane was distilled off while at the same time 90 parts of tetrahydrofuran were added. The mixture was stirred and refluxed for 20 hours. After cooling, 8 parts of 2-propanol and water were added. The layers were separated. The aqueous phase was extracted with dichloromethane. The combined organic layers were dried, filtered and evaporated. The residue was purified by column chromatography over silica gel using a mixture of trichloromethane and methanol, saturated with ammonia, (95:5 by volume) as eluent. The first fraction was collected and the eluent was evaporated. The residue was crystallized from 2,2′-oxybispropane. The product was filtered off and dried, yielding 4.03 parts (30%) of *N*-(4-butoxy-2,6-dimethylphenyl)-4-(dimethylamino)-1-(phenylmethyl)-4-piperidinecarboxyamide; mp 195°C. (compound 13).

In a similar manner there were also prepared:

4-(dimethylamino)-1-(phenylmethyl)-*N*-[3-(trifluoromethyl)phenyl]-4-piperidinecarboxyamide as a residue; (compound 14)

4-(dimethylamino-*N*-[4-(dimethylamino)-2,6-dimethylphenyl]-1-(phenylmethyl)-4-piperidinecarboxamide mp 196.8°C; (compound 15)

*N*-(3-bromo-2,6-dimethylphenyl)-4-(dimethylamino)-1-(phenylmethyl)-4-piperidinecarboxamide; (compound 16)

*N*-(2,6-dimethylphenyl)-4-(4-morpholinyl)-1-(phenylmethyl)-4-piperidinecarboxamide; (compound 17)

*trans*-4-(dimethylamino-*N*-(2,6-dimethylphenyl)-1-(methoxycyclohexyl)-4-piperidinecarboxyamide mp 131.5°C. (compound 18).

Example 18

A mixture of 31.8 parts of (phenylmethyl) 1-methyl-2,4-dioxo-3-oxa-1,8-diazaspiro[4,5]decane-8-carboxylate and 36.3 parts of 2,6-dimethylbenzenamine was stirred for 48 hours at 160°C. The reaction mixture was purified by column-chromatography over silica gel using a mixture of trichloromethane and methanol (98:2 by volume) as eluent. The pure fractions were collected and the eluent was evaporated. The residue was suspended in petroleumether. The product was filtered off and crystallized from petroleumether, yielding 22.6 parts of (phenylmethyl) 4-[(2,6-dimethylphenyl)aminocarbonyl]-4-(methylamino)-1-piperidinecarboxylate; mp 156.8°C. (compound 19).

In a similar manner there were also prepared:

(phenylmethyl 4-(methylamino)-4-(phenylaminocarbonyl)-1-piperidinecarboxylate as a residue; (compound 20) and

(phenylmethyl) 4-[[(5-chloro-2-methylphenyl)amino]carbonyl]-4-(ethylamino)-1-piperidinecarboxylate; mp 125.0°C. (compound 21).

Example 19

A mixture of 4 parts of *N*-(4-butoxy-2,6-dimethylphenyl)-4-(dimethylamino)-1-(phenylmethyl)-4-piperidinecarboxyamide and 120 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated. The residue was dissolved in dichloromethane. The solution was washed once with a sodium hydroxide solution 5%, dried, filtered and evaporated. The residue was crystallized from acetonitrile. The product was filtered off and dried, yielding 2.2 parts (70.5%) of *N*-(4-butoxy-2,6-dimethylphenyl)-4-(dimethylamino)-4-piperidinecarboxamide; mp 190°C. (compound 22).

In a similar manner there were also prepared:

4-(dimethylamino)-*N*-[3-(trifluoromethyl)phenyl]-4-piperidinecarboxamide as a residue; (compound 23)

4-(dimethylamino)-*N*-[4-(dimethylamino)-2,6-dimethylphenyl]-4-piperidinecarboxamide hemihydrate mp 167.9°C; (compound 24) and

*N*-(2,6-dimethylphenyl)-4-(4-morpholinyl)-4-piperidinecarboxamide as a residue. (compound 25).

Example 20

A mixture of 6.56 parts of *N*-(3-bromo-2,6-dimethylphenyl)-4-dimethylamino)-1-(phenylmethyl)-4-piperidinecarboxamide, 2.17 parts of ethyl carbonochloridate and 135 parts of benzene was stirred and refluxed for 20 hours. The reaction mixture was cooled and evaporated. The residue was purified by column chromatography over silica gel using a mixture of trichloromethane and methanol (98:2 by volume) as eluent. The pure fractions were collected and the eluent was evaporated, yielding 6.5 parts of ethyl 4-[(3-bromo-2,6-dimethylphenyl)aminocarbonyl]-4-(dimethylamino)-1-piperidinecarboxylate as an oily residue. (compound 26).

Example 21

To a stirred solution of 112.9 parts of ethyl 4-[acetyl(phenylmethyl)amino]-4-[[(2,6-dimethylphenyl)amino]carbonyl]-1-piperidinecarboxylate in 640 parts of 2-propanol were added 140 parts of potassium hydroxide and the whole was heated to reflux. Stirring was continued for 18 hours at reflux temperature. The reaction mixture was evaporated, the residue was taken up in 800 parts of water and the remaining 2-propanol was evaporated. The mixture was stirred for 30 minutes in a boiling water-bath. After cooling to 10°C, the product was extracted twice with 520 parts of dichloromethane. The combined extracts were washed with 200 parts of water, dried, filtered and evaporated. The residue was purified twice by column chromatography over silica gel using first a mixture of trichloromethane and methanol (90:10 by volume) and then a mixture of trichloromethane and methanol (90:10 by volume) saturated with ammonia, as eluent. The pure fractions were collected and the eluent was evaporated. The residue was suspended twice in 70 parts of 1,1′-oxybisethane. The latter was decanted. The solid residue was dried, pulverized and suspended again in 140 parts of 1,1′-oxybisethane. The product was filtered off and dried, yielding 36.4 parts (43%) of *N*-(2,6-dimethylphenyl)-4-[(phenylmethyl)amino]-4-piperidinecarboxamide; mp 132.9°C. (compound 27).

In a similar manner there were also prepared:

*N*-(2,6-dimethylphenyl)-4-(ethylamino)-4-piperidinecarboxamide; mp 140°C; (compound 28)

*N*-(2-chloro-6-methylphenyl)-4-(methylamino)-4-piperidinecarboxamide; (compound 29)

*N*-(2,6-dimethylphenyl)-3-methoxy-4-(methylamino)-4-piperidinecarboxamide; (compound 30) and

(A)-*N*-(2,6-dimethylphenyl)-3-methyl-4-(methylamino)-4-piperidinecarboxamide; (compound 31)

*N*-(4-methoxy-2-methylphenyl)-4-(methylamino)-4-piperidinecarboxamide; mp 85.8°C; (compound 32)

*N*-(2-methoxyphenyl)-4-(methylamino)-4-piperidinecarboxamide as a residue; (compound 33)

*N*-(5-chloro-2-methylphenyl)-4-(methylamino)-4-piperidinecarboxamide as a residue; (compound 34)

*N*-(4-methoxy-2,6-dimethylphenyl)-4-(methylamino)-4-piperidinecarboxamide; (compound 35) and

*N*-(3-bromo-2,6-dimethylphenyl)-4-(dimethylamino)-4-piperidinecarboxamide as a residue. (compound 36).

Example 22

A mixture of 70.1 parts of ethyl 4-(acetylmethylamino)-4-[(2,6-dimethylphenyl)aminocarbonyl]-2-methyl-1-piperidinecarboxylate, 75 parts of potassium hydroxide and 200 parts of 2-propanol was stirred and refluxed for 24 hours. The reaction mixture was evaporated, the residue was taken up twice in water and the whole was evaporated each time till all traces of 2-propanol were removed. The product was extracted twice with dichloromethane. The extract was washed with water, dried, filtered and evaporated. The residue was purified by column chromatography over silica gel using a mixture of trichloromethane and methanol (94:6 by volume) as eluent.

The first fraction was collected and the eluent was evaporated. The residue was further purified by column-chromatography over silica gel using a mixture of trichloromethane and methanol (95:5 by volume) as eluent. The pure fraction was collected and the eluent was evaporated. The residue solidified in 2,2′-oxybispropane. The product was filtered off and dried, yielding a first fraction of 11.3 parts of 1-acetyl-*N*-(2,6-dimethylphenyl)-2-methyl-4-(methylamino)-4-piperidinecarboxamide; mp 172°C.

The second fraction was collected and the eluent was evaporated, yielding 25.9 parts of 1-acetyl-*N*-(2,6-dimethylphenyl)-2-methyl-4-(methylamino)-4-piperidinecarboxamide as a residue. (compound 37).

A mixture of 30 parts of 1-acetyl-*N*-(2,6-dimethylphenyl)-2-methyl-4-(methylamino)-4-piperidinecarboxamide and 300 parts of hydrochloric acid solution 6N was stirred and refluxed for 10 hours. The reaction mixture was cooled and alkalized with ammonium hydroxide. The product was extracted twice with dichloromethane. The combined organic phases (combined aqueous phases were set aside) were washed with a small amount of water, dried, filtered and evaporated. The residue was crystallized from acetonitrile, yielding a first fraction of 3.27 parts of *N*-(2,6-dimethylphenyl)-2-methyl-4-(methylamino)-4-piperidinecarboxamide. The combined aqueous phases which were set aside (see above) were evaporated. The residue was taken up in dichloromethane. The formed precipitate was filtered off and the filtrate was evaporated. The residue was purified by column chromatography over silica gel using a mixture of trichloromethane and methanol, saturated with ammonia, (93:7 by volume) as eluent. The pure fractions were collected and the eluent was evaporated. The residue was crystallized from 2,2'-oxybispropane, yielding a second fraction of 8.8 parts of *N*-(2,6-dimethylphenyl)-2-methyl-4-(methylamino)-4-piperidinecarboxamide. Total yield: 12.07 parts. (compound 38).

## Example 23

To a stirred mixture of 47 parts of *N*-(2,6-dimethylphenyl)-4-(ethylamino)-4-piperidinecarboxamide and 750 parts of trichloromethane were added 35 parts of *N,N*-diethylethanamine. After cooling to 0°C, there was added dropwise, during a 1 hour-period, a solution of 30.5 parts of (phenylmethyl) carbonochloridate in 60 parts of trichloromethane at about 5°C. Upon completion, stirring was continued for 1 hour without ice-bath. The reaction mixture was washed with 200 parts of water. The organic phase was dried, filtered and evaporated. The residue solidified on triturating in 140 parts of 2,2'-oxybispropane. The product was filtered off and crystallized from 240 parts of acetonitrile. After cooling to 0°C, the product was filtered off and dried, yielding 55.7 parts (80%) of (phenylmethyl) 4-[[(2,6-dimethylphenyl)amino]carbonyl]-4-(ethylamino)-1-piperidinecarboxamide; mp 149.5°C (compound 39).

In a similar manner there were also prepared:

(A)-(phenylmethyl) 4-[(2,6-dimethylphenyl)aminocarbonyl]-3-methyl-4-(methylamino)-1-piperidinecarboxylate; mp 149.1°C (compound 40);

(phenylmethyl) 4-[(2,6-dimethylphenyl)aminocarbonyl]-3-methoxy-4-(methylamino)-1-piperidinecarboxylate; mp 149.6°C (compound 41);

(phenylmethyl) 4-[(2-chloro-6-methylphenyl)aminocarbonyl]-4-(methylamino)-1-piperidinecarboxylate; mp 141.8°C (compound 42);

(phenylmethyl) 4-[(2,6-dimethylphenyl)aminocarbonyl]-2-methyl-4-(methylamino)-1-piperidinecarboxylate (compound 43);

(phenylmethyl) 4-[(4-methoxy-2-methylphenyl)aminocarbonyl]-4-(methylamino)-1-piperidinecarboxylate mp 109.9°C; (compound 44)

(phenylmethyl) 4-[(2-methoxyphenyl)aminocarbonyl]-4-(methylamino)-1-piperidinecarboxylate mp 81.1°C; (compound 45)

(phenylmethyl) 4-[(5-chloro-2-methylphenyl)aminocarbonyl]-4-(methylamino)-1-piperidinecarboxylate; mp 98°C (compound 46);

(phenylmethyl) 4-(methylamino)-4-[(2,4,6-trimethylphenyl)aminocarbonyl]-1-piperidinecarboxylate; (compound 47) and

(phenylmethyl) 4-[(4-methoxy-2,6-dimethylphenyl)aminocarbonyl]-4-(methyl-amino)-1-piperidinecarboxylate mp 134.9°C (compound 48).

## Example 24

To a stirred mixture of 9.6 parts of (phenylmethyl) 4-(dimethylamino)-4-[(4-methoxy-2-methylphenyl)amino-carbonyl]-1-piperidinecarboxylate and 180 parts of *N,N*-dimethylformamide were added portionwise, during a period of 10 minutes, 1.23 parts of a sodium hydride dispersion 50%. Upon completion, stirring was continued for 10 minutes at 50°C. After cooling, to room temperature, 3.19 parts of iodomethane were added dropwise. Upon completion, the whole was stirred for 6 hours at room temperature. The reaction mixture was evaporated. Water was added to the residue. The product was extracted twice with dichloromethane. The combined organic layers were washed once with water, dried, filtered and evaporated. The residue was purified three times by column chromatography over silica gel using a mixture of trichloromethane and methanol (99:1 by volume) as eluent. The first fraction was collected and the eluent was evaporated with methylbenzene, yielding 2.7 parts (27%) of (phenylmethyl) 4-(dimethylamino)-4-[(4-methoxy-2-methylphenyl)methylamino-carbonyl]-1-piperidinecarboxylate as a residue. (compound 49).

## Example 25

To a stirred and cooled (0°C) mixture of 7.9 parts of (phenylmethyl) 4-[(2,6-dimethylphenyl)-aminocarbonyl]-4-(methylamino-1-piperidinecarboxylate and 90 parts of anhydrous methylbenzene were added dropwise 8 parts of acetic acid anhydride at a temperature between 5 and 10°C. Upon completion, stirring was continued for 1 hour. The reaction mixture was washed with 200 parts of water. The organic phase was dried, filtered and evaporated. The residue was taken up in methylbenzene and the latter was evaporated again. The residue was crystallized from 240 parts of acetonitrile. The product was filtered off

and dried, yielding 36.6 parts (80%) of (phenylmethyl) 4-(acetylmethylamino)-4-[(2,6-dimethylphenyl)-aminocarbonyl]-1-piperidinecarboxylate (compound 50).

Example 26

To a stirred and hot (50—55°C) solution of 8.9 parts of (phenylmethyl) 4-[(2,6-dimethylphenyl)-aminocarbonyl]-4-(methylamino)-1-piperidinecarboxylate in 81.9 parts of acetic acid were added portionwise, during a 30 minutes-period, 4.0 parts of sodium borohydride. Upon completion, stirring was continued for 3 hours at 50—55°C. The reaction mixture was cooled and poured onto ice-water. The whole was alkalized with sodium hydroxide and the product was extracted with dichloromethane. The extract was washed with water, dried, filtered and evaporated. The residue was crystallized from 4-methyl-2-pentanone, yielding 6.6 parts (69.5%) of (phenylmethyl) 4-[(2,6-dimethylphenyl)aminocarbonyl]-4-(ethylmethylamino)-1-piperidinecarboxylate; mp 211.7°C (compound 51).

Following the same procedure there were also prepared:

*N*-(2,6-dimethylphenyl)-4-[ethyl(phenylmethyl)amino]-1-(1-methylethyl)-4-piperidinecarboxamide (compound 52);

4-(diethylamino)-*N*-(2,6-dimethylphenyl)-1-(1-methylethyl)-4-piperidinecarboxamide; mp 177.6°C (compound 53);

1-cyclopentyl-4-(diethylamino)-*N*-(2,6-dimethylphenyl)-4-piperidinecarboxyamide; mp 193.2°C (compound 54);

(phenylmethyl) 4-(diethylamino)-4-[[(2,6-dimethylphenyl)amino]carbonyl]-1-piperidinecarboxylate; mp 219.0°C (compound 55); and

(phenylmethyl) 4-[[(5-chloro-2-methylphenyl)amino]carbonyl]-4-(diethylamino)-1-piperidinecarboxylate; mp 137.3°C (compound 56).

Example 27

A mixture of 8.03 parts of a formaldehyde solution 40% in water, 14 parts of a sulfuric acid solution 3M and 54 parts of tetrahydrofuran was stirred under nitrogen atmosphere at 10°C. A suspension of 5.9 parts of sodium borohydride and 11.5 parts of (phenylmethyl) 4-[(2,6-dimethylphenyl)aminocarbonyl]-3-methoxy-4-(methylamino)-1-piperidinecarboxylate in 54 parts of tetrahydrofuran was added portionwise (exothermic reaction: the temperature rose to about 25—30°C). When one half of the suspension was added, 14 parts of a sulfuric acid solution 3M were added. After complete addition, another portion of 7 parts of a sulfuric acid solution 3M was added and the whole was stirred for 3 hours at room temperature. 50 Parts of water were added and the whole was treated with a sodium hydroxide solution. After stirring for 5 minutes, the layers were separated. The aqueous phase was extracted with dichloromethane. The organic layer was evaporated. The residue was dissolved in dichloromethane. The solution was washed with water, dried, filtered and evaporated. The residue was crystallized from acetonitrile, yielding 9.88 parts (83.2%) of (phenylmethyl) 4-(dimethylamino)-4-[(2,6-dimethylphenyl)aminocarbonyl]-3-methoxy-1-piperidinecarboxylate; mp 182.1°C (compound 57).

In a similar manner there were also prepared:

(phenylmethyl) 4-[(2-chloro-6-methylphenyl)aminocarbonyl]-4-(dimethylamino)-1-piperidinecarboxylate; mp 228.4°C (compound 58);

(A)-(phenylmethyl) 4-(dimethylamino)-4-[(2,6-dimethylphenyl)aminocarbonyl]-3-methyl-1-piperidinecarboxylate (compound 59); and

(phenylmethyl) 4-(dimethylamino)-4-[(2,6-dimethylphenyl)aminocarbonyl]-2-methyl-1-piperidinecarboxylate (compound 60).

Example 28

A mixture of 16.6 parts of (phenylmethyl) 4-[(2,6-dimethylphenyl)aminocarbonyl]-4-(methylamino)-1-piperidinecarboxylate and 193.8 parts of iodomethane was stirred and refluxed for 10 hours. The reaction mixture was evaporated. The residue was taken up in methylbenzene and the latter was evaporated again. The residue was taken up in dichloromethane and alkaline water and the whole was stirred till all solid enters solution. The organic phase was separated, dried, filtered and evaporated. The residue was crystallized twice from 4-methyl-2-pentanone, yielding 6.4 parts (37.2%) of (phenylmethyl) 4-(dimethylamino)-4-[(2,6-dimethylphenyl)aminocarbonyl]-1-piperidinecarboxylate; mp 232.5°C (compound 61).

In a similar manner there was also prepared:

(phenylmethyl) 4-(dimethylamino)-4-(phenylaminocarbonyl)-1-piperidinecarboxylate as a residue (compound 62).

Example 29

A mixture of 20.5 parts of (phenylmethyl) 4-[(4-methoxy-2-methylphenyl)aminocarbonyl]-4-(methylamino)-1-piperidinecarboxylate, 9.6 parts of methyl 4-methylbenzenesulfonate, 9.1 parts of potassium carbonate and 360 parts of 4-methyl-2-pentanone was stirred and refluxed for 2 hours. Another portion of 9.6 parts of methyl 4-methylbenzenesulfonate and 9.1 parts of potassium carbonate was added and stirring at reflux was continued for 6 hours. The reaction mixture was cooled, water was added and the layers were separated. The organic phase was washed with water, dried, filtered and evaporated. The

residue was purified by column chromatography over silica gel using a mixture of trichloromethane and methanol (96:4 by volume) as eluent. The first fraction was collected and the eluent was evaporated. The residue was stirred in 2,2′-oxybispropane. The product was filtered off and dried, yielding 11.09 parts (52.1%) of (phenylmethyl) 4-(dimethylamino)-4-[(4-methoxy-2-methylphenyl)aminocarbonyl]-1-piperidinecarboxylate; mp 141.2°C (compound 63).

In a similar manner there were also prepared:

(phenylmethyl) 4-(dimethylamino)-4-[[(2-methoxyphenyl)amino]-carbonyl]-1-piperidinecarboxylate mp 146.1°C (compound 64)

(phenylmethyl) 4-[(5-chloro-2-methylphenyl)aminocarbonyl]-4-(dimethylamino)-1-piperidinecarboxylate (compound 65);

(phenylmethyl) 4-(dimethylamino)-4-[(2,4,6-trimethylphenyl)aminocarbonyl]-1-piperidinecarboxylate (compound 66);

(phenylmethyl) 4-(dimethylamino)-4-[(4-methoxy-2,6-dimethylphenyl)aminocarbonyl]-1-piperidinecarboxylate mp 208.2°C (compound 67).

## Example 30

A mixture of 9.3 parts of (phenylmethyl) 4-(dimethylamino)-4-[(2,6-dimethylphenyl)aminocarbonyl]-3-methoxy-1-piperidinecarboxylate and 200 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated. The residue was crystallized from acetonitrile, yielding 5.03 parts (78.5%) of 4-(dimethylamino)-*N*-(2,6-dimethylphenyl)-3-methoxy-4-piperidinecarboxamide; mp 166.3°C (compound 68).

Following the same procedure there were also prepared:

4-(methylamino)-*N*-phenyl-4-piperidinecarboxamide as a residue (compound 69);

4-(dimethylamino)-*N*-4-piperidinecarboxamide; mp 120.8°C (compound 70);

4-(dimethylamino)-*N*-(2,6-dimethylphenyl)-4-piperidinecarboxamide; mp 194.4°C (compound 71);

*N*-(2,6-dimethylphenyl)-4-(methylamino)-4-piperidinecarboxamide; mp 150°C (compound 72);

*N*-(2,6-dimethylphenyl)-4-ethylmethylamino)-4-piperidinecarboxamide; mp 176.0°C (compound 73);

4-(diethylamino-*N*-(2,6-dimethylphenyl)-4-piperidinecarboxamide; mp 195.5°C (compound 74);

4-(acetylmethylamino)-*N*-(2,6-dimethylphenyl)-4-piperidinecarboxamide; as a residue (compound 75);

(A)-4-(dimethylamino)-*N*-(2,6-dimethylphenyl)-3-methyl-4-piperidinecarboxamide; (compound 76);

4-(dimethylamino)-*N*-(2,6-dimethylphenyl)-2-methyl-4-piperidinecarboxamide; mp 121.1°C (compound 77);

4-(dimethylamino)-*N*-(4-methoxy-2-methylphenyl)-4-piperidinecarboxamide (compound 78);

4-(dimethylamino)-*N*-(2-methoxyphenyl)-4-piperidinecarboxamide as a residue (compound 79);

4-(dimethylamino)-*N*-(2,4,6-trimethylphenyl)-4-piperidinecarboxamide; mp 154.7°C (compound 80);

4-(dimethylamino)-*N*-(4-methoxy-2-methylphenyl)-*N*-methyl-4-piperidinecarboxamide as a residue (compound 81); and

4-(dimethylamino)-*N*-(4-methoxy-2,6-dimethylphenyl)-4-piperidinecarboxamide (compound 82).

## Example 31

A mixture of 11.82 parts of (phenylmethyl) 4-[(2-chloro-6-methylphenyl)aminocarbonyl]-4-(dimethylamino)-1-piperidinecarboxylate, 15.15 parts of potassium hydroxide and 80 parts of 2-propanol was stirred and refluxed for 12 hours. The reaction mixture was evaporated. Water was added to the residue and the whole was evaporated to dry. The product was extracted twice with dichloromethane. The combined organic layers were washed with water, dried, filtered and evaporated. The residue was purified by column chromatography over silica gel using a mixture of trichloromethane and methanol, saturated with ammonia, (90:10 by volume) as eluent. The pure fractions were collected and the eluent was evaporated. The residue was crystallized from acetonitrile, yielding 5.57 parts (69.8%) of *N*-(2-chloro-6-methylphenyl)-4-(dimethylamino)-4-piperidinecarboxamide; mp 173.3°C (compound 83).

Following the same procedure there were also prepared:

*N*-(5-chloro-2-methylphenyl)-4-(diethylamino)-4-piperidinecarboxamide; mp 101.2°C (compound 84);

*cis*-4-(dimethylamino)-*N*-(2,6-dimethylphenyl)-3′-methoxy-[1,4′-bipiperidine]-4-carboxamide; mp 111.3°C (compound 85);

4-(dimethylamino)-*N*-(2,6-dimethylphenyl)-[1,4′-bipiperidine]-4-carboxamide; mp 180.7°C (compound 86);

*N*-(5-chloro-2-methylphenyl)-4-(dimethylamino)-4-piperidinecarboxamide as a residue (compound 87); and

4-(methylamino)-*N*-(2,4,6-trimethylphenyl)-4-piperidinecarboxamide (compound 88).

## Example 32

A mixture of 1.5 parts of 7-oxabicyclo[4.1.0]heptane, 4.1 parts of 4-(dimethylamino)-*N*-(2,6-dimethylphenyl)-4-piperidinecarboxamide and 80 parts of ethanol was stirred and refluxed for 36 hours. The reaction mixture was evaporated and the solid residue was purified by column chromatography over silica gel using a mixture of trichloromethane and methanol (90:10 by volume), saturated with ammonia, as

eluent. The pure fractions were collected and the eluent was evaporated. The residue was crystallized from 2-propanol. The product was filtered off and dried overnight at 110°C, yielding 2.7 parts (49.7%) of *trans*-4-(dimethylamino)-*N*-(2,6-dimethylphenyl)-1-(2-hydroxycyclohexyl)-4-piperidinecarboxylate; mp 165.5°C (compound 89).

Following the same procedure and using equivalent amounts of the appropriate starting materials, there were also prepared:

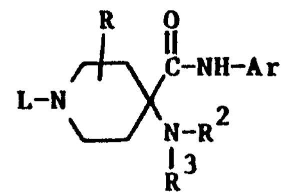

| Comp. No. | L | R | Ar | $R^2$ | $R^3$ | Base or salt form | Isomeric form | mp. in °C |
|---|---|---|---|---|---|---|---|---|
| 90 | $4-Cl-C_6H_4-O-CH_2CH(OH)CH_2$ | H | $C_6H_5$ | $CH_3$ | $CH_3$ | $2(COOH)_2$ | - | 202.1 |
| 91 | 2-OH-cyclopentyl | H | $2,6-(CH_3)_2-C_6H_3$ | $CH_3$ | $CH_3$ | base | trans | 186.8 |
| 92 | 2-OH-cyclopentyl | H | $2,6-(CH_3)_2-C_6H_3$ | $C_2H_5$ | $C_2H_5$ | base | trans | 188.3 |
| 93 | 2-OH-cyclohexyl | H | $2,6-(CH_3)_2-C_6H_3$ | $C_2H_5$ | $C_2H_5$ | base | trans | 175.5 |
| 94 | $1-C_2H_5OOC$, 4-OH-3-piperidinyl | H | $2,6-(CH_3)_2-C_6H_3$ | $CH_3$ | $CH_3$ | base | trans | 150.0 |
| 95 | $1-C_2H_5OOC$, 3-OH-4-piperidinyl | H | $2,6-(CH_3)_2-C_6H_3$ | $CH_3$ | $CH_3$ | base | trans | - |
| 96 | $(C_6H_5CH_2)_2NCH_2CH(OH)CH_2$ | H | $2,6-(CH_3)_2-C_6H_3$ | $CH_3$ | $CH_3$ | base | - | 119.6 |
| 97 | $HO-CH_2CH(OH)CH_2$ | H | $2,6-(CH_3)_2-C_6H_3$ | $CH_3$ | $CH_3$ | base | - | 199.4 |
| 98 | $4-Cl-C_6H_4-O-CH_2CH(OH)CH_2$ | H | $2,6-(CH_3)_2-C_6H_3$ | $CH_3$ | $CH_3$ | base | - | 192.8 |
| 99 | $2,3-(OH)_2$-cyclohexyl | H | $2,6-(CH_3)_2-C_6H_3$ | $CH_3$ | $CH_3$ | base | $(1\alpha,2\alpha,3\beta)$ | 201.4 203.9 |
| 100 | $2-OH,2-CH_3$-cyclohexyl | H | $2,6-(CH_3)_2-C_6H_3$ | $CH_3$ | $CH_3$ | base | trans | 182.8 |
| 101 | $C_6H_5CH(OH)CH_2$ | H | $2,6-(CH_3)_2-C_6H_3$ | $CH_3$ | $CH_3$ | base | - | 192.1 |
| 102 | $C_6H_5CH(CH_2OH)$ | H | $2,6-(CH_3)_2-C_6H_3$ | $CH_3$ | $CH_3$ | base | - | 192.0 |

| Comp. No. | L | R | Ar | $R^2$ | $R^3$ | Base or salt form | Isomeric form | mp. in °C |
|---|---|---|---|---|---|---|---|---|
| 103 | 2-OH-cyclohexyl | H | 2-Cl,6-$CH_3$-$C_6H_3$ | $CH_3$ | $CH_3$ | base | trans | 152.2 |
| 104 | 2-OH-cyclohexyl | 3-$CH_3O$ | 2,6-$(CH_3)_2$-$C_6H_3$ | $CH_3$ | $CH_3$ | base | - | 177.4 |
| 105 | 2-OH-cyclohexyl | 3-$CH_3$ | 2,6-$(CH_3)_2$-$C_6H_3$ | $CH_3$ | $CH_3$ | base | A | 256.0 |
| 106 | 2-OH-cyclohexyl | 3-$CH_3$ | 2,6-$(CH_3)_2$-$C_6H_3$ | $CH_3$ | $CH_3$ | base | B | 244.9 |
| 107 | 2-OH-cyclohexyl | H | 2,6-$(CH_3)_2$-$C_6H_3$ | $CH_3$ | H | base | trans | 173.5 |
| 108 | 2-OH-cyclohexyl | H | 2,6-$(CH_3)_2$-$C_6H_3$ | $CH_3$ | $C_6H_5CH_2$ | base | trans | 165.2 |
| 109 | 2-OH-cyclohexyl | 2-$CH_3$ | 2,6-$(CH_3)_2$-$C_6H_3$ | $CH_3$ | $CH_3$ | 1/2$H_2O$ | - | 163.4 |
| 110 | $CH_3CH(OH)CH_2$ | H | 2,6-$(CH_3)_2$-$C_6H_3$ | $CH_3$ | $CH_3$ | base | - | 158.5 |
| 111 | $C_6H_5CH_2O$-$(CH_2)_2CH(OH)CH_2$ | H | 2,6-$(CH_3)_2$-$C_6H_3$ | $CH_3$ | $CH_3$ | base | - | 95.9 |
| 112 | 2,3-$(OH)_2$cyclohexyl | H | 2,6-$(CH_3)_2$-$C_6H_3$ | $CH_3$ | $CH_3$ | base | 1α,2β,3α | 218.7 |
| 113 | $C_6H_5CH_2O$-$(CH_2)_2C(CH_3)H(OH)CH_2$ | H | 2,6-$(CH_3)_2$-$C_6H_3$ | $CH_3$ | $CH_3$ | base | - | 104.5 |
| 114 | 2-OH-cyclohexyl | H | 2-$CH_3$,4-$CH_3OC_6H_3$ | $CH_3$ | $CH_3$ | base | trans | 136.2 |
| 115 | 2-OH-cyclohexyl | H | 2-$CH_3$,5-Cl-$C_6H_3$ | $CH_3$ | $CH_3$ | base | trans | 142.0 |
| 116 | 2-OH-cyclohexyl | H | 2-$CH_3O$-$C_6H_4$ | $CH_3$ | $CH_3$ | base | trans | 156.2 |
| 117 | 2-OH-cyclohexyl | H | 2,4,6-$(CH_3)_3$-$C_6H_4$ | $CH_3$ | $CH_3$ | base | trans | 198.6 |

| Comp. No. | L | R | Ar | $R^2$ | $R^3$ | Base/ salt | Isomeric form | mp. in °C |
|---|---|---|---|---|---|---|---|---|
| 118 | 2-OH-5-(phenyl-methoxy)cyclohexyl | H | 2,6-$(CH_3)_2$-$C_6H_3$ | $CH_3$ | $CH_3$ | base | (±) [1-(1α,2β,5β)] | 182.0 |
| 119 | 2-OH-cyclohexyl | H | 2,6-$(CH_3)_3$-4-$OCH_3$-$C_6H_2$ | $CH_3$ | $CH_3$ | base | (±)trans | 200.0 |
| 120 | 2-OH-cyclohexyl | H | 2,6-$(CH_3)_3$-4-butoxy-$C_6H_2$ | $CH_3$ | $CH_3$ | base | trans | 173.6 |
| 121 | 2-OH-cyclohexyl | H | 3-$(CF_3)$-$C_6H_4$ $C_6H_2$ | $CH_3$ | $CH_3$ | (E)-2-butene-dioate (1:1) | trans | 206.6 |
| 122 | 2-OH-5-(phenyl-methoxy)cyclohexyl | H | 2,6-$(CH_3)_2$-$C_6H_3$ | $CH_3$ | $CH_3$ | base | (±) [1-(1α,2β,5α)] | 158.6 |
| 123 | 2-OH-4-(phenyl-methoxy)cyclohexyl | H | 2,6-$(CH_3)_2$-$C_6H_3$ | $CH_3$ | $CH_3$ | base | (±) [1-(1α,2β,4α)] | 200.7 |
| 124 | 2-OH-cyclohexyl | H | 3-Br,2,6-$(CH_3)_2$-$C_6H_3$ | $CH_3$ | $CH_3$ | base | trans | 188.3 |
| 125 | 2-OH-cyclohexyl | H | 2,6-$(CH_3)_2$-4-[$(CH_3)_2NH$]$C_6H_3$ | $CH_3$ | $CH_3$ | base | trans | 240.5 |

In a similar manner there were also prepared:

*trans*-4-(dimethylamino)-1-(hydroxycyclohexyl)-*N*-(4-methoxy-2-methylphenyl)-*N*-methyl-4-piperidine-carboxamide mp 133.5°C (compound 126); and

*trans*-*N*-(2,6-dimethylphenyl)-1-(2-hydroxycyclohexyl)-4-(4-morpholinyl)-4-piperidinecarboxamide mp 241.9°C (compound 127).

Example 33

A mixture of 4.12 parts of 7-(phenylmethyl)-7-azabicyclo[4.1.0]-heptane, 5.50 parts of 4-(dimethylamino)-*N*-(2,6-dimethylphenyl)-4-piperidinecarboxamide, 75 parts of water and 60 parts of ethanol was stirred and refluxed overnight with a few drops of a hydrochloric acid solution 30%. The reaction mixture was evaporated, dichloromethane was added and the whole was washed with a saturate sodium carbonate solution. The organic layer was separated, dried, filtered and evaporated. The residue was purified by column chromatography over silica gel using a mixture of trichloromethane and methanol saturated with ammonia (97:3 by volume) as eluent. The pure fractions were collected and the eluent was evaporated. The residue was triturated in 2,2'-oxybispropane. The product was filtered off and dried, yielding 4.39 parts (47%) of *trans*-4-(dimethylamino)-*N*-(2,6-dimethylphenyl)-1-[2-[(phenylmethyl)amino]-cyclohexyl]-4-piperidinecarboxamide; mp 157.6°C (compound 128).

In a similar manner there was also prepared:

*trans*-4-(dimethylamino)-*N*-(2,6-dimethylphenyl)-1-[2-[(phenylmethyl)amino]cyclopentyl])-4-piperidinecarboxamide mp 100.8°C (compound 129).

Example 34

A mixture of 2 parts of cycylpentanone, 2.9 parts of 4-(dimethylamino)-*N*-(2,6-dimethylphenyl)-4-piperidinecarboxamide, 1 part of a solution of thiophene in ethanol 4%, 2 parts of potassium acetate and 120 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated in vacuo. The solid residue was dissolved in dichloromethane. The solution was washed with a dilute sodium hydroxide solution. The organic phase was washed with water, dried, filtered and evaporated. The residue was crystallized from 48 parts of acetonitrile, yielding 2.78 parts (81%) of 1-cyclopentyl-4-(dimethylamino)-*N*-(2,6-dimethylphenyl)-4-piperidinecarboxamide; mp 175.2°C (compound 130).

Following the same procedure and using equivalent amounts of the appropriate starting materials, there were also prepared:

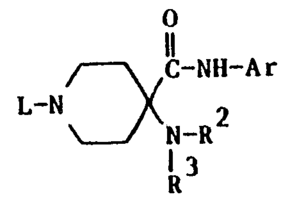

| Comp. No. | L | Ar | $R^2$ | $R^3$ | Base or salt form | Isomeric form | mp. in °C |
|---|---|---|---|---|---|---|---|
| 131 | $i.C_3H_7$ | $C_6H_5$ | $CH_3$ | $CH_3$ | base | - | 114.8 |
| 132 | $i.C_3H_7$ | $2,6-(CH_3)_2-C_6H_3$ | $CH_3$ | $CH_3$ | base | - | 146.0 |
| 133 | $i.C_3H_7$ | $2,6-(CH_3)_2-C_6H_3$ | H | $CH_3$ | base | - | 127.0 |
| 134 | $C_2H_5$ | $2,6-(CH_3)_2-C_6H_3$ | $CH_3$ | $CH_3$ | base | - | 163.5 |
| 135 | cyclopropyl-$CH_2$ | $2,6-(CH_3)_2-C_6H_3$ | $CH_3$ | $CH_3$ | base | - | 170.3 |
| 136 | $n.C_3H_7$ | $2,6-(CH_3)_2-C_6H_3$ | $CH_3$ | $CH_3$ | base | - | 147.8 |
| 137 | $n.C_4H_9$ | $2,6-(CH_3)_2-C_6H_3$ | $CH_3$ | $CH_3$ | base | - | 138.2 |
| 138 | $i.C_3H_7$ | $2,6-(CH_3)_2-C_6H_3$ | $CH_3$ | $C_2H_5$ | base | - | 161.0 |
| 139 | $C_6H_5O-CH_2CH(CH_3)$ | $2,6-(CH_3)_2-C_6H_3$ | $C_2H_5$ | $C_2H_5$ | base | - | 125.4 |
| 140 | $CH_3OCH_2CH(CH_3)$ | $2,6-(CH_3)_2-C_6H_3$ | $C_2H_5$ | $C_2H_5$ | base | - | 131.8 |
| 141 | cyclohexyl | $2,6-(CH_3)_2-C_6H_3$ | $C_2H_5$ | $C_2H_5$ | base | - | 165.9 |
| 142 | 1,3-dihydro-2H-inden-2-yl | $2,6-(CH_3)_2-C_6H_3$ | $C_2H_5$ | $C_2H_5$ | base | - | 154.1 |
| 143 | $i.C_3H_7$ | $2-CH_3,5-Cl-C_6H_3$ | $C_2H_5$ | $C_2H_5$ | base | - | 96.8 |

| Comp. No. | L | Ar | $R^2$ | $R^3$ | Base or salt form | Isomeric form | mp. in °C |
|---|---|---|---|---|---|---|---|
| 144 | $1-C_2H_5OOC,3-CH_3O-4-$piperidinyl | $2,6-(CH_3)_2-C_6H_3$ | $CH_3$ | $CH_3$ | base | cis | 140.3 |
| 145 | $1-C_2H_5OOC-4-$piperidinyl | $2,6-(CH_3)_2-C_6H_3$ | $CH_3$ | $CH_3$ | base | - | 131.5 |
| 146 | $(C_2H_5)_2N(CH_2)_3CH(CH_3)$ | $2,6-(CH_3)_2-C_6H_3$ | $CH_3$ | $CH_3$ | base | - | 98.5 |
| 147 | $1-C_2H_5OOC(CH_2)_2CH(CH_3)$ | $2,6-(CH_3)_2-C_6H_3$ | $CH_3$ | $CH_3$ | base | - | 105.5 |
| 148 | $2-[(C_2H_5OOC)CH_2]$cyclopentyl | $2,6-(CH_3)_2-C_6H_3$ | $CH_3$ | $CH_3$ | base | - | 181.5 |
| 149 | cyclohexyl | $2,6-(CH_3)_2-C_6H_3$ | $CH_3$ | $CH_3$ | base | - | 166.8 |
| 150 | 2-OH-cyclohexyl | $2,6-(CH_3)_2-C_6H_3$ | $CH_3$ | $CH_3$ | base | cis | 158.3 |
| 151 | tetrahydro-4H-pyran-4-yl | $2,6-(CH_3)_2-C_6H_3$ | $CH_3$ | $CH_3$ | base | - | 204.4 |
| 152 | $4-(C_6H_5CH_2O)$cyclohexyl | $2,6-(CH_3)_2-C_6H_3$ | $CH_3$ | $CH_3$ | base | trans | - |
| 153 | $4-(C_6H_5CH_2O)$cyclohexyl | $2,6-(CH_3)_2-C_6H_3$ | $CH_3$ | $CH_3$ | base | cis | - |
| 154 | $C_6H_5CH_2$ | $2,6-(CH_3)_2-C_6H_3$ | $CH_3$ | $CH_3$ | base | - | 171.5 |
| 155 | $CH_3OCH_2CH(CH_3)$ | $2,6-(CH_3)_2-C_6H_3$ | $CH_3$ | $CH_3$ | base | - | 121.2 |
| 156 | $2-CH_3-$cyclohexyl | $2,6-(CH_3)_2-C_6H_3$ | $CH_3$ | $CH_3$ | base | A | 150.6. |

In a similar manner there were also prepared:

4-(acetylmethylamino)-*N*-(2,6-dimethylphenyl)-1-(1-methylethyl)-4-piperidinecarboxamide; mp. 187.5°C (compound 157)

4-(dimethylamino-*N*-(2,6-dimethylphenyl)-1-(3-methylcyclohexyl)-4-piperidinecarboxamide mp 173.8°C (compound 158).

Example 35

A mixture of 8.26 parts of 1,3-cyclohexanedione, 3.47 parts of 4-(dimethylamino)-*N*-(2,6-dimethylphenyl)-4-piperidinecarboxamide and 270 parts of benzene was stirred and refluxed for 5 hours using a water separator. The reaction mixture was evaporated. The residue was purified by column chromatography over silica gel using a mixture of trichloromethane and methanol (95:5 by volume) as eluent. The first fraction was collected and the eluent was evaporated. The residue was stirred in 2,2'-oxybispropane. The product was filtered off and dried, yielding 7.10 parts (64%) of 4-(dimethylamino)-*N*-(2,6-dimethylphenyl)-1-(3-oxo-1-cyclohexenyl)-4-piperidinecarboxamide; mp 270°C (compound 159).

Example 36

A mixture of 5.2 parts of 4-(dimethylamino)-*N*-(2,6-dimethylphenyl)-1-(3-oxo-1-cyclohexenyl)-4-piperidinecarboxamide and 200 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated. The residue was purified by column chromatography over silica gel using a mixture of trichloromethane and methanol, saturated with ammonia, (93:7 by volume) as eluent. The first fraction was collected and the eluent was evaporated. The residue was stirred in 2,2'-oxybispropane. The product was filtered off and dried, yielding 1.34 parts of *cis*-4-(dimethylamino)-*N*-(2,6-dimethylphenyl)-1-(3-hydroxycyclohexyl)-4-piperidinecarboxamide; mp 147.8°C (compound 160).

The second fraction was collected and the eluent was evaporated. The residue was further purified by column chromatography (HPLC) over silica gel using a mixture of trichloromethane and methanol, saturated with ammonia, (90:10 by volume) as eluent. The pure fractions were collected and the eluent was evaporated. The residue was crystallized from acetonitrile. The product was filtered off and dried, yielding 0.64 parts (12%) of *trans*-4-(dimethylamino)-*N*-(2,6-dimethylphenyl)-1-(3-hydroxycyclohexyl)-4-piperidinecarboxamide; mp 219.4°C. (compound 161).

Example 37

A mixture of 2.3 parts of 3-bromo-1-propene, 2.7 parts of 4-(dimethylamino)-*N*-(2,6-dimethylphenyl)-4-piperidinecarboxamide, 1.6 parts of sodium carbonate, 0.1 parts of potassium iodide and 40 parts of 4-methyl-2-pentanone was stirred and refluxed for 4 hours using a water-separator. The reaction mixture was cooled to room temperature and washed with water. The organic phase was dried, filtered and evaporated. The residue was purified by column-chromatography over silica gel using a mixture of trichloromethane and methanol (90:10 by volume) as eluent. The pure fractions were collected and the eluent was evaporated. The residue was crystallized from acetonitrile. The product was filtered off and dried, yielding 1.38 parts (43.8%) of 4-(dimethylamino)-*N*-(2,6-dimethylphenyl)-1-(2-propenyl)-4-piperidinecarboxamide; mp 144.6°C (compound 162).

In a similar manner there were also prepared:

1-[2-(diethylamino)ethyl]-4-(dimethylamino)-*N*-(2,6-dimethylphenyl)-4-piperidinecarboxamide; mp 97.4°C (compound 163);

*N*-(2,6-dimethylphenyl)-1-(1-methylethyl)-4-[(phenylmethyl)amino]-4-piperidinecarboxamide; mp 152.8°C (compound 164);

1-cyclopentyl-*N*-(2,6-dimethylphenyl)-4-[(phenylmethyl)amino]-4-piperidinecarboxamide; mp 171.4°C (compound 165);

*N*-(5-chloro-2-methylphenyl)-1-cyclopentyl-4-(diethylamino)-4-piperidinecarboxamide dihydrochloride hemihydrate; mp 201.2°C (compound 166);

4-(dimethylamino)-*N*-(2,6-dimethylphenyl)-1-(2-hydroxyethyl)-4-piperidinecarboxamide; mp 167.0°C (compound 167);

*trans*-1-[2-[(diethylamino)methyl]cyclohexyl]-4-(dimethylamino)-*N*-(2,6-dimethylphenyl)-4-piperidine carboxamide; mp 144.5—147.1°C (compound 168);

4-(dimethylamino)-*N*-(2,6-dimethylphenyl)-1-(2-pyrimidinyl)-4-piperidinecarboxamide; mp 160.4°C (compound 169);

4-(dimethylamino)-*N*-(2,6-dimethylphenyl)-1-[2-(1-piperidinyl)ethyl]-4-piperidinecarboxamide; mp 144.4°C (compound 170); and

1-[2-(diethylamino)-1-methylethyl]-4-(dimethylamino)-*N*-(2,6-dimethylphenyl)-4-piperidine-carboxamide; mp 121.4°C (compound 171).

Example 38

A mixture of 2 parts of 1-(2-chloroacetyl)piperidine, 2.75 parts of 4-(dimethylamino)-*N*-(2,6-dimethylphenyl)-4-piperidinecarboxamide, 1.96 parts of *N,N*-diethylethanamine and 90 parts of *N,N*-dimethylformamide was stirred for 18 hours at 70°C. The reaction mixture was evaporated. The residue

was purified by column-chromatography over silica gel using a mixture of trichloromethane and methanol (92:8 by volume) as eluent. The first fraction was collected and the eluent was evaporated. The residue was crystallized from acetonitrile, yielding 2.01 parts (50%) of 4-(dimethylamino-*N*-(2,6-dimethylphenyl)-1-[2-oxo-2-(1-piperidinyl)ethyl]-4-piperidinecarboxamide; mp 160.1°C (compound 172).

In a similar manner there were also prepared:

*N*-(2,6-dimethylphenyl)-4-(ethylmethylamino)-1-(1-phenylethyl)-4-piperidinecarboxamide; mp 183.0°C (compound 173);

4-[(2,6-dimethylphenyl)aminocarbonyl)-4-(ethylamino)-α-methyl-1-piperidinecarboxamide; mp 214.4°C (compound 174);

4-(diethylamino)- *N*-(2,6-dimethylphenyl)-1-(1-oxopropyl)-4-piperidinecarboxamide; mp 154.1°C (compound 175);

4-(dimethylamino)-*N*-(2,6-dimethylphenyl)-1-(1-phenylethyl)-4-piperidinecarboxamide; mp 183.3°C (compound 176);

4-(diethylamino)-*N*-(2,6-dimethylphenyl)-1-(3,4,5-trimethoxybenzoyl)-4-piperidinecarboxamide; mp 197.6°C (compound 177);

4-(dimethylamino)-*N*-(2,6-dimethylphenyl)-1-[2-(phenylamino)ethyl]-4-piperidinecarboxamide; mp 157.0°C (compound 178);

1-(1-benzoylethyl)-4-(dimethylamino)-*N*-(2,6-dimethylphenyl)-4-piperidinecarboxamide; mp 162.9°C (compound 179);

4-(dimethylamino)-*N*-(2,6-dimethylphenyl)-4-[(2,6-dimethylphenyl)aminocarbonyl]-1-piperidine-acetamide; mp 202.8°C (compound 180);

4-(dimethylamino)-*N*-(2,6-dimethylphenyl)-1-(3-phenyl-2-propenyl)-4-piperidinecarboxamide; mp 155.5°C (compound 181);

4-(dimethylamino)-4-[(2,6-dimethylphenyl)aminocarbonyl]-1-piperidineacetamide; mp 241.7°C (compound 182);

*trans*-4-(dimethylamino)-4-[(2,6-dimethylphenyl)aminocarbonyl]-*N*-(2-hydroxycyclohexyl)-1-piperidineacetamide monohydrate; mp 171.5°C (compound 183);

4-(dimethylamino)-*N*-(2,6-dimethylphenyl)-1-(3-phenylpropyl)-4-piperidinecarboxamide; mp 107.5°C (compound 184);

1-(cyclohexylcarbonyl)-4-(dimethylamino)-*N*-(2,6-dimethylphenyl)-4-piperidinecarboxamide; mp 175.6°C (compound 185); and

ethyl 4-(dimethylamino)-4-[(2,6-dimethylphenyl)aminocarbonyl]-1-piperidineacetate; mp 127.4°C (compound 186).

Example 39

A mixture of 10.5 parts of *trans*-*N*-(2,6-dimethylphenyl)-1(2-hydroxycyclohexyl)-4-[(phenylmethyl)-amino]-4-piperidinecarboxamide and 200 parts of methanol was hydrogenated at normal pressure and at 50°C with 2 parts of palladium-on-charcoal catalyst 10%. After the calculated amount of hydrogen was taken up, the catalyst was filtered off and the filtrate was evaporated. The residue was crystallized twice from acetonitrile. The product was filtered off and dried, yielding 6.11 parts (73.4%) of *trans*-4-amino-*N*-(2,6-dimethylphenyl)-1-(2-hydroxycyclohexyl)-4-piperidinecarboxamide; mp 174.8°C (compound 187).

In a similar manner there were also prepared:

4-amino-*N*-(2,6-dimethylphenyl)-1-(1-methylethyl)-4-piperidinecarboxamide; mp 102.9°C (compound 188);

*N*-(2,6-dimethylphenyl)-4-(ethylamino)-1-(1-methylethyl)-4-piperidinecarboxamide; mp 145.8°C (compound 189); and

4-amino-1-cyclopentyl-*N*-(2,6-dimethylphenyl)-4-piperidinecarboxamide; mp 124.1°C (compound 190).

Example 40

A mixture of 11 parts of 1-[3-bis(phenylmethyl)amino]-2-hydroxypropyl]-4-(dimethylamino)-*N*-(2,6-dimethylphenyl)-4-piperidinecarboxamide and 120 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated. The residue was crystallized from acetonitrile. The product was filtered off and dried, yielding 5.7 parts (78%) of 1-(3-amino-2-hydroxypropyl)-4-(dimethylamino)-*N*-(2,6-dimethylphenyl)-4-piperidinecarboxamide; mp 156.2°C (compound 191).

Example 41

A mixture of 5.3 parts of 1-[3-[bis(phenylmethyl)-amino]-2-hydroxypropyl]-4-(dimethylamino)-*N*-(2,6-dimethylphenyl)-4-piperidinecarboxamide, 6.4 parts of a solution of 2-propanol saturated with hydrogen chloride and 120 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated. The residue was dissolved in water and the solution was alkalized with sodium carbonate. The product was extracted with trichloromethane. The extract was washed with water, dried, filtered and evaporated. The oily residue was purified by column chromatography over silica gel using a mixture of trichloromethane and methanol (90:10 by volume)

saturated with ammonia, as eluent. The pure fractions were collected and the eluent was evaporated. The oily residue solidified on triturating in 2,2'-oxybispropane. The produce was filtered off and dried, yielding 2.67 parts (60.9%) of 4-(dimethylamino)-*N*-(2,6-dimethylphenyl)-1-[2-hydroxy-3-[(phenylmethyl)amino]-propyl-4-piperidinecarboxamide; mp 130.7°C (compound 192).

Example 42

A mixture of 20 parts of an acetaldehyde solution in tetrahydrofuran, 5.5 parts of 1-(3-amino-2-hydroxypropyl)-4-(dimethylamino)-*N*-(2,6-dimethylphenyl)-4-piperidinecarboxamide, 1 part of a solution of thiophene in ethanol and 120 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated. The oily residue was purified by column chromatography over silica gel using a mixture of trichloromethane and methanol (90:10 by volume) saturated with ammonia, as eluent. The pure fractions were collected and the eluent was evaporated. The oily residue solidified on triturating in 2,2'-oxybispropane. The product was filtered off and dried, yielding 1.87 parts (29%) of 1-[3-(diethylamino)-2-hydroxypropyl]-4-(dimethylamino)-*N*-(2,6-dimethylphenyl)-4-piperidinecarboxamide; mp 113.7°C (compound 193).

Example 43

A mixture of 1.7 parts of 4-(dimethylamino)-*N*-(2,6-dimethylphenyl)-1-(2-pyrimidinyl)-4-piperidinecarboxamide, 1 part of acetic acid and 120 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated. The solid residue was taken up in alkaline water and the product was extracted with trichloromethane. The extract was dried, filtered and evaporated. The solid residue was crystallized from acetonitrile. The product was filtered off, washed with 2,2'-oxybispropane and dried, yielding 0.51 parts (30%) of 4-(dimethylamino)-*N*-(2,6-dimethylphenyl)-1-(1,4,5,6-tetrahydro-2-pyrimidinyl)-4-piperidinecarboxamide; mp 238.1°C (compound 194).

Example 44

A mixture of 7.2 parts of 1-(1-benzoylethyl)-4-(dimethylamino)-*N*-(2,6-dimethylphenyl)-4-piperidinecarboxamide, 0.6 parts of acetic acid, 160 parts of methanol and 100 parts of water was hydrogenated at normal pressure and at room temperature with 2 parts of platinum-on-charcoal catalyst 5%. After the calculated amount of hydrogen was taken up, the catalyst was filtered off and the filtrate was concentrated. The concentrate was treated with a dilute sodium hydroxide solution. The product was extracted twice with dichloromethane. The combined extracts were washed with water, dried, filtered and evaporated. The residue was purified by column chromatography over silica gel using a mixture of trichloromethane and methanol, saturated with ammonia, (93:7 by volume) as eluent. The first fraction was collected and the eluent was evaporated. The residue was crystallized twice from acetonitrile, yielding 3.44 parts (49.4%) of 4-(dimethylamino)-*N*-(2,6-dimethylphenyl)-1-(2-hydroxy-1-methyl-2-phenylethyl)-4-piperidinecarboxamide; 185.8°C (compound 195).

Example 45

A mixture of 1.4 parts of *trans*-4-(dimethylamino)-*N*-(2,6-dimethylphenyl)-1-[4-(phenylmethoxy)cyclohexyl]-4-piperidinecarboxamide and 120 parts of methanol was hydrogenated at normal pressure and at room temperature with 1 part of palladium-on-charcoal catalyst 10%. After the calculated amount of hydrogen was taken up, the catalyst was filtered off and the filtrate was evaporated. The residue was purified by column chromatography over silica gel using a mixture of trichloromethane and methanol (92:2 by volume), saturated with ammonia, as eluent. The first fraction was collected and the eluent was evaporated. The residue was stirred in 2,2'-oxybispropane. The product was filtered off and dried, yielding 0.38 parts (34%) of *trans*-4-(dimethylamino)-*N*-(2,6-dimethylphenyl)-1-(4-hydroxycyclohexyl)-4-piperidinecarboxamide; mp 184.3°C (compound 196).

In a similar manner there were also prepared:

*cis*-4-(dimethylamino)-*N*-(2,6-dimethylphenyl)-1-(4-hydroxycyclohexyl)-4-piperidinecarboxamide; mp 215.2°C (compound 197);

4-(dimethylamino)-1-(2,4-dihydroxybutyl)-*N*-(2,6-dimethylphenyl)-4-piperidinecarboxamide mp. 175.1°C (compound 198);

1-(2,4-dihydroxy-2-methylbutyl)-4-(dimethylamino)-*N*-(2,6-dimethylphenyl)-4-piperidinecarboxamide mp 162.8°C (compound 199);

(±)-[1-(1α,2β,5β)]-1-(2,5-dihydroxycyclohexyl)-4-(dimethylamino-*N*-(2,6-dimethylphenyl)-4-piperidinecarboxamide; mp 188.5°C (compound 200);

(±)-[1-(1α,2β,5α)]-1-(2,5-dihydroxycyclohexyl)-4-(dimethylamino)-*N*-(2,6-dimethylphenyl)-4-piperidinecarboxamide mp 187.0°C (compound 201); and

(±)-1-(1α,2β,4α)]-1-(2,4-dihydroxycyclohexyl)-4-(dimethylamino-*N*-(2,6-dimethylphenyl)-4-piperidinecarboxamide; mp 212.0°C (compound 202).

Example 46

To a stirred and cooled mixture of 5.6 parts of *trans*-4-(dimethylamino)-*N*-(2,6-dimethylphenyl)-1-(2-hydroxycyclohexyl)-4-piperidinecarboxamide, 2.05 parts of *N,N*-dimethyl-4-pyridinamine and 65 parts of dichloromethane were added 1.5 parts of propanoyl chloride and stirring was continued for 3 hours at room temperature. 4 Parts of methanol and water were added and the layers were separated. The organic layer was washed with water, dried, filtered and evaporated. The residue was purified by column chromatography over silica gel using a mixture of trichloromethane and methanol (97:3 by volume) as eluent. The first fraction was collected and the eluent was evaporated. The residue was crystallized from acetonitrile. The product was filtered off and dried, yielding 4.32 parts (67%) of *trans*-[2-[4-(dimethylamino)-4-[(2,6-dimethylphenyl)aminocarbonyl]-1-piperidinyl]cyclohexyl] propanoate; mp 174.3°C (compound 203).

In a similar manner there were also prepared:

*trans*-4-(dimethylamino)-*N*-(2,6-dimethylphenyl)-1-(2-hydroxycyclohexyl)-4-piperidinecarboxamide acetate (ester); mp 168.4°C (compound 204);

*trans*-[2-[4-(dimethylamino)-4-[(2,6-dimethylphenyl)aminocarbonyl]-1-piperidinyl]cyclohexyl]benzoate; mp 160.9°C (compound 205).

Example 47

To a stirred and cooled (ice bath) mixture of 8.2 parts of *trans*-4-(dimethylamino)-*N*-(2,6-dimethylphenyl)-1-(2-hydroxycyclohexyl)-4-piperidinecarboxamide, 3.1 parts of *N,N*-dimethyl-4-pyridinamine and 260 parts of dichloromethane was added a solution of 6 parts of (+)-α-methoxy-α-(trifluoromethyl)benzeneacetyl chloride in 65 parts of dichloromethane. The whole was stirred over weekend at room temperature. 8 Parts of methanol were added. The solution was washed twice with 50 parts of water. The organic layer was dried, filtered and evaporated. The residue was purified by column chromatography over silica gel using a mixture of trichloromethane and methanol (98:2 by volume) as eluent.

The first fraction was collected and the eluent was evaporated. The residue was crystallized from 2-propanol. The product was filtered off and dried, yielding 2.8 parts (21%) of ($A^-$)-[2-[4-(dimethylamino)-4-[(2,6-dimethylphenyl)aminocarbonyl]-1-piperidinyl]cyclohexyl]-α-methoxy-α-(trifluoromethyl)benzeneacetate; mp 190.0°C, $\lambda_{589}$ = −22.5715° (c = 1% in trichloromethane) (compound 206).

The second fraction was collected and the eluent was evaporated. The residue was crystallized from 2-propanol. The product was filtered off and dried, yielding 1.7 parts (13%) of ($B^+$)-[2-[4-(dimethylamino)-4-[(2,6-dimethylphenyl)aminocarbonyl]-1-piperidinyl]cyclohexyl-α-methoxy-α-(trifluoromethyl)benzeneacetate; mp 176.7°C, $\lambda_{589}$ = +63.0332° (c = 1% in trichloromethane) (compound 207).

In a similar manner there were also prepared:

($A^+$)-[2-[4-(dimethylamino)-4-[(2,6-dimethylphenyl)aminocarbonyl]-1-piperidinyl]cyclohexyl]-α-methoxy-α-(trifluoromethyl)benzeneacetate mp 200.2°C, $\lambda_{589}$ = +22.02° (c = 1% in trichloromethane) (compound 208); and

($B^-$)-[2-[4-(dimethylamino)-4-[(2,6-dimethylphenyl)aminocarbonyl]-1-piperidinyl]cyclohexyl]-α-methoxy-α-(trifluoromethyl)benzeneacetate mp 176.0°C, $\lambda_{589}$ = −64.35° (c = 1% in trichloromethane) (compound 209).

Example 48

A mixture of 5.6 parts of ethyl *trans*-4-(dimethylamino)-4-[[(2,6-dimethylphenyl)amino]carbonyl]-3′-hydroxy-[1,4′-bipiperidine]-1′-carboxylate, 7.0 parts of potassium hydroxide and 60 parts of 2-propanol was stirred and refluxed for 20 hours. The reaction mixture was evaporated and 100 parts of water were added to the residue. The whole was concentrated to half its volume. After cooling, the product was extracted with dichloromethane. The extract was washed with water, dried, filtered and evaporated. The oily residue was purified by column chromatography over silica gel using a mixture of trichloromethane and methanol (90:10 by volume), saturated with ammonia, as eluent. The pure fractions were collected and the eluent was evaporated. The residue was crystallized from acetonitrile. The product was filtered off and dried, yielding 3.09 parts (67%) of *trans*-4-(dimethylamino)-*N*-(2,6-dimethylphenyl)-3′-hydroxy-[1,4′-bipiperidine]-4-carboxamide; mp 166.6°C (compound 210).

Following the same procedure and starting from the corresponding 3-piperidinyl analog there was also prepared:

*trans*-4-(dimethylamino)-*N*-(2,6-dimethylphenyl)-4′-hydroxy-[1,3′-bipiperidine]-4-carboxamide; mp 180.6°C (compound 211).

Example 49

A mixture of 10 parts of an acetaldehyde solution 2N in tetrahydrofuran, 4 parts of *trans*-4-(dimethylamino)-*N*-(2,6-dimethylphenyl)-4′-hydroxy-[1,3′-bipiperidine]-4-carboxamide, 1 part of a solution of thiophene in ethanol 4% and 80 parts of methanol was hydrogenated at normal pressure and at room temperature with 2 parts of palladium-on-charcoal catalyst 10%. After the calculated amount of hydrogen was taken up, the catalyst was filtered off the filtrate was evaporated. The solid residue was crystallized from acetonitrile. The product was filtered off and dried, yielding 3.0 parts (68.6%) of *trans*-4-(dimethylamino)-*N*-(2,6-dimethylphenyl)-1′-ethyl-4′-hydroxy[1,3′-bipiperidine]-4-carboxamide; mp 173.3°C (compound 212).

In a similar manner there were also prepared:

4-(dimethylamino)-*N*-(2,6-dimethylphenyl)-1'-ethyl-[1,4'-bipiperidine]-4-carboxamide; mp 147.3°C (compound 213);

*trans*-4-(dimethylamino)-*N*-(2,6-dimethylphenyl)-1'-ethyl-3'-hydroxy[1,4'-bipiperidine]-4-carboxamide; mp 182.1°C (compound 214); and

*cis*-4-(dimethylamino)-*N*-(2,6-dimethylphenyl)-1'-ethyl-3'-methoxy-[1,4'-bipiperidine]-4-carboxamide; mp 156.5°C (compound 215).

Example 50

A mixture of 3.9 parts of *trans*-4-(dimethylamino)-*N*-(2,6-dimethylphenyl)-1-[2-[(phenylmethyl)amino]-cyclohexyl]-4-piperidinecarboxamide and 200 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated. The residue was stirred in warm 2,2'-oxybispropane. After cooling, the precipitated product was filtered off, dried and purified by column chromatography over silica gel using a mixture of trichloromethane and methanol saturated with ammonia (90:10 by volume) as eluent. The pure fractions were collected and the eluent was evaporated. The residue was crystallized from acetonitrile. The product was filtered off and dried in a dry-pistol, yielding 1.94 parts (51.8%) of *trans*-1-(2-aminocyclohexyl)-4-(dimethylamino)-*N*-(2,6-dimethylphenyl)-4-piperidine-carboxamide; mp. 168.3°C (compound 216).

In a similar manner there were also prepared:

*trans*-1-(2-aminocyclopentyl)-4-(dimethylamino)-*N*-(2,6-dimethylphenyl)-4-piperidinecarboxamide mp. 131.5°C (compound 217).

Example 51

To a stirred mixture of 1.2 parts of *trans*-1-(2-aminocyclohexyl)-4-(dimethylamino)-*N*-(2,6-dimethylphenyl)-4-piperidinecarboxamide and 50 parts of acetic acid were added dropwise 0.3 parts of acetic acid anhydride. Upon completion, stirring was continued overnight at room temperature. The reaction mixture was evaporated. Water was added and the whole was treated with a sodium hydroxide solution 50%. The product was extracted with dichloromethane. The obtained emulsion was filtered over Hyflo. From the filtrate, the layers were separated. The aqueous phase was extracted with dichloromethane. The combined organic layers were washed with water, dried, filtered and evaporated. The residue was boiled in acetonitrile for 2 hours. After cooling, the product was filtered off and dried in a dry-pistol, yielding 0.88 parts (66.6%) of *trans*-1-[2-(acetylamino)cyclohexyl]-4-(dimethylamino)-*N*-(2,6-dimethylphenyl)-4-piperidinecarboxamide; mp. 275.3°C (compound 218).

In a similar manner there were also prepared:

*trans*-1-[2-(acetylamino)cyclopentyl]-4-(dimethylamino)-*N*-(2,6-dimethylphenyl)-4-piperidinecarboxamide mp. 199.4°C (compound 219).

Example 52

To a stirred mixture of 2.6 parts of *trans*-1-(2-aminocyclohexyl)-4-(dimethylamino)-*N*-(2,6-dimethylphenyl)-4-piperidinecarboxamide, 1.35 parts of N,N-diethylethanamine and 75 parts of trichloromethane were added dropwise 0.9 parts of methanesulfonyl chloride while cooling. Upon completion, stirring was continued overnight at room temperature. Trichloromethane was added till homogeneous. The whole was washed once with water. The organic layer was dried, filtered and evaporated. The residue was crystallized from ethanol. The product was filtered off and dried, yielding 2.48 parts (80.0%) of *trans*-4-(dimethyl-amino)-*N*-(2,6-dimethylphenyl)-1-[2-[(methylsulfonyl)amino]-cyclohexyl]-4-piperidinecarboxamide; mp. 234.6°C (compound 220).

In a similar manner there were also prepared:

ethyl *trans*-[2-[4-(dimethylamino)-4-[(2,6-dimethylphenyl)aminocarbonyl]-1-piperidinyl]cyclohexyl]-carbamate mp. 243.7°C (compound 221);

*trans*-4-(dimethylamino)-*N*-(2,6-dimethylphenyl)-1-[2-[(1-oxopropyl)amino]-cyclohexyl]-4-piperidine-carboxamide; mp. 269.7°C (compound 222); and

*trans*-1-[2-(benzoylamino)cyclohexyl]-4-(dimethylamino)-*N*-(2,6-dimethylphenyl)-4-piperidinecarboxamide mp. 248.9°C (compound 223).

Example 53

To a stirred solution of 3.16 parts of *trans*-1-(2-aminocyclohexyl)-4-(dimethylamino)-*N*-(2,6-dimethylphenyl)-4-piperidinecarboxamide in 160 parts of water were added dropwise 5.52 parts of concentrate hydrochloric acid. Upon completion, a solution of 8.16 parts of potassium cyanate in 40 parts of water was added dropwise. Upon completion, stirring was continued for 10 days at room temperature. The reaction mixture was treated with ammonium hydroxide. The product was extracted twice with trichloromethane. The organic layer was washed with water, dried, filtered and evaporated. The residue was crystallized from acetonitrile. The product was filtered off and boiled in acetonitrile. The product was filtered off while hot and dried, yielding 0.77 parts (22%) of *trans*-1-[2-[(aminocarbonyl)amino]cyclohexyl]-4-(dimethylamino)-*N*-(2,6-dimethylphenyl)-4-piperidinecarboxamide; mp. 243.2°C. (compound 224).

Example 54

22 Parts of *trans*-4-(dimethylamino)-*N*-(2,6-dimethylphenyl)-1-[2-[(phenylmethyl)amino]cyclopentyl]-4-piperidinecarboxamide were dissolved in 80 parts of warm methanol. The whole was cooled in an ice bath while stirring. Then there were added 10 parts of cooled oxirane (0°C). The mixture was stirred in that ice bath overnight while the temperature was allowed to rise spontaneously to room temperature. The reaction mixture was evaporated, cooled and extracted three times with trichloromethane. The combined extracts were dried, filtered and evaporated. The residue was purified by column chromatography over silica gel using a mixture of trichloromethane and methanol, saturated with ammonia, (97:3 by volume) as eluent. The third fraction was collected and the eluent was evaporated. The residue was crystallized from 2,2'-oxybispropane, yielding 3.7 parts (15%) of *trans*-4-(dimethylamino)-*N*-(2,6-dimethylphenyl)-1-[2-[(2-hydroxyethyl)(phenylmethyl)amino]cyclopentyl]-4-piperidinecarboxamide; mp. 161.3°C. (compound 225).

Example 55

A mixture of 2 parts of *trans*-1-(2-aminocyclohexyl)-4-(dimethylamino)-*N*-(2,6-dimethylphenyl)-4-piperidinecarboxamide, 2 parts of poly(oxymethylene), 1 part of a solution of thiophene in methanol 4% and 200 parts of methanol was hydrogenated at normal pressure and at 50°C with 2 parts of palladium-on-charcoal catalyst 10%. After the calculated amount of hydrogen was taken up, the catalyst was filtered off and the filtrate was evaporated. The residue was taken up in water. The product was extracted twice with dichloromethane. The aqueous phase was salted out with sodium chloride and extracted with dichloromethane. The combined extracts were dried, filtered and evaporated. The residue was purified by column chromatography over silica gel using a mixture of trichloromethane and methanol, saturated with ammonia (90:10 by volume) as eluent. The first fraction was collected and the eluent was evaporated. The residue was crystallized from acetonitrile. The product was filtered off and dried, yielding 1.13 parts (52.3%) of *trans*-4-(dimethylamino)-1-[2-(dimethylamino)cyclohexyl]-*N*-(2,6-dimethylphenyl)-4-piperidine-carboxamide; mp. 170.8°C. (compound 226).

In a similar manner there were also prepared:

*trans*-4-(dimethylamino)-*N*-(2,6-dimethylphenyl)-1-[2-[methyl(phenylmethyl)-amino]cyclohexyl]-4-piperidinecarboxamide (compound 227);

*trans*-4-(dimethylamino)-*N*-(2,6-dimethylphenyl)-1-[2-[ethyl(phenylmethyl)amino]cyclohexyl]-4-piperidinecarboxamide as a residue (compound 228).

Example 56

A mixture of 2.4 parts of *trans*-4-(dimethylamino)-*N*-(2,6-dimethylphenyl)-1-[2-[methyl(phenylmethyl)-amino]cyclohexyl]-4-piperidinecarboxamide and 120 parts of methanol was hydrogenated at normal pressure and at 50°C with 1 part of palladium-on-charcoal catalyst 10%. After the calculated amount of hydrogen was taken up, the catalyst was filtered off and the filtrate was evaporated. The residue was purified by column chromatography over silica gel using a mixture of trichloromethane and methanol saturated with ammonia (95:5 by volume) as eluent. The pure fractions were collected and the eluent was evaporated. The residue was crystallized from acetonitrile. The product was filtered off and dried, yielding 1.37 parts (71%) of *trans*-4-(dimethylamino)-*N*-(2,6-dimethylphenyl)-1-[2-(methylamino)cyclohexyl]-4-piperidinecarboxamide; mp. 188.4°C. (compound 229).

In a similar manner there were also prepared:

*trans*-4-(dimethylamino)-*N*-(2,6-dimethylphenyl)-1-[2-(ethylamino)cyclohexyl]-4-piperidinecarboxamide mp. 176.1°C (compound 230); and

*trans*-4-(dimethylamino)-*N*-(2,6-dimethylphenyl)-1-[2-[(2-hydroxyethyl)amino]cyclopentyl]-4-piperidinecarboxamide mp. 168.8°C (compound 231).

Example 57

To a stirred solution of 1.4 parts of *N*-(2,6-dimethylphenyl)-4-(methylamino)-1-(1-methylethyl)-4-piperidinecarboxamide in 60 parts of 2-propanone were added a few drops of 2-propanol saturated with hydrogen chloride. The precipitated product was filtered off and stirred for 2 hours in 2-propanone and a small amount of water. It was filtered off again and dried at the air, yielding 1.40 parts of *N*-(2,6-dimethylphenyl)-4-(methylamino)-1-(1-methylethyl)-4-piperidinecarboxamide dihydrochloride dihydrate; mp. 219.8°C (compound 232).

Example 58

A mixture of 6 parts of *trans*-4-(dimethylamino)-*N*-(2,6-dimethylphenyl)-1-(2-hydroxycyclohexyl)-4-piperidinecarboxamide, 1.2 parts of (−)-[S-(R*,R*)]-2,3-dihydroxybutanedioic acid, 120 parts of acetonitrile and 120 parts of 2-propanol was stirred and refluxed for about 10 minutes. The clear solution was allowed to crystallize. The product was filtered off and recrystallized from 200 parts of a mixture of acetonitrile and 2-propanol (50:50 by volume). The product was filtered off and recrystallized again from 144 parts of a mixture of acetonitrile and 2-propanol, yielding 2.58 parts (69%) of (−)-*trans*-4-(dimethylamino)-*N*-(2,6-dimethylphenyl) - 1 - (2 - hydroxycyclohexyl) - 4 - piperidinecarboxamide (−)-[S-(R*,R*)] - 2,3 - dihydroxybutanedioate(2:1); $[\alpha]_{589} = -7.1251°$ (c = 1% in methanol) (compound 233).

A solution of 2.58 parts of (−)*trans*-4-(dimethylamino)-*N*-(2,6-dimethylphenyl)-1-(2-hydroxycyclohexyl)-4-piperidinecarboxamide (−)-[S-(R*,R*)]-2,3-dihydroxybutanedioate(2:1) in 100 parts of water was

treated with sodium carbonate. The product was extracted twice with dichloromethane. The combined organic layers were washed with 50 parts of water, dried, filtered and evaporated. The residue was crystallized from 2-propanol. The product was filtered off and dried in a dry-pistol, yielding 1.55 parts (72%) of (+)*trans*-4-(dimethylamino)-*N*-(2,6-dimethylphenyl)-1-(2-hydroxycyclohexyl)-4-piperidinecarboxamide; mp. 167.8°C; $[\alpha]_{589} = +2.37°$ (c = 1% in methanol) (compound 234).

Example 59

A mixture of 10 parts of *trans*-4-(dimethylamino)-*N*-(2,6-dimethylphenyl)-1-(2-hydroxycyclohexyl)-4-piperidinecarboxamide, 2.05 parts of (+)-[R-(R*,R*)]-2,3-dihydroxybutanedioic acid and 240 parts of a mixture of 2-propanol and acetonitrile (50:50 by volume) was stirred and refluxed for 15 minutes. The clear solution was allowed to crystallize. The product was filtered off and recrystallized five times from resp. 360, 360, 304, 288 and 240 parts of a mixture of acetonitrile and 2-propanol (50:50 by volume), yielding 3.49 parts of (+)-*trans*-4-(dimethylamino)-*N*-(2,6-dimethylphenyl)-1-(2-hydroxycyclohexyl)-4-piperidinecarboxamide (+)-[R-(R*,R*)]-2,3-dihydroxybutanedioate (2:1); $[\alpha]_{589} = +7{,}8156°$ (c = 1% in $CH_3OH$) (compound 235).

A solution of 3.49 parts of (+)-*trans*-4-(dimethylamino)-*N*-(2,6-dimethylphenyl)-1-(2-hydroxycyclohexyl)-4-piperidinecarboxamide (+)-[R-(R*,R*)]-2,3-dihydroxybutanedioate (2:1) in 150 parts of water was treated with sodium carbonate. The product was extracted twice with dichloromethane. The combined extracts were washed with 80 parts of water, dried, filtered and evaporated. The residue was crystallized from 2-propanol. The product was filtered off and dried, yielding 2.09 parts (71%) of (−)-*trans*-4-(dimethylamino)-*N*-(2,6-dimethylphenyl)-1-(2-hydroxycyclohexyl)-4-piperidinecarboxamide; mp. 167.2°C (compound 236).

Example 60

To 80 parts of 2-propanol (which were cooled to −5°C) were added 3.4 parts of methanamine. 6.6 Parts of acetic acid were added dropwise at 0°C. Upon completion, the whole was stirred for 15 minutes at room temperature. After the addition of 14.05 parts of ethyl 3-oxo-1-piperidinecarboxylate, stirring was continued for 30 minutes at room temperature. 10 Parts of 2-isocyano-1,3-dimethylbenzene were added and the whole was stirred for 3 hours at reflux. The reaction mixture was evaporated, yielding 28 parts (98%) of ethyl 3-(acetylmethylamino)-3-[(2,6-dimethylphenyl)aminocarbonyl]-1-piperidinecarboxylate as a residue (compound 237).

A mixture of 28 parts of ethyl 3-(acetylmethylamino)-3-[(2,6-dimethylphenyl)aminocarbonyl]-1-piperidinecarboxylate, 30 parts of potassium hydroxide and 100 parts of 2-propanol was stirred and refluxed overnight. The reaction mixture was evaporated. The residue was taken up in water three times and the latter was evaporated each time. The product was extracted twice with dichloromethane. The combined extracts were washed once with water, dried, filtered and evaporated. The residue was purified by column chromatography over silica gel using a mixture of trichloromethane and methanol (93:7 by volume), saturated with ammonia, as eluent. The first fraction was collected and the eluent was evaporated. The residue was stirred in 2,2'oxybispropane, yielding 8.52 parts of *N*-(2,6-dimethylphenyl)-3-(methylamino)-3-piperidinecarboxamide (compound 238).

To a stirred and cooled (−10°C) mixture of 8.52 parts of *N*-(2,6-dimethylphenyl)-3-(methylamino)-3-piperidinecarboxamide, 6.79 parts of *N,N*-diethylethanamine and 300 parts of trichloromethane were added dropwise 6.3 parts of (phenylmethyl) carbonochloridate at 0°C. Upon completion, stirring was continued first for 2 hours in an ice-bath and further overnight at room temperature. The reaction mixture was washed twice with water. The organic layer was dried, filtered and evaporated. The residue was purified by column chromatography over silica gel using a mixture of trichloromethane and methanol (96:4 by volume) as eluent. The first fraction was collected and the eluent was evaporated. The residue was solidified in 2,2'-oxybispropane. The product was filtered off and dried, yielding 10.7 parts (82%) of (phenylmethyl) 3-[(2,6-dimethylphenyl)aminocarbonyl]-3-(methylamino)-1-piperidinecarboxylate; mp. 107.6°C (compound 239).

5.67 Parts of sodium borohydride were suspended in a solution of 10.2 parts of (phenylmethyl) 3-[(2,6-dimethylphenyl)aminocarbonyl]-3-(methylamino)-1-piperidinecarboxylate in 90 parts of tetrahydrofuran. This suspension was added portionwise to a stirred and cooled mixture of 7.55 parts of a formaldehyde solution 40% in water, 9.44 parts of a sulfuric acid solution 3M and 90 parts of tetrahydrofuran at about 10°C and at a pH below 5 (exothermic reaction: temp. rose to about 30°C). Upon completion, stirring was continued for 3 hours at room temperature. Water was added and the whole was treated with a sodium hydroxide solution 50%. After stirring for 5 minutes, the layers were separated and the aqueous phase was extracted with dichloromethane. The combined organic phases were evaporated and the residue was taken up in trichloromethane. The organic phase was washed with water, dried, filtered and evaporated. The residue was purified by column chromatography over silica gel using a mixture of trichloromethane and methanol (96:4 by volume) as eluent. The pure fractions were collected and the eluent was evaporated, yielding 11.52 parts of (phenylmethyl) 3-(dimethylamino)-*N*-[(2,6-dimethylphenyl)aminocarbonyl]-1-piperidinecarboxylate as a residue (compound 240).

A mixture of 6.4 parts of (phenylmethyl) 3-(dimethylamino)-*N*-[(2,6-dimethylphenyl)aminocarbonyl]-1-piperidinecarboxylate and 120 parts of methanol was hydrogenated at normal pressure and at 50°C with 2 parts of palladium-on-charcoal catalyst 10%. After the calculated amount of hydrogen was taken up, the

catalyst was filtered off and the filtrate was evaporated. The residue was stirred in 2,2′-oxybispropane. The product was filtered off and dried, yielding 3.40 parts (77%) of 3-(dimethylamino)-*N*-(2,6-dimethylphenyl)-3-piperidinecarboxamide (compound 241).

A mixture of 1.2 parts of 7-oxabicyclo[4.1.0]heptane, 2.75 parts of 3-(dimethylamino)-*N*-(2,6-dimethylphenyl)-3-piperidinecarboxamide and 45 parts of a mixture of water and ethanol (50:50 by volume) was stirred and refluxed overnight. The reaction mixture was evaporated and water was added to the residue. The product was extracted twice with dichloromethane. The combined extracts were dried, filtered and evaporated. The residue was purified by column chromatography over silica gel using a mixture of trichloromethane and methanol (95:5 by volume) as eluent. The pure fractions were collected and the eluent was evaporated, yielding 2.55 parts (68.3%) of *trans*-3-(dimethylamino)-*N*-(2,6-dimethylphenyl)-1-(2-hydroxycyclohexyl)-3-piperidinecarboxamide; mp. 174.0°C (compound 242).

Example 61

A solution of 9.88 parts of (phenylmethyl) 4-[(2,6-dimethylphenyl)aminocarbonyl]-4-(methylamino)-1-piperidinecarboxylate and 0.9 parts of poly(oxymethylene) in 36 parts of *N,N*-dimethylformamide was stirred and heated slowly in an oil-bath to 100°C over a 1 hour period. After stirring for 2 hours at 100°C, another 0.9 parts of poly(oxymethylene) was added and stirring was continued while heating to 140°C. The whole was further stirred overnight at 140°C. The reaction mixture was evaporated in vacuo and the oily residue was dissolved in 375 parts of trichloromethane. The solution was washed with 100 parts of water. The aqueous phase was extracted with trichloromethane. The combined organic phases were washed with water, dried, filtered and evaporated. The oily residue was boiled three times in 175 parts of 2,2′-oxybispropane and the latter was decanted each time. The combined 2,2′-oxybispropane phases were stirred with activated charcoal. The latter was filtered off and the filtrate was evaporated in vacuo. The residue was dissolved in methylbenzene and the whole was evaporated again in vacuo, yielding 6 parts (58.9%) of phenylmethyl 3-(2,6-dimethylphenyl)-1-methyl-4-oxo-1,3,8-triazaspiro[4.5]decane-8-carboxylate as an oily residue (compound 243).

In a similar manner there was also prepared:

(phenylmethyl) 3-(2,6-dimethylphenyl)-1-ethyl-4-oxo-1,3,8-triazaspiro[4,5]decan-4-one as a residue (compound 244).

Example 62

A mixture of 6 parts of (phenylmethyl) 3-(2,6-dimethylphenyl)-1-methyl-4-oxo-1,3,8-triazaspiro[4,5]-decane-8-carboxylate and 120 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated. The residue was dissolved in dichloromethane and the solution was washed with a sodium hydroxide solution 10%. The organic phase was dried, filtered and evaporated in vacuo, yielding 3.7 parts (90.2%) of 3-(2,6-dimethylphenyl)-1-methyl-1,3,8-triazaspiro[4,5]decan-4-one as an oily residue (compound 245).

In a similar manner there was also prepared:

3-(2,6-dimethylphenyl)-1-ethyl-1,3,8-triazaspiro[4,5]decan-4-one as a residue (compound 246).

Example 63

A solution of 1.33 parts of 7-oxabicyclo[4,1,0]heptane and 3.7 parts of 3-(2,6-dimethylphenyl)-1-methyl-1,3,8-triazaspiro[4,5]decan-4-one in 24 parts of ethanol and 30 parts of water was stirred and refluxed in an oil-bath (110°C) for 24 hours. The reaction mixture was evaporated in vacuo. The solid residue was purified by column chromatography over silica gel using a mixture of trichloromethane and methanol (97:3 by volume), saturated with ammonia, as eluent. The pure fractions were collected and the eluent was evaporated. The solid residue was crystallized from acetonitrile, yielding 2.6 parts (52%) of *trans*-3-(2,6-dimethylphenyl)-8-(2-hydroxycyclohexyl)-1-methyl-1,3,8-triazaspiro[4,5]decan-4-one; mp. 169.0°C (compound 247).

Example 64

A mixture of 4 parts of 2-propanone, 3.9 parts of 3-(2,6-dimethylphenyl)-1-ethyl-1,3,8-triazaspiro[4,5]-decan-4-one, 1 part of a solution of thiophene in ethanol 5% and 120 parts of methanol was hydrogenated at normal pressure and at room temperature with 2 parts of palladium-on-charcoal catalyst 10%. After the calculated amount of hydrogen was taken up, the catalyst was filtered off and the filtrate was evaporated. The residue was purified by column chromatography over silica gel using a mixture of trichloromethane and methanol (90:10 by volume) as eluent. The desired fractions were collected and the eluent was evaporated. The residue was triturated in 2,2′-oxybispropane. The product was filtered off and dried, yielding 0.36 parts (7.3%) of 3-(2,6-dimethylphenyl)-1-ethyl-8-(1-methylethyl)-1,3,8-triazaspiro[4,5]decan-4-one monohydrochloride; mp. 250.8°C (compound 248). On the column the product was further eluated with a mixture of trichloromethane and methanol (90:10 by volume). The desired fractions were collected and the eluent was evaporated. The residue was crystallized from 2,2′-oxybispropane. After cooling (2-propanone/$CO_2$ bath) the product was filtered off and dried, yielding 0.85 parts (19%) of 3-(2,6-dimethylphenyl)-1-ethyl-8-(1-methylethyl)-1,3,8-triazaspiro[4,5]decan-4-one; mp. 114.2°C (compound 249).

Example 65

To a stirred mixture of 8.25 parts of 4-(dimethylamino)-*N*-(2,6-dimethylphenyl)-4-piperidinecarboxamide, 6.36 parts of sodium carbonate and 180 parts of tetrahydrofuran were added dropwise 2.49 parts of 2-chloroacetonitrile. Upon completion, stirring was continued overnight at reflux. After cooling, the precipitated product was filtered off and washed with trichloromethane. The organic layer was dried, filtered and evaporated. The residue was solidified in 2,2'-oxybispropane. The precipitated product was filtered off and crystallized from a mixture of trichloromethane and methanol (95:5 by volume). The product was filtered off and dried, yielding 6.53 parts (69.2%) of 1-(cyanomethyl)-4-(dimethylamino)-*N*-(2,6-dimethylphenyl)-4-piperidinecarboxamide; mp. 193.3°C (compound 250).

A mixture of 6 parts of 1-(cyanomethyl)-4-(dimethylamino)-*N*-(2,6-dimethylphenyl)-4-piperidinecarboxamide and 320 parts of methanol saturated with ammonia was hydrogenated at normal pressure and at 20°C with 3 parts of Raney-nickel catalyst. After the calculated amount of hydrogen was taken up, the catalyst was filtered off and the filtrate was evaporated. The residue was stirred in 2,2'-oxybispropane. The product was filtered off and purified by column chromatography over silica gel using a mixture of trichloromethane and methanol (90:10 by volume), saturated with ammonia, as eluent. The first fraction was collected and the eluent was evaporated. The residue was stirred in 2,2'-oxybispropane. The product was filtered off and dried, yielding 5.14 parts (84%) of 1-(2-aminoethyl)-4-(dimethylamino)-*N*-(2,6-dimethylphenyl)-4-piperidinecarboxamide; mp. 162.6°C (compound 251).

A mixture of 1.2 parts of 7-oxabicyclo[4.1.0]heptane, 3.19 parts of 1-(2-aminoethyl)-4-(dimethylamino)-*N*-(2,6-dimethylphenyl)-4-piperidinecarboxamide, 25 parts of water and 20 parts of ethanol was stirred and refluxed for 30 hours. The reaction mixture was evaporated. The residue was dissolved in trichloromethane. The solution was dried, filtered and evaporated. The residue was purified by column chromatography over silica gel using a mixture of trichloromethane and methanol (93:7 by volume), saturated with ammonia, as eluent. The first fraction was collected and the eluent was evaporated. The residue was crystallized from acetonitrile. The product was filtered off and dried, yielding 2.63 parts (63.2%) of *trans*-4-(dimethylamino)-*N*-(2,6-dimethylphenyl)-1-[2-[(2-hydroxycyclohexyl)amino]ethyl]-4-piperidinecarboxamide; mp. 141.8°C (compound 252).

## Claims

1. A chemical compound having the formula

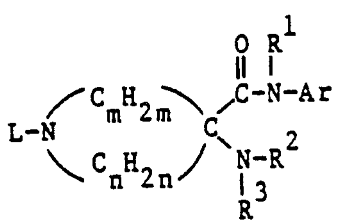

(I),

a pharmaceutically acceptable acid addition salt or a possible stereochemically isomeric form thereof, wherein one of the hydrogen atoms within the radical $—C_mH_{2m}—$ or $—C_nH_{2n}$ may be replaced by hydroxy, lower alkyloxy or lower alkyl; and wherein:

m and n are each the integer 1, 2 or 3, the sum of m and n being 3 or 4;

$R^1$, $R^2$ and $R^3$ are each independently selected from the group consisting of hydrogen, lower alkyl, (aryl)lower alkyl and lower alkylcarbonyl; or $R^1$ and $R^2$, when taken together, may form a methylene function; or $R^2$ and $R^3$, taken together with the nitrogen atom, may form a 1-piperidinyl, 1-pyrrolidinyl or 4-morpholinyl radical;

Ar is aryl; and

L is a member selected from the group consisting of hydrogen; pyrimidinyl; tetrahydropyrimidinyl; tetrahydropyranyl; dihydroindenyl; lower alkenyl; (aryl)lower alkenyl; cycloalkenyl optionally substituted with a hydroxy or an oxo radical; cycloalkyl optionally substituted with up to two radicals selected from the group consisting of lower alkyl, substituted lower alkyl, a radical of formula $—OR^4$, a radical of formula $—O—CO—R^5$, a radical of formula $—NR^6R^8$ and a radical of formula $—NH—CO—R^7$, wherein said substituted lower alkyl is lower alkyl being substituted with a member selected from the group consisting of (lower alkyl)carbonyl, (lower alkyloxy)carbonyl, amino, mono- and di(lower alkyl)amino, $R^4$ is hydrogen, lower alkyl or aryllower alkyl, $R^5$ is lower alkyl, aryl, or aryllower alkyl, wherein up to two hydrogen radicals in the lower alkyl part of the said aryllower alkyl radical may independently be replaced by a member selected from the group consisting of lower alkyloxy and trifluoromethyl, $R^6$ is hydrogen, lower alkyl, aryllower alkyl, lower alkylsulfonyl, or hydroxylower alkyl, $R^7$ is amino, lower alkyl, aryl, lower alkyloxy, or aryllower alkyl and $R^8$ is hydrogen, lower alkyl, hydroxylower alkyl, or aryllower alkyl;

a radical of formula

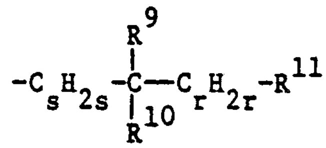

(a)

wherein r and s are independently 0 or an integer of from 1 to 6 inclusive, $R^9$ is hydrogen or hydroxy, $R^{10}$ is hydrogen, lower alkyl or hydroxylower alkyl, and $R^{11}$ is hydrogen, hydroxy, lower alkyloxy, aryloxy, aryllower alkyloxy, cyano, amino, mono- and di(lower alkyl)amino, arylamino, mono- and di(aryllower alkyl)-amino, aminocarbonyl, mono- and di(lower alkyl)aminocarbonyl, 1-piperidinyl, 4-morpholinyl, 1-pyrrolidinyl, arylaminocarbonyl, 1-piperidinylcarbonyl, 4-morpholinylcarbonyl, 1-pyrrolidinylcarbonyl, lower alkyloxycarbonyl, arylcarbonyl, aryl, cycloalkyl, cycloalkylamino or cycloalkylaminocarbonyl, said cycloalkyl, as embraced in the definition of $R^{11}$, being optionally substituted with a hydroxy radical;

a radical of formula

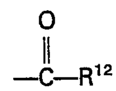

(b)

wherein $R^{12}$ is lower alkyl, lower alkyloxy, (aryl)lower alkyloxy, aryl or cycloalkyl optionally substituted with hydroxy; and

a radical of formula

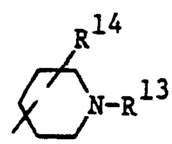

(c)

wherein $R^{13}$ is hydrogen, lower alkyl, lower alkyloxycarbonyl or [(aryl)lower alkyloxy]carbonyl, and $R^{14}$ is hydrogen, hydroxy or lower alkyloxy;

wherein aryl is phenyl optionally substituted with up to three substituents each independently selected from the group consisting of lower alkyl, halo, hydroxy, lower alkyloxy, trifluoromethyl, amino, mono- and di(lower alkyl)amino and nitro, and wherein the term "lower alkyl" is meant to include straight and branch chained saturated hydrocarbon radicals having from 1 to 6 carbon atoms, the term "lower alkenyl" refers to alkenyl radicals having from 3 to 6 carbon atoms, the term "cycloalkyl" is generic to cyclopropyl, cyclobutyl, cyclopentyl and cyclohexyl; and the term "cycloalkenyl" refers to cyclopentenyl and cyclohexenyl.

2. A chemical compound according to claim 1 wherein $R^1$ is hydrogen and Ar is 2,6-dimethylphenyl.

3. A chemical compound according to claim 1 wherein $R^1$ is hydrogen, Ar is 2,6-dimethylphenyl and L is cycloalkyl optionally substituted with hydroxy.

4. A chemical compound according to claim 1 wherein the compound of formula (I) is 4-(dimethylamino)-*N*-(2,6-dimethylphenyl)-1-(2-hydroxycyclohexyl)-4-piperidinecarboxamide.

5. A pharmaceutical composition comprising per dosage unit an effective amount of at least one compound as claimed in any of claims 1 to 4.

6. A pharmaceutical composition comprising per dosage unit an effective antiarrhythmic amount of *trans*-4-(dimethylamino)-*N*-(2,6-dimethylphenyl)-1-(2-hydroxycyclohexyl)-4-piperidinecarboxamide.

7. A process for preparing a chemical compound as claimed in claim 1, characterized by

a) amidating a carboxylic acid of formula

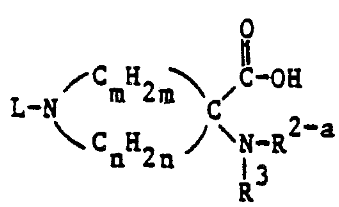

(II)

or a halide, anhydride, or ester derivative thereof, with an amine of formula

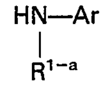

(III)

wherein $R^{1-a}$ and $R^{2-a}$ are as previously described for $R^1$ and $R^2$, provided that $R^1$ and $R^2$ combined, do not

form a methylene radical, in an appropriate reaction-inert medium, thus yielding a compound of formula

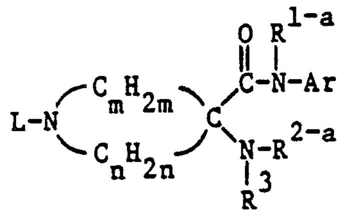

(I-a); or

b) amidating an intermediate of formula

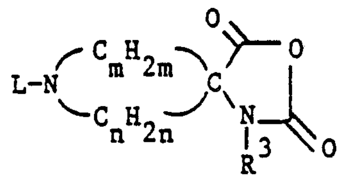

(IV)

with an amine of formula (III) in a suitable reaction-inert medium thus yielding a compound of formula

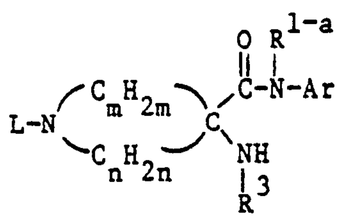

(I-a-1); or

c) condensing a ketone of formula

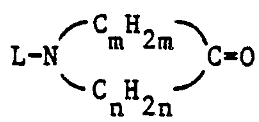

(V)

with an amine of formula

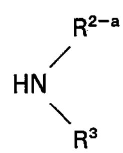

(VI)

and an isonitrile of formula

CN—Ar (VII)

in the presence of a suitable anion, in a suitable reaction-inert medium, thus yielding a compound of formula

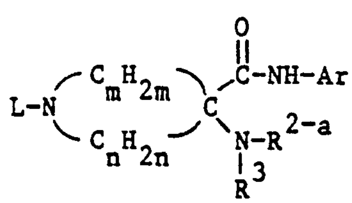

(I-a-2); and

optionally

i) cyclizing a compound of formula

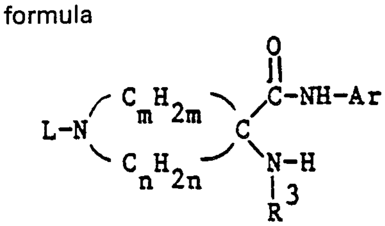

(I-a-1-a)

with formaldehyde or a polymeric form thereof, in a suitable reaction-inert medium, thus yielding a compound of formula

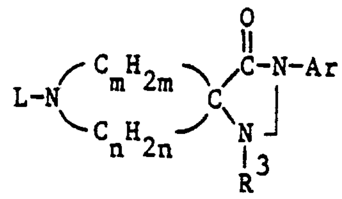

(I-b); or

ii) N-alkylating a compound of formula

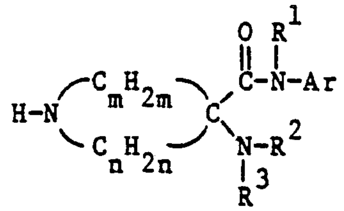

(I-c)

with a reagent of formula

$L^1$—W (VIII),

said W being an appropriate leaving group, in a suitable reaction-inert medium, thus yielding a compound of formula

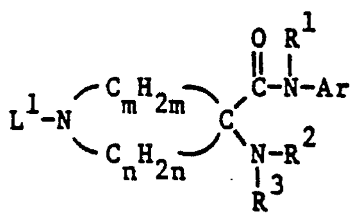

(I-d)

wherein $L^1$ is as previously described for L, provided that $L^1$ is other than hydrogen; or

iii) reductively N-alkylating a compound of formula (I—c) with an appropriate carbonyl-compound of formula

$L^{2'}$=C=O (IX)

in a suitable reaction-inert medium, thus yielding a compound of formula

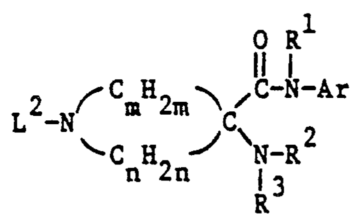

(I-e)

wherein $L^2$ is as previously described for L, provided that $L^2$ is not hydrogen or a radical of formula (b) and $L^{2'}$=C=O is a compound of formula $L^2$—H, wherein a —$CH_2$— radical is oxidated to a carbonyl radical; or

iv) condensing a compound of formula (I—c) with an appropriate cycloalkanone of formula

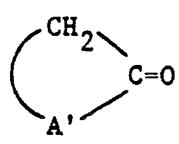

(X),

thus yielding a compound of formula

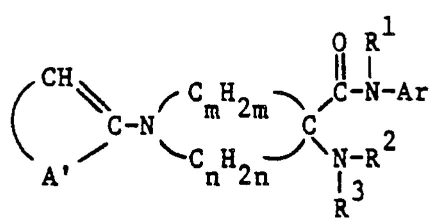

(I-f)

in a suitable reaction-inert medium; wherein in (X) and (I—f), A' taken together with the ethanediyl respectively ethenediyl radical, represents a cycloalkyl, respectively cycloalkenyl radical optionally substituted with hydroxy or carbonyl; or

v) reducing a compound of formula (I—f) with an appropriate reductive agent, thus yielding a compound of formula (I) wherein L is a 5 or 6-membered cycloalkyl radical optionally substituted with hydroxy or carbonyl; or

vi) condensing a compound of formula (I—c) with a reagent of formula

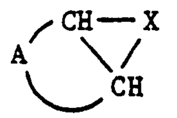

(XI),

in a suitable reaction-inert medium, thus yielding a compound of formula

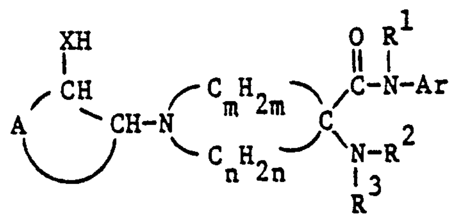

(I-g)

wherein A, taken together with the ethanediyl radical, represents an optionally substituted cycloalkyl radical and X is O or $NR^{15}$, said $R^{15}$ in combination with N, forming an appropriate substituent of the said cycloalkyl radical; or

vii) converting a compound of formula (I), wherein L is an aryllower alkyl radical into a compound of formula (I), wherein L is a lower alkyloxycarbonyl radical with an appropriate carbonohalidate in a suitable reaction-inert medium; or

viii) converting a compound of formula (I—d) into a compound of formula

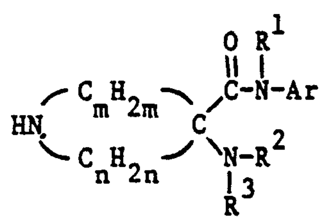

(I-c)

with a suitable reagent for converting tertiary amines or amides into secondary amines; and optionally, if desired, converting the compounds of formula (I) into a therapeutically active non-toxic acid-addition salt form by treatment with an appropriate acid or, conversely, converting the acid-addition salt into the free base form with alkali; and/or preparing stereochemically isomeric forms thereof.

8. A process for preparing 4-(dimethylamino)-*N*-(2,6-dimethylphenyl)-1-(2-hydroxycyclohexyl)-4-piperidinecarboxamide, characterized by the reaction of 7-oxabicyclo[4.1.0]heptane with 4-(dimethyl-amino)-*N*-(2,6-dimethylphenyl)-4-piperidinecarboxamide.

9. A process for preparing a pharmaceutical composition as claimed in any one of claims 5 and 6, characterized by mixing an effective amount of a compound as claimed in any one of claims 1 to 4 with an inert carrier.

10. A compound as claimed in any one of claims 1 to 4 for use as an antiarrhythmic agent.

**Patentansprüche**

1. Chemische Verbindung mit der Formel

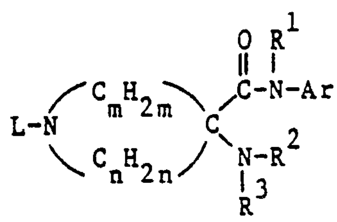

(I),

pharmazeutische annehmbares Säureadditionssalz oder mögliche steriochemisch isomere Form hievon,

worin eines der Wasserstoffatome innerhalb des Restes $—C_mH_{2m}—$ oder $—C_nH_{2n}—$ durch Hydroxy, Niederalkyloxy oder Niederalkyl ersetzt sein kann; und worin:

m und n jeweils eine ganze Zahl 1, 2 oder 3 bedeuten, wobei die Summe aus m und n 3 oder 4 beträgt;

$R^1$, $R^2$ und $R^3$ jeweils unabhängig voneinander aus der aus Wasserstoff, Niederalkyl, (Aryl)niederalkyl und Niederalkylcarbonyl bestehenden Gruppe ausgewählt sind; oder $R^1$ und $R^2$ zusammengenommen eine Methylenfunktion darstellen können; oder $R^2$ und $R^3$, zusammen mit dem Stickstoffatom, einen 1-Piperidinyl-, 1-Pyrrolidinyl- oder 4-Morpholinylrest ausbilden können;

Ar für Aryl steht; und

L für ein Glied steht, das aus der Gruppe ausgewählt ist, die aus Wasserstoff; Pyrimidinyl; Tetrahydropyrimidinyl; Tetrahydropyranyl; Dihydroindenyl; Niederalkenyl; (Aryl)niederalkenyl; Cycloalkenyl, das gegebenenfalls durch einen Hydroxy- oder einen Oxorest substituiert ist; Cykloalkyl, das gegebenenfalls mit bis zu zwei Resten, ausgewählt aus der aus Niederalkyl, substituiertem Niederalkyl, einem Rest der Formel $—OR^4$, einem Rest der Formel $—O—CO—R^5$, einem Rest der Formel $—NR^6R^8$ und einem Rest der Formel $—NH—CO—R^7$ bestehenden Gruppe substituiert ist, worin das genannte substituierte Niederalkyl ein Niederalkylrest ist, der durch ein aus der aus (Niederalkyl)carbonyl, (Niederalkyloxy)carbonyl, Amino, Mono- und Di(niederalkyl)amino bestehenden Gruppe ausgewähltes Glied substituiert ist, $R^4$ für Wasserstoff, Niederalkyl oder Arylniederalkyl steht, $R^5$ für Niederalkyl, Aryl oder Arylniederalkyl steht, wobei bis zu zwei Wasserstoffreste in dem Niederalkylteil des genannten Arylniederalkylrestes unabhängig voneinander durch ein aus der aus Niederalkyloxy und Trifluormethyl bestehenden Gruppe ausgewähltes Glied ersetzt sein können, $R^6$ für Wasserstoff, Niederalkyl, Arylniederalkyl, Niederalkylsulfonyl oder Hydroxyniederalkyl steht, $R^7$ Amino, Niederalkyl, Aryl, Niederalkyloxy oder Arylniederalkyl bedeutet und $R^8$ für Wasserstoff, Niederalkyl, Hydroxyniederalkyl oder Arylniederalkyl steht;

einem Rest der Formel

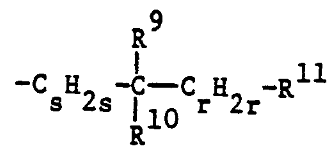

(a)

worin r und s unabhängig voneinander 0 oder eine ganze Zahl von 1 bis einschließlich 6 bedeuten, $R^9$ Wasserstoff oder Hydroxy ist, $R^{10}$ für Wasserstoff, Niederalkyl oder Hydroxyniederalkyl steht und $R^{11}$ Wasserstoff, Hydroxy, Niederalkyloxy, Aryloxy, Arylniederalkyloxy, Cyano, Amino, Mono- und Di(niederalkyl)amino, Arylamino, Mono- und Di(arylniederalkyl)amino, Aminocarbonyl, Mono- und Di(niederalkyl)-aminocarbonyl, 1-Piperidinyl, 4-Morpholinyl, 1-Pyrrolidinyl, Arylaminocarbonyl, 1-Piperidinylcarbonyl, 4-Morpholinylcarbonyl, 1-Pyrrolidinylcarbonyl, Niederalkyloxycarbonyl, Arylcarbonyl, Aryl, Cycloalkyl, Cycloalkylamino oder Cycloalkylaminocarbonyl bedeutet, wobei das in der Definition von $R^{11}$ enthaltene Cycloalkyl gewünschtenfalls durch einen Hydroxyrest substituiert ist;

einem Rest der Formel

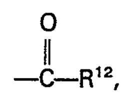

(b)

worin $R^{12}$ für Niederalkyl, Niederalkyloxy, (Aryl)niederalkyloxy, Aryl oder gegebenenfalls durch Hydroxy substituiertes Cycloalkyl steht; und

einem Rest der Formel

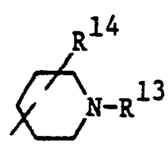

(c)

worin $R^{13}$ für Wasserstoff, Niederalkyl, Niederalkoxycarbonyl oder [(Aryl)niederalkyloxy]carbonyl steht und $R^{14}$ Wasserstoff, Hydroxy oder Niederalkyloxy bedeutet;

besteht; worin Aryl Phenyl bedeutet, das gegebenenfalls mit bis zu 3 Substituenten, jeweils unabhängig voneinander ausgewählt aus der aus Niederalkyl, Halogen, Hydroxy, Niederalkyloxy, Trifluormethyl, Amino, Mono- und Di(niederalkyl)amino und Nitro bestehenden Gruppe, substituiert ist, und worin der Ausdruck "Niederalkyl" gerade und verzweigtkettige gesättigte Kohlenwasserstoffreste mit 1 bis 6 Kohlenstoffatomen bezeichnen soll, der Ausdruck "Niederalkenyl" sich auf Alkenylreste mit 3 bis 6 Kohlenstoffatomen bezieht, der Ausdruck "Cycloalkyl" generisch für Cyclopropyl, Cyclobutyl, Cyclopentyl und Cyclohexyl steht; und der Ausdruck "Cycloalkenyl" sich auf Cyclopentenyl und Cyclohexenyl bezieht.

2. Chemische Verbindung nach Anspruch 1, worin $R^1$ für Wasserstoff steht und Ar 2,6-Dimethylphenyl bedeutet.

3. Chemische Verbindung nach Anspruch 1, worin $R^1$ Wasserstoff ist, Ar für 2,6-Dimethylphenyl steht und L gegebenenfalls durch Hydroxy substituiertes Cycloalkyl darstellt.

4. Chemische Verbindung nach Anspruch 1, worin die Verbindung der Formel (I) 4-(Dimethylamino)-N-(2,6-dimethylphenyl)-1-(2-hydroxycyclohexyl)-4-piperidincarboxamid ist.

5. Pharmazeutische Zusammensetzung, umfassend je Dosiseinheit eine wirksame Menge wenigstens einer Verbindung, wie in einem der Ansprüche 1 bis 4 beansprucht.

6. Pharmazeutische Zusammensetzung, enthaltend je Dosiseinheit eine antiarrhytmisch wirksame Menge von trans-4-(Dimethylamino)-N-(2,6-dimethylphenyl)-1-(2-hydroxycyclohexyl)-4-piperidincarboxamid.

7. Verfahren zur Herstellung einer chemischen Verbindung, wie in Anspruch 1 beansprucht, gekennzeichnet durch

a) Amidieren einer Carbonsäure der Formel

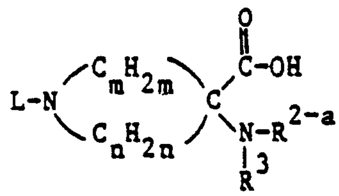

(II)

oder eines Halogenids, Anhydrids oder Esterderivats hievon, mit einem Amin der Formel

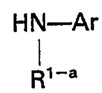

, (III)

worin $R^{1-a}$ und $R^{2-a}$ wie zuvor für $R^1$ und $R^2$ beschrieben definiert sind, mit der Maßgabe, daß $R^1$ und $R^2$ zusammengenommen nicht einen Methylenrest ausbilden, in einem geeigneten reaktionsinerten Medium unter Ausbildung einer Verbindung der Formel

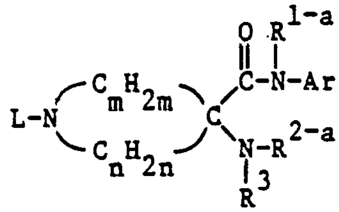

(I-a)

oder

b) Amidieren einer Zwischenverbindung der Formel

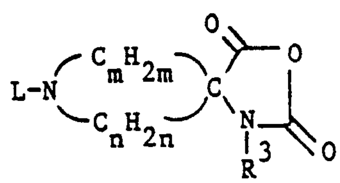

(IV)

mit einem Amin der Formel (III) in einem geeigneten reaktionsinerten Medium unter Ausbildung einer Verbindung der Formel

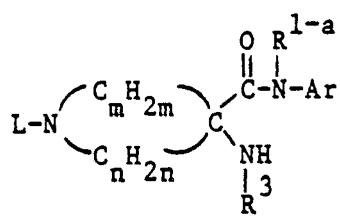

(I-a-1)

oder

c) Kondensieren eines Ketons der Formel

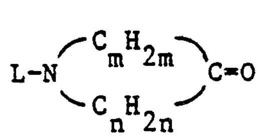

(V)

mit einem Amin der Formel

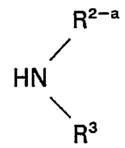

(VI)

und einem Isonitril der Formel

CN—Ar (VII)

in Gegenwart eines geeigneten Anions in einem geeigneten reaktions-inerten Medium unter Ausbildung einer Verbindung der Formel

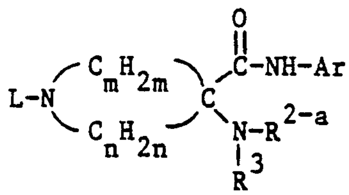

(I-a-2)

und gewünschtenfalls

i) Cyklisieren einer Verbindung der Formel

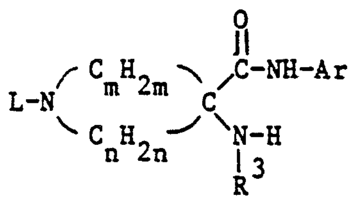

(I-a-1-a)

mit Formaldehyd oder einer polymeren Form hievon, in einem geeigneten reaktions-inerten Medium, unter Ausbildung einer Verbindung der Formel

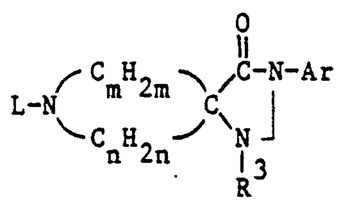

(I-b)

ii) N-Alkylieren einer Verbindung der Formel

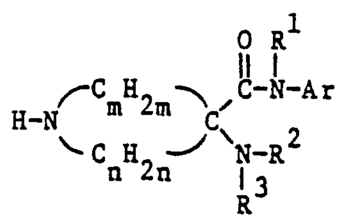

(I-c)

mit einem Reagens der Formel

$L^1$—W (VIII),

worin W eine geeignete Leaving-Gruppe darstellt, in einem geeigneten reaktions-inerten Medium unter Ausbildung einer Verbindung der Formel

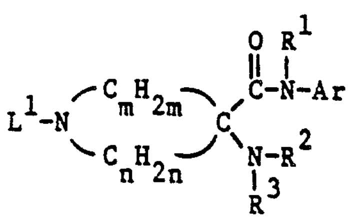

(I-d)

worin $L^1$ wie zuvor für L angegeben definiert ist, mit der Maßgabe, daß $L^1$ eine andere Bedeutung als Wasserstoff hat; oder

iii) reduktives N-Alkylieren einer Verbindung der Formel (I—c) mit einer geeigneten Carbonyl-Verbindung der Formel

$$L^{2'}=C=O \qquad (IX)$$

in einem geeigneten reaktions-inerten Medium unter Ausbildung einer Verbindung der Formel

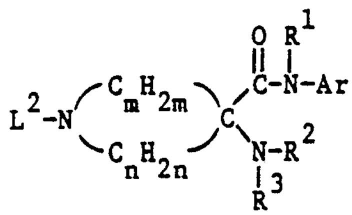

(I-e)

worin $L^2$ wie zuvor für L angegeben definiert ist, mit der Maßgabe, daß $L^2$ nicht für Wasserstoff oder einen Rest der Formel (b) steht und worin $L^{2'}=C=O$ eine Verbindung der Formel $L^2$—H ist, worin eine —$CH_2$-Gruppe zu einem Carbonylrest oxidiert ist; oder

iv) Kondensieren einer Verbindung der Formel (I—c) mit einem entsprechenden Cycloalkanon der Formel

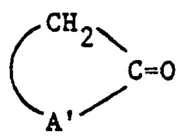

(X),

in einem geeigneten reaktions-inerten Medium unter Ausbildung einer Verbindung der Formel

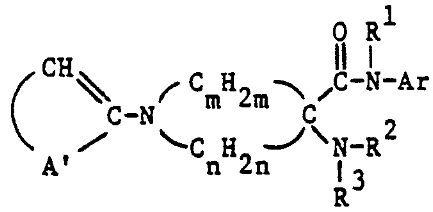

(I-f)

wobei in den Verbindungen der Formeln (X) und (I—f) A', zusammen mit dem Ethandiyl- bzw. Ethendiylrest, einen Cycloalkyl- bzw. Cycloalkenylrest bedeutet, der gegebenenfalls durch Hydroxy oder Carbonyl substituiert ist; oder

v) Reduzieren einer Verbindung der Formel (I—f) mit einem entsprechenden Reduktionsmittel unter Ausbildung einer Verbindung der Formel (I), worin L einen 5- oder 6-gliedrigen Cycloalkylrest bedeutet, der gegebenenfalls durch Hydroxy oder Carbonyl substituiert ist; oder

vi) Kondensieren einer Verbindung der Formel (I—c) mit einem Reagens der Formel

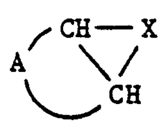

(XI),

in einem geeigneten reaktions-inerten Medium unter Ausbildung einer Verbindung der Formel

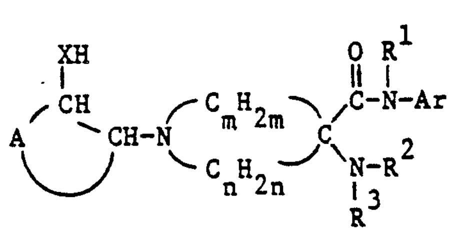

(I-g)

worin A, zusammen mit dem Ethandiylrest, einen gegebenenfalls substituierten Cycloalkylrest bedeutet und X für Sauerstoff oder $NR^{15}$ steht, wobei der Rest $R^{15}$, zusammen mit N, einen entsprechenden Substituenten des Cycloalkylrestes darstellt; oder

vii) Überführen einer Verbindung der Formel (I), worin L einen Arylniederalkylrest darstellt, mit einem geeigneten Carbonohalidat in einem geeigneten reaktions-inerten Medium in eine Verbindung der Formel (I), worin L einen Niederalkyloxycarbonylrest bedeutet; oder

viii) Überführen einer Verbindung der Formel (I—d) in eine Verbindung der Formel

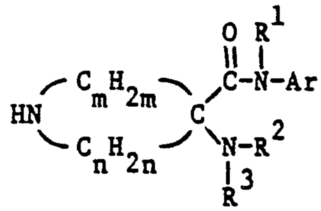

(I-c)

mit einem geeigneten Reagens zur Umwandlung tertiärer Amine oder Amide in sekundäre Amine; und gewünschtenfalls Überführen der Verbindungen der Formel (I) in eine therapeutisch wirksame nichttoxische Säureadditionssalzform durch Behandlung mit einer entsprechenden Säure, oder umgekehrt, Überführen des Säureadditionssalzes in die freie Basenform mit Alkali; und/oder Bereiten stereochemisch isomerer Formen hievon.

8. Verfahren zur Herstellung von 4-(Dimethylamino)-N-(2,6-dimethylphenyl)-1-(2-hydroxycyclohexyl)-4-piperidincarboxamid, gekennzeichnet durch die Umsetzung von 7-Oxabicyclo[4.1.0]heptan mit 4-(Dimethylamino)-N-(2,6-dimethylphenyl)-4-piperidincarboxamid.

9. Verfahren zur Herstellung einer pharmazeutischen Zusammensetzung, wie in einem der Ansprüche 5 und 6 beansprucht, gekennzeichnet durch Vermischen einer wirksamen Menge einer Verbindung, wie in einem der Ansprüche 1 bis 4 beansprucht, mit einem inerten Träger.

10. Verbindung, wie in einem der Ansprüche 1 bis 4 beansprucht, zur Anwendung als ein antiarrhytmisches Mittel.

**Revendications**

1. Composé chimique ayant pour formule

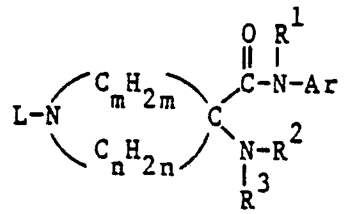

(I),

leurs sels d'addition d'acides convenant en pharmacie et leurs formes isomères stéréochimiques possibles; où un des atomes d'hydrogène, dans le radical $—C_mH_{2m}—$ ou $—C_nH_{2n}—$, peut être remplacé par un hydroxy, un alkyloxy inférieur ou un alkyle inférieur; et où:

m et n sont chacun l'entier 1, 2 ou 3, la somme de m et n étant 3 ou 4;

$R^1$, $R^2$, et $R^3$ sont chacun indépendamment choisis dans le groupe constitué par un hydrogène, un alkyle inférieur, un arylalkyle inférieur et un (alkyl inférieur)carbonyle; ou $R^1$ et $R^2$, pris ensemble, peuvent former une fonction méthylène; ou $R^2$ et $R^3$, pris ensemble avec l'atome d'azote, peuvent former un radical 1-pipéridyle, 1-pyrrolidinyle ou 4-morpholinyle;

Ar est un aryle; et

L est un composant choisi dans le groupe constitué par l'hydrogène; un pyrimidinyle; un tétrahydropyrimidinyle; un tétrahydropyrannyle; un dihydro-indényle; un alcényle inférieur; un aryl-alcényle inférieur; un cycloalcényle facultativement substitué par un radical hydroxy ou oxo; un cycloalkyle facultativement substitué par jusqu'à deux radicaux choisis dans le groupe constitué par un alkyle inférieur, un alkyle inférieur substitué, un radical de formule $—OR^4$, un radical de formule $—O—CO—R^5$, un radical de formule $—NR^6R^8$ et un radical de formule $—NH—CO—R^7$, où ledit alkyle inférieur substitué est un alkyle inférieur substitué par un composant choisi dans le groupe constitué par un (alkyl inférieur)carbonyle, un (alkyloxy inférieur)carbonyle, un amino, un mono et un di(alkyl inférieur)amino, $R^4$ est un hydrogène, un alkyle inférieur ou un aryl-alkyle inférieur, $R^5$ est un alkyle inférieur, un aryle ou un aryl-alkyle inférieur, où jusqu'à deux atomes d'hydrogène de la partie alkyle inférieur dudit radical aryl-alkyle inférieur peuvent indépendamment être remplacés par un composant choisi dans le groupe constitué par un alkyloxy inférieur et un trifluorométhyle, $R^6$ est un hydrogène, un alkyle inférieur, un aryl-alkyle inférieur, un alkylsulfonyle inférieur ou un hydroxyalkyle inférieur, $R^7$ est un amino, un alkyle inférieur, un aryle, un alkyloxy inférieur ou un aryl-alkyle inférieur et $R^8$ est un hydrogène, un alkyle inférieur, un hydroxyalkyle inférieur ou un aryl-alkyle inférieur;

un radical de formule

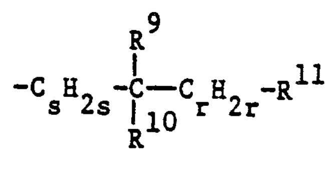

(a)

dans laquelle r et s sont indépendamment 0 ou un entier de 1 à 6 inclusivement, $R^9$ est un hydrogène ou un hydroxy, $R^{10}$ est un hydrogène, un alkyle inférieur ou un hydroxyalkyle inférieur et $R^{11}$ est un hydrogène, un hydroxy, un alkyloxy inférieur, un aryloxy, un aryl-alkyloxy inférieur, un cyano, un amino, un mono ou di(alkyl inférieur)amino, un arylamino, un mono ou di(aryl-alkyl inférieur)amino, un aminocarbonyle, un mono ou di(alkyl inférieur)aminocarbonyle, un 1-pipéridyle, un 4-morpholinyle, un 1-pyrrolidinyle, un arylaminocarbonyle, un 1-pipéridylcarbonyle, un 4-morpholinylcarbonyle, un 1-pyrrolidinylcarbonyle, un (alkyloxy inférieur)carbonyle, un arylcarbonyle, un aryle, un cycloalkyle, un cycloalkylamino ou un cycloalkylaminocarbonyle, ledit cycloalkyle, dans la définition de $R^{11}$, étant facultativement substitué par un radical hydroxy;

un radical de formule

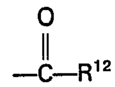

(b)

dans laquelle $R^{12}$ est un alkyle inférieur, un alkyloxy inférieur, un aryl-alkyloxy inférieur, un aryle ou un cycloalkyle facultativement substitué par un hydroxy; et

un radical de formule

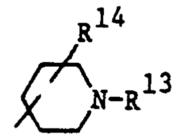

(c)

dans laquelle $R^{13}$ est un hydrogène, un alkyle inférieur, un (alkyloxy inférieur)carbonyle ou un (aryl-alkyloxy inférieur)carbonyle et $R^{14}$ est un hydrogène, un hydroxy ou un alkyloxy inférieur;

où un aryle est un phényle facultativement substitué par jusqu'à trois substituants choisis chacun indépendamment dans le groupe constitué par un alkyle inférieur, un halogéno, un hydroxy, un alkyloxy inférieur, un trifluorométhyle, un amino, un mono ou di(alkyl inférieur)amino et un nitro, et ou le terme "alkyle inférieur" comprend les radicaux hydrocarbonés saturés droits ou ramifiés ayant 1 à 6 atomes de carbone, le terme "alcényle inférieur" désigne les radicaux alcényles ayant 3 à 6 atomes de carbone, le terme "cycloalkyle" désigne de façon générale cyclopropyle, cyclobutyle, cyclopentyle et cyclohexyle et le terme "cycloalcényle" désigne cyclopentényle et cyclohexényle.

2. Composé chimique selon la revendication 1, où $R^1$ est un hydrogène et Ar est un 2,6-diméthylphényle.

3. Composé chimique selon la revendication 1, où $R^1$ est un hydrogène, Ar est un 2,6-diméthylphényle et L est un cycloalkyle facultativement substitué par un hydroxy.

4. Composé chimique selon la revendication 1, où le composé de formule (I) est le 4-(diméthylamino)-N-(2,6-diméthylphényl)-1-(2-hydroxycyclohexyl)-4-pipéridinecarboxamide.

5. Composition pharmaceutique comprenant par dose unitaire une quantité efficace d'au moins un composé selon l'une quelconque des revendications 1 à 4.

6. Composition pharmaceutique comprenant par dose unitaire une quantité antiarythmique efficace de trans-4-(diméthylamino)-N-(2,6-diméthylphényl)-1-(2-hydroxycyclohexyl)-4-pipéridinecarboxamide.

7. Procédé pour préparer un composé chimique selon la revendication 1, caractérisé par

a) l'amidation d'un acide carboxylique de formule

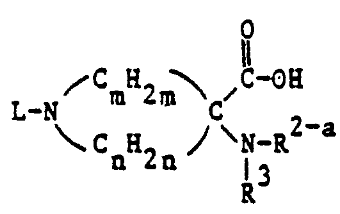

(II)

ou d'un halogénure, anhydride ou ester en dérivant, avec une amine de formule

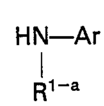

(III)

dans laquelle $R^{1-a}$ et $R^{2-a}$ sont comme précédemment décrit pour $R^1$ et $R^2$, sous réserve que $R^1$ et $R^2$ combinés ne forment pas un radical méthylène, dans un milieu approprié inerte dans la réaction, pour former un composé de formule

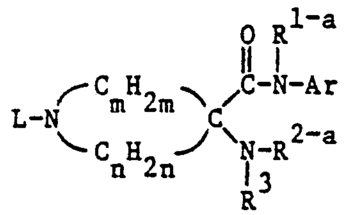

(I-a)

ou

b) l'amidation d'un intermédiaire de formule

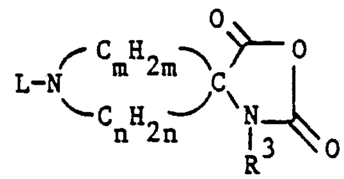

(IV)

avec une amine de formule (III) dans un milieu approprié inerte dans la réaction pour former un composé de formule

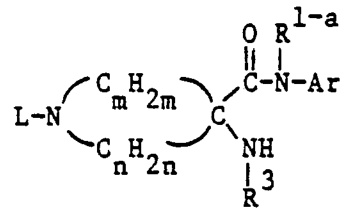

(I-a-1)

ou

c) la condensation d'une cétone de formule

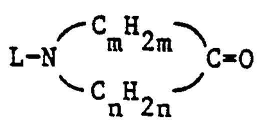

(V)

avec une amine de formule

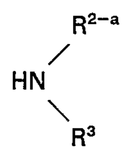

(VI)

et un isonitrile de formule

CN—Ar (VII)

en présence d'un anion approprié, dans un milieu approprié inerte dans la réaction pour former un composé de formule

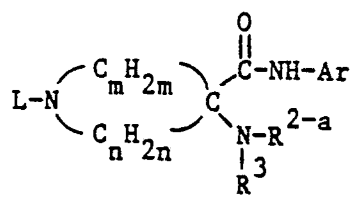

(I-a-2)

et facultativement,

i) la cyclisation d'un composé de formule

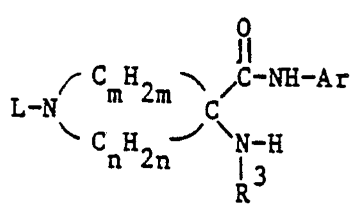

(I-a-1-a)

avec le formaldéhyde ou une forme polymère de celui-ci, dans un milieu approprié inerte dans la réaction, pour former un composé de formule

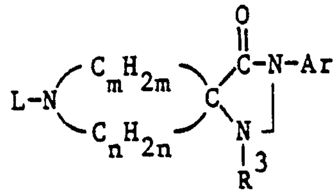

(I-b)

ou

ii) la N-alkylation d'un composé de formule

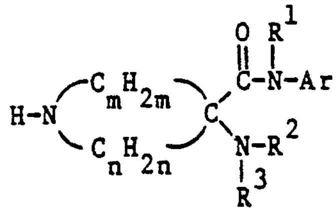

(I-c)

avec un composé réagissant de formule

$L^1—W$ (VIII),

ledit W étant un groupe labile approprié, dans un milieu approprié inerte dans la réaction, pour former un composé de formule

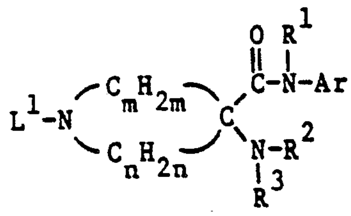

(I-d)

dans laquelle $L^1$ est comme précédemment décrit pour L, sous réserve que $L^1$ soit autre qu'un hydrogène; ou

iii) la N-alkylation par réduction d'un composé de formule (I—c) avec un composé carbonylé approprié de formule

$L^{2'}=C=O$ (IX)

dans un milieu approprié inerte dans la réaction, pour former un composé de formule

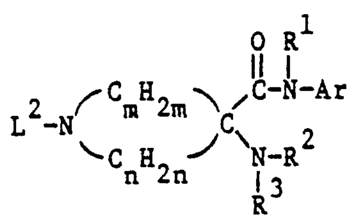

(I-e)

dans laquelle $L^2$ est comme précédemment décrit pour L, sous réserve que $L^2$ ne soit pas un hydrogène ou un radical de formule (b) et $L^{2'}=C=O$ est un composé de formule $L^2—H$ dans laquelle un radical $—CH^2—$ est oxydé en un radical carbonyle; ou

iv) la condensation d'un composé de formule (I—c) avec une cycloalcanone appropriée de formule

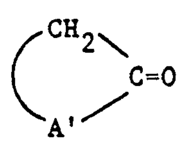

(X),

pour former un composé de formule

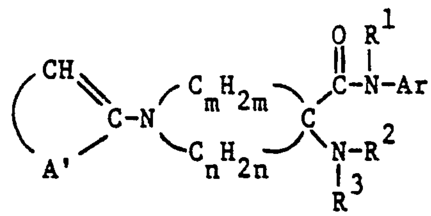

(I-f)

dans un milieu approprié inerte dans la réaction; où dans (X) et dans (I—f), A', pris avec respectivement le radical éthanediyle et le radical éthènediyle, représente respectivement un radical cycloalkyle ou un radical cycloalcényle facultativement substitué par un hydroxy ou un carbonyle; ou

v) la réduction d'un composé de formule (I—f) avec un agent réducteur approprié pour former un composé de formule (I) dans laquelle L est un radical cycloalkyle à 5 ou 6 chaînons facultativement substitué par un hydroxy ou un carbonyle; ou

vi) la condensation d'un composé de formule (I—c) avec un composé réagissant de formule

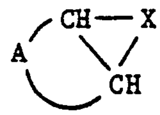

(XI),

dans un milieu approprié inerte dans la réaction, pour former un composé de formule

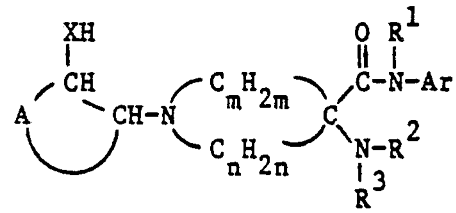

(I-g)

dans laquelle A, pris avec le radical éthanediyle, représente un radical cycloalkyle facultativement substitué et X est O ou $NR^{15}$, ledit $R^{15}$, en combinaison avec N, formant un substituant approprié dudit radical cycloalkyle; ou

vii) la conversion d'un composé de formule (I) dans laquelle L est un radical aryle-alkyle inférieur en un composé de formule (I) dans laquelle L est un radical (alkyloxy inférieur) carbonyle avec un halogéno-formiate approprié dans un milieu approprié inerte dans la réaction; ou

viii) la conversion d'un composé de formule (I—d) en un composé de formule

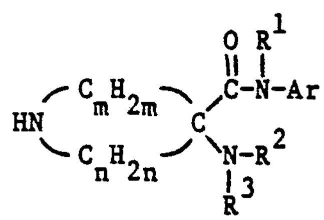

(I-c)

avec un composé réagissant approprié pour transformer les amines ou les amides tertiaires en amines secondaires, et facultativement, si on le désire, la conversion des composés de formule (I) en un sel d'addition d'acide non toxique à activité thérapeutique par traitement avec un acide approprié ou, inversement, la conversion du sel d'addition d'acide en la base libre avec un alcali et/ou la préparation des formes isomères stéréochimiques correspondantes.

8. Procédé pour préparer le 4-(diméthylamino)-N-(2,6-diméthylphényl)-1-(2-hydroxycyclohexyl)-4-pipéridinecarboxamide caractérisé par la réaction du 7-oxabicyclo[4.1.0]heptane avec le 4-(diméthyl-amino)-N-(2,6-diméthylphényl)-4-pipéridinecarboxamide.

9. Procédé pour préparer une composition pharmaceutique selon l'une des revendications 5 et 6 caractérisé par le mélange d'une quantité efficace d'un composé selon l'une quelconque des revendications 1 à 4 avec un support inerte.

10. Composé selon l'une quelconque des revendications 1 à 4 pour l'utilisation comme agent antiarythmique.